(19) Europäisches Patentamt / European Patent Office / Office européen des brevets

(11) EP 4 620 956 A1

(12) **EUROPEAN PATENT APPLICATION**
published in accordance with Art. 153(4) EPC

(43) Date of publication:
**24.09.2025 Bulletin 2025/39**

(21) Application number: **23906100.5**

(22) Date of filing: **22.12.2023**

(51) International Patent Classification (IPC):
*C07D 471/04* (2006.01) *A61K 31/4353* (2006.01) *A61K 31/495* (2006.01) *A61P 35/00* (2006.01)

(52) Cooperative Patent Classification (CPC):
**A61K 31/4353; A61K 31/495; A61P 35/00; C07D 471/04**

(86) International application number:
**PCT/CN2023/141057**

(87) International publication number:
**WO 2024/131945 (27.06.2024 Gazette 2024/26)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **23.12.2022 CN 202211668333**
**08.11.2023 CN 202311484011**

(71) Applicant: **Zhejiang Yangli Pharmaceutical Technology Co., Ltd.**
**Hangzhou, Zhejiang 310015 (CN)**

(72) Inventors:
- **SHA, Wei**
**Shenzhen, Guangdong 518001 (CN)**
- **LIU, Yang**
**Shenzhen, Guangdong 518001 (CN)**
- **LIU, Yueqin**
**Shenzhen, Guangdong 518001 (CN)**
- **FANG, Douglas**
**Shenzhen, Guangdong 518001 (CN)**
- **ZHANG, Haolong**
**Shenzhen, Guangdong 518001 (CN)**

(74) Representative: **Novitas Patent AB**
**P.O. Box 55557**
**102 04 Stockholm (SE)**

# (54) PARP1 INHIBITORS

(57) Provided in the present invention are PARP1 inhibitors as shown in formula (I), or pharmaceutically acceptable salts, isotopic variants, tautomers, stereoisomers, prodrugs, polymorphs, hydrates or solvates thereof. Further provided in the present invention are a preparation method of the compounds, a pharmaceutical composition containing the compounds, and the use of the compounds in prevention and treatment of cancers, ischemic diseases or neurodegenerative diseases.

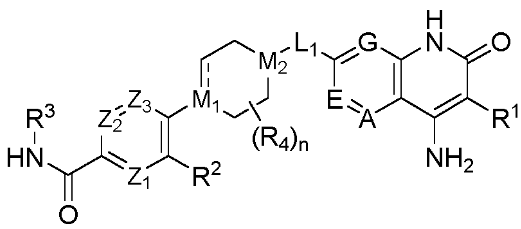

(I)



Processed by Luminess, 75001 PARIS (FR)

## Description

The present application claims the right of priority for Chinese Application No. 202211668333.2 filed on December 23, 2022, and Chinese Application No. 202311484011.7 filed on November 08, 2023, which are incorporated herein by reference in their entirety.

## Technical Field

The present invention relates to new PARP1 inhibitors, or pharmaceutically acceptable salts, isotopic variants, tautomers, stereoisomers, prodrugs, polymorphs, hydrates or solvates thereof. The present invention further relates to a preparation method of the compounds, a pharmaceutical composition containing the compounds, and the use of the compounds in prevention and treatment of PARP1-mediated diseases such as cancer.

## Background Art

ADP-ribosylation is a reaction process in which, under the action of an enzymatic reaction, the substrate nicotinamide adenine dinucleotide (NAD+) is broken down into nicotinamide and an ADP-ribose group, and the latter is covalently linked to a receptor protein. ADP-ribosylation is a common, reversible post-translational modification of proteins that plays an important role in a series of biological processes including DNA damage repair, cell proliferation and differentiation, metabolism, stress, etc.

Poly ADP-ribose polymerase (PARP) is a family of proteins that can catalyse ADP-ribosylation. PARP1 is the most widely studied and important member of the family of proteins. It is highly expressed and shows a quick response in cells. It can rapidly catalyse and modify DNA repair factors, and interact with them to participate in various DNA repair processes. In normal cells, DNA single-strand breaks can be repaired by base excision repair. PARP1 uses NAD+ as a substrate, binds to the damage site through its zinc finger domain to cause a conformational change in itself, and catalyses the transfer of ADP-ribose groups, ultimately completing single-strand repair. In cancer cells with double-strand breaks (DSBs), DNA breaks are mainly repaired by homologous recombination (HR). BRCA1 and BRCA2 are key proteins that mediate HR. In cancer cells with BRCA1/2 mutations, PARP1 function is simultaneously inhibited, causing obstruction of the main DNA repair pathway and ultimately leading to cell death. This is the synthetic lethality effect that has been fully validated clinically at present. Therefore, PARP1 has become a hot target for cancer treatment.

In recent years, four small molecule PARP inhibitors (PARPi) have been approved by the US FDA for the treatment of cancer, namely olaparib, niraparib, rucaparib and telazoparib. These drugs have shown excellent clinical therapeutic effects in the treatment of patients with BRCA1/2-mutated ovarian cancer and/or breast cancer, and have also demonstrated great promise in other BRCA-mutated cancer patients, including those with prostate cancer and pancreatic cancer.

Recent studies have shown that the mechanism of action of PARPi can be attributed to two different but interrelated mechanisms. First, by PARP1 catalytic inhibition, these PARPi block the synthesis of PAR chains, thereby suppressing the occurrence of poly-ADP ribosylation modification (PARylation) and blocking the PARP1-mediated DNA damage repair signals. Second, PARP inhibitors may also cause DNA double-strand breaks by inducing PARP1 trapping, ultimately killing cancer cells. When the PARP1 protein itself undergoes PARylation modification, it will dissociate from the site of DNA damage due to the steric hindrance and charge repulsion of the PAR chains. However, the treatment with PARP inhibitors blocks the self-modification of PARP1, resulting in PARP1 being trapped at the site of DNA damage. Long-existing PARP-DNA complexes occupy sites of DNA damage and interfere with subsequent DNA replication, leading to replication fork stalling and subsequent double-strand DNA damage, thus causing cell death. However, PARP1 trapping occurs concomitantly with the inhibition of PARP1 catalytic activity. Therefore, the inhibition of PARP1 catalytic activity and PARP1 trapping caused by PARP inhibitors are functionally completely different but intrinsically related.

Based on the mechanism of synthetic lethality, cell lines carrying BRCA1/2 mutations or with homologous recombination deficiency (HRD) are extremely sensitive to PARP inhibitors, which provides a very broad therapeutic safety window for drug treatment. However, since some normal cells in the body, such as myeloid-derived cells, are often in a state of rapid proliferation, DNA damage inevitably occurs. At the same time, many of the selected candidates have germline mutations, which leads to varying degrees of haematotoxicity when using the approved non-selective PARP1/2 inhibitors, including anaemia, neutropenia, thrombocytopenia, etc. The occurrence of grade III or IV hematologic toxicity often leads to drug reduction, withdrawal, or interruption of medication, seriously affecting the therapeutic effect of PARP inhibitors on patients.

On the other hand, there is a potential synergistic basis for combinations of chemotherapeutic drugs and PARP inhibitors. However, since chemotherapeutic drugs not only inhibit rapidly proliferating cells but also have strong haematotoxicity, the combined use of the two may lead to the superimposition of haematotoxicity, which greatly limits the combined use with PARP inhibitors.

Studies have shown that the anaemia response caused by PARP inhibitors may be mainly due to the inhibition of PARP2 (Farrés J, et al. Cell Death Differ. 2015 Jul; 22(7):1144-57.). Currently, all the marketed PARPi possess inhibitory activities against PARP1/2. If a highly selective PARP1 inhibitor can be developed to reduce the inhibition of PARP2, it may be possible to avoid the anaemia toxicity caused by the existing PARP1/2 inhibitors. WO 2021013735 A1 discloses a series of PARP1 selective inhibitors, such as AZD5305.

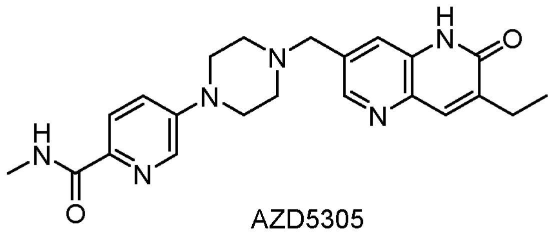

AZD5305

Therefore, developing more highly selective PARP1 inhibitors has important clinical application value, and there is a demand for such inhibitors in the art.

## Summary of the Invention

The present invention uses PARP1 as a target to develop new small molecule inhibitors that can be used to treat various cancers.

The compounds of the present invention target PARP1 and have excellent PARP1 inhibitory activity and selectivity, thereby avoiding anaemia toxicity. Meanwhile, the compounds of the present invention have different distribution volumes (which can reduce the toxicity to neutrophils and platelets) and different trapping activities (which are helpful for adjusting the drug dosage). While maintaining the clinical efficacy of PARP1/2 inhibitors, the compounds reduce the haematotoxicity, making it possible to further combine highly selective PARP1 inhibitors with chemotherapeutic drugs.

In one aspect, the present invention provides a compound of formula (I), or a pharmaceutically acceptable salt, an isotopic variant, a tautomer, a stereoisomer, a prodrug, a polymorph, a hydrate or a solvate thereof:

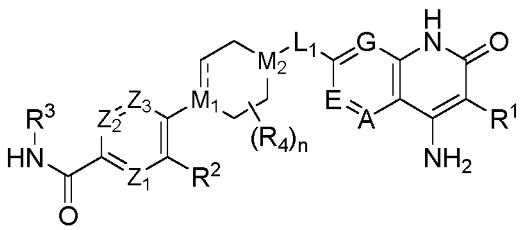

(I)

wherein

- $L_1$ is selected from CRR', O, S, NH, -C(O)-, -S(O)- or $-S(O)_2-$;
- R and R' are independently selected from H, D, halogen, $C_{1-6}$ alkyl or $C_{1-6}$ haloalkyl;
- A is selected from $CR_A$ or N;
- E is selected from $CR_E$ or N;
- G is selected from $CR_G$ or N;
- $R_A$, $R_E$ and $R_G$ are independently selected from H, D, halogen, CN, $-L-OR_a$, $-L-SR_a$, $-L-NR_bR_c$, $C_{1-6}$ alkyl, $C_{1-6}$ haloalkyl, $C_{2-6}$ alkenyl, $C_{2-6}$ alkynyl, $-L-C_{3-10}$ cycloalkyl or -L-3- to 10-membered heterocyclyl;
- $R_1$ is selected from H, D, halogen, CN, $-L-OR_a$, $-L-SR_a$, $-L-NR_bR_c$, $C_{1-6}$ alkyl, $C_{1-6}$ haloalkyl, $C_{2-6}$ alkenyl, $C_{2-6}$ alkynyl, $-L-C_{3-10}$ cycloalkyl, -L-3- to 10-membered heterocyclyl, $-L-C_{6-10}$ aryl or -L-5- to 10-membered heteroaryl, which is optionally substituted with 1, 2 or 3 $R_{1s}$;
- $R_{1s}$ is independently selected from H, D, halogen, CN, $OR_a$, $SR_a$, $NR_bR_c$, $C_{1-6}$ alkyl, $C_{1-6}$ haloalkyl, $C_{2-6}$ alkenyl, $C_{2-6}$ alkynyl, $C_{3-10}$ cycloalkyl, 3- to 10-membered heterocyclyl, $C_{6-10}$ aryl or 5- to 10-membered heteroaryl;
- - - - represents a single bond or a double bond;
- $M_1$ is selected from N, C or $CR_5$;
- $M_2$ is N or $CR_6$;
- $R_5$ and $R_6$ are independently selected from H, D, halogen, $C_{1-6}$ alkyl or $C_{1-6}$ haloalkyl;
- $R_4$ is independently selected from H, D, halogen, CN, =O, $OR_a$, $SR_a$, $NR_bR_c$, $C_{1-6}$ alkyl, $C_{1-6}$ haloalkyl, $C_{3-7}$ cycloalkyl

or 3- to 7-membered heterocyclyl, which is optionally substituted with 1, 2 or 3 $R_{4s}$;

alternatively, $R_4$ located at the ortho position of $M_1$ and $R_2$ together with the atom to which they are attached form $C_{5-7}$ cycloalkyl or 5- to 7-membered heterocyclyl; alternatively, $R_4$ located at the ortho position of $M_1$ and $Z_3$ together with the atom to which they are attached form $C_{5-7}$ cycloalkyl or 5- to 7-membered heterocyclyl, which is optionally substituted with 1, 2 or 3 $R_{4s}$;

$R_{4s}$ is independently selected from H, D, halogen, CN, $C_{1-6}$ alkyl, $C_{1-6}$ haloalkyl, $C_{3-7}$ cycloalkyl or 3- to 7-membered heterocyclyl;

n is 0, 1, 2, 3 or 4;

$Z_1$ is selected from $CR_7$ or N;

$Z_2$ is selected from $CR_8$ or N;

$Z_3$ is selected from $CR_9$ or N;

$R_2$, $R_7$, $R_8$ and $R_9$ are independently selected from H, D, halogen, CN, $OR_a$, $SR_a$, $NR_bR_c$, $C_{1-6}$ alkyl, $C_{1-6}$ haloalkyl, $C_{3-7}$ cycloalkyl or 3- to 7-membered heterocyclyl, which is optionally substituted with 1, 2 or 3 $R_{2s}$;

$R_{2s}$ is independently selected from H, D, halogen, CN, $C_{1-6}$ alkyl, $C_{1-6}$ haloalkyl, $C_{3-7}$ cycloalkyl or 3- to 7-membered heterocyclyl;

$R_3$ is selected from H, $-L-OR_a$, $-L-SR_a$, $-L-NR_bR_c$, $C_{1-6}$ alkyl, $C_{1-6}$ haloalkyl, $C_{2-6}$ alkenyl, $C_{2-6}$ alkynyl, $-L-C_{3-10}$ cycloalkyl, -L-3- to 10-membered heterocyclyl, $-L-C_{6-10}$ aryl or -L-5- to 10-membered heteroaryl, which is optionally substituted with 1, 2 or 3 $R_{3s}$;

$R_{3s}$ is independently selected from H, D, halogen, CN, $OR_a$, $SR_a$, $NR_bR_c$, $C_{1-6}$ alkyl, $C_{1-6}$ haloalkyl, $C_{2-6}$ alkenyl, $C_{2-6}$ alkynyl, $C_{3-10}$ cycloalkyl, 3- to 10-membered heterocyclyl, $C_{6-10}$ aryl or 5- to 10-membered heteroaryl;

L is a chemical bond, $C_{1-6}$ alkylene, $C_{2-6}$ alkenylene or $C_{2-6}$ alkynylene, which is optionally substituted with 1, 2 or 3 groups selected from H, D, halogen, $C_{1-6}$ alkyl, $C_{1-6}$ haloalkyl, $C_{2-6}$ alkenyl or $C_{2-6}$ alkynyl;

$R_a$, $R_b$ and $R_c$ are each independently selected from H, $C_{1-6}$ alkyl, $C_{1-6}$ haloalkyl, $C_{3-10}$ cycloalkyl or 3- to 10-membered heterocyclyl; alternatively, $R_b$ and $R_c$ together with the atom to which they are attached form 5- to 10-membered heterocyclyl;

wherein each of the above-defined groups is optionally deuterated, up to fully deuterated.

In another aspect, the present invention provides a pharmaceutical composition comprising the compounds of the present invention, and optionally a pharmaceutically acceptable excipient, such as a carrier, adjuvant or vehicle.

In another aspect, the present invention provides the use of the compounds of the present invention or the pharmaceutical compositions of the present invention in the preparation of a medicament for treating and/or preventing a PARP-mediated disease, wherein preferably, the PARP is PARP1.

In another aspect, the present invention provides a method for treating and/or preventing a PARP-mediated disease in a subject, comprising administering to the subject the compounds of the present invention or the pharmaceutical compositions of the present invention, wherein preferably, the PARP is PARP1.

In another aspect, the present invention provides the compounds of the present invention or the pharmaceutical compositions of the present invention for use in the treatment and/or prevention of a PARP-mediated disease, wherein preferably, the PARP is PARP1.

In a specific embodiment, the present invention is useful in the treatment and/or prevention of cancer, ischemic diseases and neurodegenerative diseases.

In another specific embodiment, the cancer lacks an HR-dependent DNA DSB repair pathway.

In another specific embodiment, the cancer has a BRCA1- or BRCA2-deficient phenotype.

In another specific embodiment, the present invention is useful in the treatment and/or prevention of the following cancers: breast cancer, ovarian cancer, pancreatic cancer, prostate cancer, blood cancer, gastrointestinal cancer and lung cancer.

Definitions

Chemical definitions

Definitions of specific functional groups and chemical terms are described in more detail below.

When a numerical range is listed, every value and sub-range within the stated range are intended to be included. For example, "$C_{1-6}$ alkyl" includes $C_1$, $C_2$, $C_3$, $C_4$, $C_5$, $C_6$, $C_{1-6}$, $C_{1-5}$, $C_{1-4}$, $C_{1-3}$, $C_{1-2}$, $C_{2-6}$, $C_{2-5}$, $C_{2-4}$, $C_{2-3}$, $C_{3-6}$, $C_{3-5}$, $C_{3-4}$, $C_{4-6}$, $C_{4-5}$ and $C_{5-6}$ alkyl.

"$C_{1-6}$ alkyl" refers to a linear or branched saturated hydrocarbon group having 1 to 6 carbon atoms. In some embodiments, $C_{1-4}$ alkyl, $C_{1-3}$ alkyl and $C_{1-2}$ alkyl are preferred. Examples of $C_{1-6}$ alkyl include: methyl ($C_1$), ethyl ($C_2$), n-propyl ($C_3$), isopropyl ($C_3$), n-butyl ($C_4$), tert-butyl ($C_4$), sec-butyl ($C_4$), isobutyl ($C_4$), n-pentyl ($C_5$), 3- pentyl ($C_5$), pentyl ($C_5$), neopentyl ($C_5$), 3-methyl-2-butyl ($C_5$), tert-pentyl ($C_5$) and n-hexyl ($C_6$). The term "$C_{1-6}$ alkyl" further includes a

heteroalkyl group in which one or more (e.g., 1, 2, 3, or 4) carbon atoms are replaced with heteroatoms (e.g., oxygen, sulphur, nitrogen, boron, silicon, and phosphorus). The alkyl group may be optionally substituted with one or more substituents, for example, with 1 to 5 substituents, 1 to 3 substituents, or 1 substituent. Common alkyl abbreviations include: Me($-CH_3$), Et($-CH_2CH_3$), iPr($-CH(CH_3)_2$), nPr($-CH_2CH_2CH_3$), n-Bu($-CH_2CH_2CH_2CH_3$) or i-Bu($-CH_2CH(CH_3)_2$).

"$C_{2-6}$ alkenyl" refers to a linear or branched hydrocarbon group having 2 to 6 carbon atoms and at least one carbon-carbon double bond. In some embodiments, $C_{2-4}$ alkenyl is preferred. Examples of $C_{2-6}$ alkenyl include: ethenyl ($C_2$), 1-propenyl ($C_3$), 2-propenyl ($C_3$), 1-butenyl ($C_4$), 2-butenyl ($C_4$), butadienyl ($C_4$), pentenyl ($C_5$), pentadienyl ($C_5$), hexenyl ($C_6$), etc. The term "$C_{2-6}$ alkenyl" further includes a heteroalkenyl group in which one or more (e.g., 1, 2, 3, or 4) carbon atoms are replaced with heteroatoms (e.g., oxygen, sulphur, nitrogen, boron, silicon, and phosphorus). The alkenyl group may be optionally substituted with one or more substituents, for example, with 1 to 5 substituents, 1 to 3 substituents, or 1 substituent.

"$C_{2-6}$ alkynyl" refers to a linear or branched hydrocarbon group having 2 to 6 carbon atoms, at least one carbon-carbon triple bond, and optionally one or more carbon-carbon double bonds. In some embodiments, $C_{2-4}$ alkynyl is preferred. Examples of $C_{2-6}$ alkynyl include, but are not limited to: ethynyl ($C_2$), 1-propynyl ($C_3$), 2-propynyl ($C_3$), 1-butynyl ($C_4$), 2-butynyl ($C_4$), pentynyl ($C_5$), hexynyl ($C_6$), etc. The term "$C_{2-6}$ alkynyl" further includes a heteroalkynyl group in which one or more (e.g., 1, 2, 3, or 4) carbon atoms are replaced with heteroatoms (e.g., oxygen, sulphur, nitrogen, boron, silicon, and phosphorus). The alkynyl group may be optionally substituted with one or more substituents, for example, with 1 to 5 substituents, 1 to 3 substituents, or 1 substituent.

"$C_{1-6}$ alkylene" refers to a divalent group formed by removing another hydrogen from $C_{1-6}$ alkyl, and may be substituted or unsubstituted. In some embodiments, $C_{1-4}$ alkylene, $C_{2-4}$ alkylene and $C_{1-3}$ alkylene are preferred. The unsubstituted alkylene groups include, but are not limited to: methylene ($-CH_2-$), ethylene ($-CH_2CH_2-$), propylene ($-CH_2CH_2CH_2-$), butylene ($-CH_2CH_2CH_2CH_2-$), pentylene ($-CH_2CH_2CH_2CH_2CH_2-$), hexylene ($-CH_2CH_2CH_2CH_2CH_2CH_2-$), etc. Exemplary substituted alkylene groups, for example, the alkylene groups substituted with one or more alkyl (methyl) groups, include, but are not limited to: substituted methylene ($-CH(CH_3)$- or $-C(CH_3)_2-$), substituted ethylene ($-CH(CH_3)CH_2-$, $-CH_2CH(CH_3)-$, - $C(CH_3)_2CH_2-$, or $-CH_2C(CH_3)_2-$), substituted propylene ($-CH(CH_3)CH_2CH_2-$, - $CH_2CH(CH_3)CH_2-$, $-CH_2CH_2CH(CH_3)-$, $-C(CH_3)_2CH_2CH_2-$, $-CH_2C(CH_3)_2CH_2-$, or $-CH_2CH_2C(CH_3)_2-$), etc.

"$C_{2-6}$ alkenylene" refers to a divalent group formed by removing another hydrogen from $C_{2-6}$ alkenyl, and may be substituted or unsubstituted. **In** some embodiments, $C_{2-4}$ alkenylene is particularly preferred. Exemplary unsubstituted alkenylene groups include, but are not limited to: vinylene (-CH=CH-) and propenylene (such as $-CH=CHCH_2-$ or $-CH_2-CH=CH-$). Exemplary substituted alkenylene groups, for example, the alkenylene groups substituted with one or more alkyl (methyl) groups, include, but are not limited to: substituted ethylene ($-C(CH_3)=CH-$ or $-CH=C(CH_3)-$), substituted propenylene ($-C(CH_3)=CHCH_2-$, - $CH=C(CH_3)CH_2-$, $-CH=CHCH(CH_3)-$, $-CH=CHC(CH_3)_2-$, $-CH(CH_3)-CH=CH-$, - $C(CH_3)_2-CH=CH-$, $-CH_2-C(CH_3)=CH-$, or $-CH_2-CH=C(CH_3)-$), etc.

"$C_{2-6}$ alkynylene" refers to a divalent group formed by removing another hydrogen from $C_{2-6}$ alkynyl, and may be substituted or unsubstituted. **In** some embodiments, $C_{2-4}$ alkynylene is particularly preferred. Exemplary alkynylene groups include, but are not limited to: ethynylene ($-C\equiv C-$), substituted or unsubstituted propynylene ($-C\equiv CCH_2-$), etc.

"$C_{0-6}$ alkylene" refers to a chemical bond and the above-mentioned "$C_{1-6}$ alkylene", and "$C_{0-4}$ alkylene" refers to a chemical bond and the above-mentioned "$C_{1-4}$ alkylene".

"Halo" or "halogen" refers to fluorine (F), chlorine (Cl), bromine (Br) and iodine (I).

Therefore, "$C_{1-6}$ haloalkyl" refers to the above-mentioned "$C_{1-6}$ alkyl" which is substituted with one or more halogen groups. In some embodiments, $C_{1-4}$ haloalkyl is particularly preferred, $C_{1-3}$ haloalkyl is more preferred, and $C_{1-2}$ haloalkyl is even more preferred. Exemplary haloalkyl groups include, but are not limited to: $-CF_3$, - $CH_2F$, $-CHF_2$, $-CHFCH_2F$, $-CH_2CHF_2$, $-CF_2CF_3$, $-CCl_3$, $-CH_2Cl$, $-CHCl_2$, 2,2,2-trifluoro-1,1-dimethyl-ethyl, etc. The haloalkyl group may be substituted at any available point of attachment, for example, with 1 to 5 substituents, 1 to 3 substituents, or 1 substituent.

"$C_{1-6}$ deuterated alkyl" refers to the above-mentioned "$C_{1-6}$ alkyl" which is substituted with one or more deuterium groups. In some embodiments, $C_{1-4}$ deuterated alkyl is particularly preferred, $C_{1-2}$ deuterated alkyl is more preferred, and $C_{1-2}$ deuterated alkyl is even more preferred. Exemplary deuterated alkyl groups include, but are not limited to: $-CD_3$, $-CHD_2$, $-CH_2D$, $-CHDCH_2D$, $-CH_2CD_3$, - $CD_2CD_3$, etc. The deuterated alkyl group may be substituted at any available point of attachment, for example, with 1 to 5 substituents, 1 to 3 substituents, or 1 substituent.

"$C_{3-10}$ cycloalkyl" refers to a non-aromatic cyclic hydrocarbon group having 3 to 10 ring carbon atoms and zero heteroatoms, optionally containing 1, 2 or 3 double or triple bonds. In some embodiments, $C_{5-10}$ cycloalkyl, $C_{3-7}$ cycloalkyl and $C_{3-6}$ cycloalkyl are particularly preferred, and $C_{5-7}$ cycloalkyl and $C_{5-6}$ cycloalkyl are more preferred. The cycloalkyl group further includes a ring system in which the above-mentioned cycloalkyl ring is fused with one or more aryl or heteroaryl groups, wherein the point of attachment is on the cycloalkyl ring; and in such cases, the number of carbons continues to be indicative of the number of carbons in the cycloalkyl system. The cycloalkyl group further includes the above-mentioned cycloalkyl ring in which the substituents on any non-adjacent carbon atoms are connected to form a

bridge ring, together forming a polycyclic alkane that shares two or more carbon atoms. The cycloalkyl group further includes the above-mentioned cycloalkyl ring in which the substituents on the same carbon atom are connected to form a ring, together forming a polycyclic alkane that shares a common carbon atom. Exemplary cycloalkyl groups include, but are not limited to: cyclopropyl ($C_3$), cyclopropenyl ($C_3$), cyclobutyl ($C_4$), cyclobutenyl ($C_4$), cyclopentyl ($C_5$), cyclopentenyl ($C_5$), cyclohexyl ($C_6$), cyclohexenyl ($C_6$), cyclohexadienyl ($C_6$), cycloheptyl ($C_7$), cycloheptenyl ($C_7$), cycloheptadienyl ($C_7$), cycloheptatrienyl ($C_7$), etc. The cycloalkyl group may be optionally substituted with one or more substituents, for example, with 1 to 5 substituents, 1 to 3 substituents, or 1 substituent.

"$C_{3-10}$ cycloalkylene" refers to a divalent group formed by removing another hydrogen from $C_{3-10}$ cycloalkyl, and may be substituted or unsubstituted. In some embodiments, $C_{5-10}$ cycloalkylene, $C_{5-7}$ cycloalkylene, $C_{3-7}$ cycloalkylene, $C_{3-6}$ cycloalkylene and $C_{3-4}$ cycloalkylene are particularly preferred, and cyclopropylene is especially preferred.

"3- to 10-membered heterocyclyl" refers to a saturated or unsaturated group of a 3- to 10-membered non-aromatic ring system having ring carbon atoms and 1 to 5 ring heteroatoms, optionally containing 1, 2 or 3 double or triple bonds, wherein each heteroatom is independently selected from nitrogen, oxygen, sulphur, boron, phosphorus and silicon. In heterocyclyl groups containing one or more nitrogen atoms, the point of attachment may be a carbon or nitrogen atom, as valency permits. In some embodiments, 5- to 10-membered heterocyclyl is preferred, which is a 5- to 10-membered non-aromatic ring system having ring carbon atoms and 1 to 5 ring heteroatoms; in some embodiments, 3- to 7-membered heterocyclyl is preferred, which is a 3- to 7-membered non-aromatic ring system having ring carbon atoms and 1 to 4 ring heteroatoms; 5- to 7-membered heterocyclyl is preferred, which is a 5- to 7-membered non-aromatic ring system having ring carbon atoms and 1 to 3 ring heteroatoms; 3- to 6-membered heterocyclyl is preferred, which is a 3- to 6-membered non-aromatic ring system having ring carbon atoms and 1 to 3 ring heteroatoms; 4- to 6-membered heterocyclyl is preferred, which is a 4- to 6-membered non-aromatic ring system having ring carbon atoms and 1 to 3 ring heteroatoms; and 5- to 6-membered heterocyclyl is more preferred, which is a 5- to 6-membered non-aromatic ring system having ring carbon atoms and 1 to 3 ring heteroatoms. The heterocyclyl group further includes a ring system in which the above-mentioned heterocyclyl ring is fused with one or more cycloalkyl groups, wherein the point of attachment is on the heterocyclyl ring, or a ring system in which the above-mentioned heterocyclyl ring is fused with one or more aryl or heteroaryl groups, wherein the point of attachment is on the heterocyclyl ring; and in such cases, the number of ring members continues to be indicative of the number of ring members in the heterocyclyl ring system. The heterocyclyl group further includes the above-mentioned heterocyclyl ring in which the substituents on any non-adjacent carbon or nitrogen atoms are connected to form a bridge ring, together forming a polycyclic heteroalkane that shares two or more carbon or nitrogen atoms. The heterocyclyl group further includes the above-mentioned heterocyclyl ring in which the substituents on the same carbon atom are connected to form a ring, together forming a polycyclic heteroalkane that shares a common carbon atom. Exemplary 3-membered heterocyclyl groups containing one heteroatom include, but are not limited to: aziridinyl, oxiranyl and thiorenyl. Exemplary 4-membered heterocyclyl groups containing one heteroatom include, but are not limited to: azetidinyl, oxetanyl and thietanyl. Exemplary 5-membered heterocyclyl groups containing one heteroatom include, but are not limited to: tetrahydrofuryl, dihydrofuryl, 2,5-dihydrofuryl, tetrahydrothienyl, dihydrothienyl, pyrrolidyl, dihydropyrrolyl and pyrrolyl-2,5-dione. Exemplary 5-membered heterocyclyl groups containing two heteroatoms include, but are not limited to: pyrazolidinyl, dioxolanyl, oxasulphuranyl, disulphuranyl and oxazolidin-2-one. Exemplary 5-membered heterocyclyl groups containing three heteroatoms include, but are not limited to: triazolinyl, oxadiazolinyl and thiadiazolinyl. Exemplary 6-membered heterocyclyl groups containing one heteroatom include, but are not limited to: piperidyl, tetrahydropyranyl, dihydropyridyl and thianyl. Exemplary 6-membered heterocyclyl groups containing two heteroatoms include, but are not limited to: piperazinyl, morpholinyl, dithianyl and dioxanyl. Exemplary 6-membered heterocyclyl groups containing three heteroatoms include, but are not limited to: triazinanyl. Exemplary 7-membered heterocyclyl groups containing one heteroatom include, but are not limited to: azepanyl, oxepanyl and thiepanyl. Exemplary 5-membered heterocyclyl groups fused with a $C_6$ aryl ring (also referred to herein as 5,6-bicyclic heterocyclyl) include, but are not limited to: indolinyl, isoindolinyl, dihydrobenzofuryl, dihydrobenzothienyl, benzoxazolinonyl, etc. Exemplary 6-membered heterocyclyl groups fused with a $C_6$ aryl ring (also referred to herein as 6,6-bicyclic heterocyclyl) include, but are not limited to: tetrahydroquinolyl, tetrahydroisoquinolyl, etc. The heterocyclyl group further includes the case where the above-mentioned heterocyclyl group shares one or two atoms with a cycloalkyl group, a heterocyclyl group, an aryl group or a heteroaryl group to form a bridge ring or a spiro ring, and the shared atom may be a carbon or nitrogen atom, as valency permits. The heterocyclyl group further includes the case where the above-mentioned heterocyclyl and heterocyclyl groups may be optionally substituted with one or more substituents, for example, with 1 to 5 substituents, 1 to 3 substituents, or 1 substituent.

"$C_{6-10}$ aryl" refers to a group of a monocyclic or polycyclic (e.g., bicyclic) 4n+2 aromatic ring system (e.g., having 6 or 10 $\pi$ electrons shared in a cyclic arrangement) having 6-10 ring carbon atoms and zero heteroatoms. In some embodiments, the aryl group has six ring carbon atoms ("$C_6$ aryl"; e.g., phenyl). In some embodiments, the aryl group has ten ring carbon atoms ("$C_{10}$ aryl"; e.g., naphthyl, such as 1-naphthyl and 2-naphthyl). The aryl group further includes a ring system in which the above-mentioned aryl ring is fused with one or more cycloalkyl or heterocyclyl groups, and the point of attachment is on the aryl ring; and in such cases, the number of carbon atoms continues to be indicative of the number of

carbon atoms in the aryl ring system. The aryl group may be optionally substituted with one or more substituents, for example, with 1 to 5 substituents, 1 to 3 substituents, or 1 substituent.

"5- to 10-membered heteroaryl" refers to a group of a 5- to 10-membered monocyclic or bicyclic 4n+2 aromatic ring system (e.g., having 6 or 10 $\pi$ electrons shared in a cyclic arrangement) having ring carbon atoms and 1-4 ring heteroatoms, wherein each heteroatom is independently selected from nitrogen, oxygen and sulphur. In heteroaryl groups containing one or more nitrogen atoms, the point of attachment may be a carbon or nitrogen atom, as valency permits. The heteroaryl bicyclic ring system may include one or more heteroatoms in one or both rings. The heteroaryl group further includes a ring system in which the above-mentioned heteroaryl ring is fused with one or more cycloalkyl or heterocyclyl groups, and the point of attachment is on the heteroaryl ring; and in such cases, the number of carbon atoms continues to be indicative of the number of carbon atoms in the heteroaryl ring system. In some embodiments, 5- to 6-membered heteroaryl is particularly preferred, which is a 5- to 6-membered monocyclic or bicyclic 4n+2 aromatic ring system having ring carbon atoms and 1-4 ring heteroatoms. Exemplary 5-membered heteroaryl groups containing one heteroatom include, but are not limited to: pyrrolyl, furyl and thienyl. Exemplary 5-membered heteroaryl groups containing two heteroatoms include, but are not limited to: imidazolyl, pyrazolyl, oxazolyl, isoxazolyl, thiazolyl and isothiazolyl. Exemplary 5-membered heteroaryl groups containing three heteroatoms include, but are not limited to: triazolyl, oxadiazolyl (e.g., 1,2,4-oxadiazolyl) and thiadiazolyl. Exemplary 5-membered heteroaryl groups containing four heteroatoms include, but are not limited to: tetrazolyl. Exemplary 6-membered heteroaryl groups containing one heteroatom include, but are not limited to: pyridyl or pyridonyl. Exemplary 6-membered heteroaryl groups containing two heteroatoms include, but are not limited to: pyridazinyl, pyrimidyl and pyrazinyl. Exemplary 6-membered heteroaryl groups containing three or four heteroatoms respectively include, but are not limited to: triazinyl and tetrazinyl. Exemplary 7-membered heteroaryl groups containing one heteroatom include, but are not limited to: azacycloheptatrienyl, oxacycloheptatrienyl and thiacycloheptatrienyl. Exemplary 5,6-bicyclic heteroaryl groups include, but are not limited to: indolyl, isoindolyl, indazolyl, benzotriazolyl, benzothienyl, isobenzothienyl, benzofuryl, benzisofuryl, benzimidazolyl, benzoxazolyl, benzisoxazolyl, benzoxadiazolyl, benzothiazolyl, benzisothiazolyl, benzothiadiazolyl, indazinyl and purinyl. Exemplary 6,6-bicyclic heteroaryl groups include, but are not limited to: naphthyridinyl, pteridinyl, quinolyl, isoquinolyl, cinnolinyl, quinoxalinyl, phthalazinyl and quinazolinyl. The heteroaryl group may be optionally substituted with one or more substituents, for example, with 1 to 5 substituents, 1 to 3 substituents, or 1 substituent.

The divalent groups formed by removing another hydrogen from the groups such as alkyl, alkenyl, alkynyl, cycloalkyl, heterocyclyl, aryl and heteroaryl as defined above are collectively referred to as "subunits". Ring-forming groups such as cycloalkyl, heterocyclyl, aryl and heteroaryl are collectively referred to as "cyclic groups".

Alkyl, alkenyl, alkynyl, cycloalkyl, heterocyclyl, aryl and heteroaryl groups and the like as defined herein are optionally substituted groups.

Exemplary substituents on carbon atoms include, but are not limited to: halogen, -CN, $-NO_2$, $-N_3$, $-SO_2H$, $-SO_3H$, -OH, $-OR^{aa}$, $-ON(R^{bb})_2$, $-N(R^{bb})_2$, $-N(R^{bb})_3{}^+X^-$, $-N(OR^{cc})R^{bb}$, -SH, $-SR^{aa}$, $-SSR^{cc}$, $-C(=O)R^{aa}$, $-CO_2H$, -CHO, $-C(OR^{cc})_2$, $-CO_2R^{aa}$, $-OC(=O)R^{aa}$, $-OCO_2R^{aa}$, $-C(=O)N(R^{bb})_2$, $-OC(=O)N(R^{bb})_2$, $-NR^{bb}C(=O)R^{aa}$, $-NR^{bb}CO_2R^{aa}$, $-NR^{bb}C(=O)N(R^{bb})_2$, $-C(=NR^{bb})R^{aa}$, $-C(=NR^{bb})OR^{aa}$, $-OC(=NR^{bb})R^{aa}$, $-OC(=NR^{bb})OR^{aa}$, $-C(=NR^{bb})N(R^{bb})2$, $-OC(=NR^{bb})N(R^{bb})_2$, $-NR^{bb}C(=NR^{bb})N(R^{bb})2$, $-C(=O)NR^{bb}SO_2R^{aa}$, $-NR^{bb}SO_2R^{aa}$, $-SO_2N(R^{bb})_2$, $-SO_2R^{aa}$, $-SO_2OR^{aa}$, $-OSO_2R^{aa}$, $-S(=O)R^{aa}$, $-OS(=O)R^{aa}$, $-Si(R^{aa})_3$, $-OSi(R^{aa})_3$, $-C(=S)N(R^{bb})_2$, $-C(=O)SR^{aa}$, $-C(=S)SR^{aa}$, $-SC(=S)SR^{aa}$, $-SC(=O)SR^{aa}$, $-OC(=O)SR^{aa}$, $-SC(=O)OR^{aa}$, $-SC(=O)R^{aa}$, $-P(=O)_2R^{aa}$, $-OP(=O)_2R^{aa}$, $-P(=O)(R^{aa})_2$, $-OP(=O)(R^{aa})_2$, $-OP(=O)(OR^{cc})_2$, $-P(=O)_2N(R^{bb})_2$, $-OP(=O)_2N(R^{bb})_2$, $-P(=O)(NR^{bb})_2$, $-OP(=O)(NR^{bb})_2$, $-NR^{bb}(=O)(OR^{cc})_2$, $-NR^{bb}P(=O)(NR^{bb})_2$, $-P(R^{cc})_2$, $-P(R^{cc})_3$, $-OP(R^{cc})_2$, $-OP(R^{cc})_3$, $-B(R^{aa})_2$, $-B(OR^{cc})_2$, $-BR^{aa}(OR^{cc})$, alkyl, haloalkyl, alkenyl, alkynyl, cycloalkyl, heterocyclyl, aryl and heteroaryl, wherein each of alkyl, alkenyl, alkynyl, cycloalkyl, heterocyclyl, aryl and heteroaryl is independently substituted with 0, 1, 2, 3, 4 or 5 $R^{dd}$ groups;

alternatively, two geminal hydrogens on a carbon atom are substituted with group =O, =S, $=NN(R^{bb})_2$, $=NNR^{bb}C(=O)R^{aa}$, $=NNR^{bb}C(=O)OR^{aa}$, $=NNR^{bb}S(=O)_2R^{aa}$, $=NR^{bb}$ or $=NOR^{cc}$;

each $R^{aa}$ is independently selected from alkyl, haloalkyl, alkenyl, alkynyl, cycloalkyl, heterocyclyl, aryl and heteroaryl; alternatively, two $R^{aa}$ groups combine to form a heterocyclyl or heteroaryl ring, wherein each of alkyl, alkenyl, alkynyl, cycloalkyl, heterocyclyl, aryl and heteroaryl is independently substituted with 0, 1, 2, 3, 4 or 5 $R^{dd}$ groups;

each $R^{bb}$ is independently selected from: hydrogen, -OH, $-OR^{aa}$, $-N(R^{cc})_2$, -CN, $-C(=O)R^{aa}$, $-C(=O)N(R^{cc})_2$, $-CO_2R^{aa}$, $-SO_2R^{aa}$, $-C(=NR^{cc})OR^{aa}$, $-C(=NR^{cc})N(R^{cc})_2$, $-SO_2N(R^{cc})_2$, $-SO_2R^{cc}$, $-SO_2OR^{cc}$, $-SOR^{aa}$, $-C(=S)N(R^{cc})_2$, $-C(=O)SR^{cc}$, $-C(=S)SR^{cc}$, $-P(=O)_2R^{aa}$, $-P(=O)(R^{aa})_2$, $-P(=O)_2N(R^{cc})_2$, $-P(=O)(NR^{cc})_2$, alkyl, haloalkyl, alkenyl, alkynyl, cycloalkyl, heterocyclyl, aryl and heteroaryl; alternatively, two $R^{bb}$ groups combine to form a heterocyclyl or heteroaryl ring, wherein each of alkyl, alkenyl, alkynyl, cycloalkyl, heterocyclyl, aryl and heteroaryl is independently substituted with 0, 1, 2, 3, 4 or 5 $R^{dd}$ groups;

each $R^{cc}$ is independently selected from hydrogen, alkyl, haloalkyl, alkenyl, alkynyl, cycloalkyl, heterocyclyl, aryl and heteroaryl; alternatively, two $R^{cc}$ groups combine to form a heterocyclyl or heteroaryl ring, wherein each of alkyl, alkenyl, alkynyl, cycloalkyl, heterocyclyl, aryl and heteroaryl is independently substituted with 0, 1, 2, 3, 4 or 5 $R^{dd}$

groups;

each $R^{dd}$ is independently selected from: halogen, -CN, $-NO_2$, $-N_3$, $-SO_2H$, - $SO_3H$, -OH, $-OR^{ee}$, $-ON(R^{ff})_2$, $-N(R^{ff})_2$, $-N(R)_3^+X^-$, $-N(OR^{ee})R^{ff}$, -SH, $-SR^{ee}$, $-SSR^{ee}$, $-C(=O)R^{ee}$, $-CO_2H$, $-CO_2R^{ee}$, $-OC(=O)R^{ee}$, $-OCO_2R^{ee}$, $-C(=O)N(R^{ff})_2$, $-OC(=O)N(R\ )_2$, $-NR^{ff}C(=O)R^{ee}$, $-NR^{ff}CO_2R^{ee}$, $-NRC(=O)N(R^{ff})_2$, $-C(=NR^{ff})OR^{ee}$, $-OC(=NR^{ff)}R^{ee}$, $-OC(=NR^{ff})OR^{ee}$, $-C(=NR^{ff})N(R^{ff})_2$, $-OC(=NR^{ff})N(R^{ff})_2$, - $NR^{ff}C(=NR^{ff})N(R^{ff})_2$, $-NR^{ff}SO_2R^{ee}$, $-SO_2N(R^{ff})_2$, $-SO_2R^{ee}$, $-SO_2OR^{ee}$, $-OSO_2R^{ee}$, - $S(=O)R^{ee}$, $-Si(R^{ee})_3$, $-OSi(R^{ee})_3$, $-C(=S)N(R^{ff})_2$, $-C(=O)SR^{ee}$, $-C(=S)SR^{ee}$, - $SC(=S)SR^{ee}$, $-P(=O)_2R^{ee}$, $-P(=O)(R^{ee})_2$, $-OP(=O)(R^{ee})_2$, $-OP(=O)(OR^{ee})_2$, alkyl, haloalkyl, alkenyl, alkynyl, cycloalkyl, heterocyclyl, aryl or heteroaryl, wherein each of alkyl, alkenyl, alkynyl, cycloalkyl, heterocyclyl, aryl and heteroaryl is independently substituted with 0, 1, 2, 3, 4 or 5 $R^{gg}$ groups; alternatively, two geminal $R^{dd}$ substituents may combine to form =O or =S;

each $R^{ee}$ is independently selected from alkyl, haloalkyl, alkenyl, alkynyl, cycloalkyl, aryl, heterocyclyl and heteroaryl, wherein each of alkyl, alkenyl, alkynyl, cycloalkyl, heterocyclyl, aryl and heteroaryl is independently substituted with 0, 1, 2, 3, 4 or 5 $R^{gg}$ groups;

each $R^{ff}$ is independently selected from hydrogen, alkyl, haloalkyl, alkenyl, alkynyl, cycloalkyl, heterocyclyl, aryl and heteroaryl; alternatively, two $R^{ff}$ groups combine to form a heterocyclyl or heteroaryl ring, wherein each of alkyl, alkenyl, alkynyl, cycloalkyl, heterocyclyl, aryl and heteroaryl is independently substituted with 0, 1, 2, 3, 4 or 5 $R^{gg}$ groups;

each $R^{gg}$ is independently: halogen, -CN, $-NO_2$, $-N_3$, $-SO_2H$, $-SO_3H$, -OH, $-OC_{1-6}$ alkyl, $-ON(C_{1-6}$ alkyl$)_2$, $-N(C_{1-6}$ alkyl$)_2$, $-N(C_{1-6}$ alkyl$)_3^+X^-$, $-NH(C_{1-6}$ alkyl$)_2^+X^-$, - $NH_2(C_{1-6}$ alkyl$)^+X^-$, $-NH_3^+X^-$, $-N(OC_{1-6}$ alkyl$)(C_{1-6}$ alkyl), $-N(OH)(C_{1-6}$ alkyl), - NH(OH), -SH, $-SC_{1-6}$ alkyl, $-SS(C_{1-6}$ alkyl), $-C(=O)(C_{1-6}$ alkyl), $-CO_2H$, $-CO_2(C_{1-6}$ alkyl), $-OC(=O)(C_{1-6}$ alkyl), $-OCO_2(C_{1-6}$ alkyl), $-C(=O)NH_2$, $-C(=O)N(C_{1-6}$ alkyl$)_2$, $-OC(=O)NH(C_{1-6}$ alkyl), $-NHC(=O)(C_{1-6}$ alkyl), $-N(C_{1-6}$ alkyl) $C(=O)(C_{1-6}$ alkyl), - $NHCO_2(C_{1-6}$ alkyl), $-NHC(=O)N(C_{1-6}$ alkyl$)_2$, $-NHC(=O)NH(C_{1-6}$ alkyl), - $NHC(=O)NH_2$, -C(=NH) $O(C_{1-6}$ alkyl), $-OC(=NH)(C_{1-6}$ alkyl), $-OC(=NH)OC_{1-6}$ alkyl, $-C(=NH)N(C_{1-6}$ alkyl$)_2$, $-C(=NH)NH(C_{1-6}$ alkyl), -C(=NH) $NH_2$, - $OC(=NH)N(C_{1-6}$ alkyl$)_2$, $-OC(NH)NH(C_{1-6}$ alkyl), $-OC(NH)NH_2$, $-NHC(NH)N(C_{1-6}$ alkyl$)_2$, $-NHC(=NH)NH_2$, $-NHSO_2(C_{1-6}$ alkyl), $-SO_2N(C_{1-6}$ alkyl$)_2$, $-SO_2NH(C_{1-6}$ alkyl), $-SO_2NH_2$, $-SO_2C_{1-6}$ alkyl, $-SO_2OC_{1-6}$ alkyl, $-OSO_2C_{1-6}$ alkyl, $-SOC_{1-6}$ alkyl, - $Si(C_{1-6}$ alkyl$)_3$, $-OSi(C_{1-6}$ alkyl$)_3$, $-C(=S)N(C_{1-6}$ alkyl$)_2$, $C(=S)NH(C_{1-6}$ alkyl), $C(=S)NH_2$, -C(=O) $S(C_{1-6}$ alkyl), $-C(=S)SC_{1-6}$ alkyl, $-SC(=S)SC_{1-6}$ alkyl, $-P(=O)_2(C_{1-6}$ alkyl), $-P(=O)(C_{1-6}$ alkyl$)_2$, $-OP(=O)(C_{1-6}$ alkyl$)_2$, $-OP(=O)(OC_{1-6}$ alkyl$)_2$, $C_{1-6}$ alkyl, $C_{1-6}$ haloalkyl, $C_2$-$C_6$ alkenyl, $C_2$-$C_6$ alkynyl, $C_3$-$C_7$ cycloalkyl, $C_6$-$C_{10}$ aryl, $C_3$-$C_7$ heterocyclyl, or $C_5$-$C_{10}$ heteroaryl; alternatively, two geminal $R^{gg}$ substituents may combine to form =O or =S; wherein $X^-$ is a counter ion.

Exemplary substituents on nitrogen atoms include, but are not limited to: hydrogen, -OH, $-OR^{aa}$, $-N(R^{cc})_2$, -CN, $-C(=O)R^{aa}$, $-C(=O)N(R^{cc})_2$, $-CO_2R^{aa}$, $-SO_2R^{aa}$, $-C(=NR^{bb})R^{aa}$, $-C(=NR^{cc})OR^{aa}$, $-C(=NR^{cc})N(R^{cc})_2$, $-SO_2N(R^{cc})_2$, $-SO_2R^{cc}$, $-SO_2OR^{cc}$, $-SOR^{aa}$, $-C(=S)N(R^{cc})_2$, $-C(=O)SR^{cc}$, $-C(=S)SR^{cc}$, $-P(=O)_2R^{aa}$, $-P(=O)(R^{aa})_2$, - $P(=O)_2N(R^{cc})_2$, $-P(=O)(NR^{cc})_2$, alkyl, haloalkyl, alkenyl, alkynyl, cycloalkyl, heterocyclyl, aryl and heteroaryl; alternatively, two $R^{cc}$ groups attached to the nitrogen atom combine to form a heterocyclyl or heteroaryl ring, wherein each of alkyl, alkenyl, alkynyl, cycloalkyl, heterocyclyl, aryl and heteroaryl is independently substituted with 0, 1, 2, 3, 4 or 5 $R^{dd}$ groups, and wherein $R^{aa}$, $R^{bb}$, $R^{cc}$ and $R^{dd}$ are as described above.

Other definitions

The term "cancer" includes, but is not limited to the following cancers: heart: sarcomas (angiosarcoma, fibrosarcoma, rhabdomyosarcoma, and liposarcoma), myxoma, rhabdomyoma, fibroma, lipoma, and teratoma; lung: bronchogenic carcinoma (squamous cell, undifferentiated small cell, undifferentiated large cell, and adenocarcinoma), alveolar (bronchiolar) carcinoma, bronchial adenoma, sarcoma, lymphoma, chondroma, hamartoma, and mesothelioma; gastrointestinal tract: oesophagus (squamous cell carcinoma, adenocarcinoma, leiomyosarcoma, and lymphoma), stomach (carcinoma, lymphoma, and leiomyosarcoma), pancreas (ductal adenocarcinoma, insulinoma, glucagonoma, gastrinoma, carcinoid tumour, and haemangioma), small intestine (adenocarcinoma, lymphoma, carcinoid tumour, Kaposi's sarcoma, leiomyoma, haemangioma, lipoma, neurofibroma, and fibroma), and large intestine (adenocarcinoma, tubular adenoma, villous adenoma, hamartoma, and leiomyoma); urogenital tract: kidney (adenocarcinoma, Wilms' tumour (nephroblastoma), lymphoma, and leukaemia), bladder and urethra (squamous cell carcinoma, transitional cell carcinoma, and adenocarcinoma), prostate (adenocarcinoma and sarcoma), and testis (seminoma, teratoma, embryonal carcinoma, teratocarcinoma, choriocarcinoma, sarcoma, stromal cell carcinoma, fibroma, fibroadenoma, adenomatous tumour, and lipoma); liver: hepatoma (hepatocellular carcinoma), cholangiocarcinoma, hepatoblastoma, angiosarcoma, hepatocellular adenoma, and haemangioma; biliary tract: gallbladder cancer, ampullary cancer, and bile duct cancer; bone: osteosarcoma, fibrosarcoma, malignant fibrous histiocytoma, chondrosarcoma, Ewing's sarcoma, malignant lymphoma (reticular cell sarcoma), multiple myeloma, malignant giant cell chordoma, osteocartilaginous exostoses, benign chondroma, chondroblastoma, fibrochondroma, chondromyxoid fibroma, osteoid osteoma and giant cell tumour of bone; nervous system: skull (osteoma, haemangioma, granuloma, xanthoma, and osteitis deformans), meninges

(meningioma, meningosarcoma, and glioma), and brain (astrocytoma, medulloblastoma, glioma, ependymoma, germinoma (pinealoma), glioblastoma multiforme, oligodendroglioma, schwannoma, retinoblastoma, congenital tumour, spinal neurofibroma, meningioma, glioma, and sarcoma); gynaecology: uterus (endometrial cancer), cervix (cervical cancer, preneoplastic cervical dysplasia, etc.), ovary (ovarian cancer, serous cystadenocarcinoma, mucinous cystadenocarcinoma, unclassified cancer, granulosa cell tumour, Sertoli-Leydig cell tumour, dysgerminoma, and malignant teratoma), vulva (squamous cell carcinoma, intraepithelial carcinoma, adenocarcinoma, fibrosarcoma, and melanoma), vagina (clear cell carcinoma, squamous cell carcinoma, and botryoid sarcoma (embryonal rhabdomyosarcoma)), and fallopian tube (cancer); haematology: blood (myeloid leukaemia (acute and chronic), acute lymphocytic leukaemia, chronic lymphocytic leukaemia, diffuse large B-cell lymphoma, mantle cell lymphoma (MCL), follicular lymphoma, myeloproliferative disorders, multiple myeloma, and myelodysplastic syndrome), Hodgkin’s disease, and non-Hodgkin’s lymphoma (malignant lymphoma); skin: malignant melanoma, basal cell carcinoma, squamous cell carcinoma, Kaposi’s sarcoma, nevus dysplasia, lipoma, haemangioma, dermatofibroma, keloid, and psoriasis; and adrenal glands: neuroblastoma.

In one embodiment, the term "cancer" includes, but is not limited to the following cancers: breast cancer, ovarian cancer, pancreatic cancer, prostate cancer, blood cancer, gastrointestinal cancer and lung cancer.

As used herein, the term "treating" relates to reversing, alleviating, inhibiting the progress of, or preventing the disorder or condition to which such term applies, or one or more symptoms of such disorder or condition. As used herein, the noun "treatment" relates to the action of the verb treat, which is as just defined.

As used herein, the term "pharmaceutically acceptable salt" refers to those carboxylate salts and amino acid addition salts of the compounds of the present invention which, within the scope of sound medical judgment, are suitable for use in contact with the tissues of patients without undue toxicity, irritation, allergic response, etc., are commensurate with a reasonable benefit/risk ratio, and are effective for their intended use, including (where possible) zwitterionic forms of the compounds of the present invention.

Pharmaceutically acceptable base addition salts are formed with metals or amines, such as alkali metal and alkaline earth metal hydroxides or organic amines. Examples of metals used as cations include sodium, potassium, magnesium, calcium, etc. Examples of suitable amines are N,N’-dibenzylethylenediamine, chloroprocaine, choline, diethanolamine, ethylenediamine, N-methylglucamine and procaine.

The base addition salts of acidic compounds can be prepared by contacting the free acid form with a sufficient amount of the desired base to form the salt in a conventional manner. The free acid can be regenerated by contacting the salt form with an acid and then isolating the free acid in a conventional manner. The free acid forms differ somewhat in certain physical properties from their respective salt forms, for example, in solubility in polar solvents. However, for the purposes of the present invention, the salts are equivalent to their respective free acids.

Salts may be sulphates, pyrosulphates, bisulphates, sulphites, bisulphites, nitrates, phosphates, monohydrogenphosphates, dihydrogenphosphates, metaphosphates, pyrophosphates, chlorides, bromides, and iodides prepared from inorganic acids such as hydrochloric acid, nitric acid, sulphuric acid, hydrobromic acid, hydroiodic acid, and phosphoric acid. Representative salts include: hydrobromides, hydrochlorides, sulphates, bisulphates, nitrates, acetates, oxalates, valerates, oleates, palmitates, stearates, laurates, borates, benzoates, lactates, phosphates, toluenesulfonates, citrates, maleates, fumarates, succinates, tartrates, naphthoates, methanesulfonates, glucoheptonates, lactobionates, lauryl sulfonates, isethionates, etc. Salts may also be prepared from organic acids such as aliphatic mono- and dicarboxylic acids, phenyl-substituted alkanoic acids, hydroxyalkanoic acids, alkanedioic acids, aromatic acids, and aliphatic and aromatic sulfonic acids. Representative salts include acetates, propionates, octanoates, isobutyrates, oxalates, malonates, succinates, suberates, sebacates, fumarates, maleates, mandelates, benzoates, chlorobenzoates, methylbenzoates, dinitrobenzoates, naphthoates, benzenesulfonates, toluenesulfonates, phenylacetates, citrates, lactates, maleates, tartrates, methanesulfonates, etc. Pharmaceutically acceptable salts may include cations based on alkali metals and alkaline earth metals, such as sodium, lithium, potassium, calcium and magnesium, as well as non-toxic ammonium, quaternary ammonium and amine cations, including but not limited to ammonium, tetramethylammonium, tetraethylammonium, methylamine, dimethylamine, trimethylamine, triethylamine, ethylamine, etc. Also contemplated are salts of amino acids, such as argininates, gluconates and galacturonates (see, e.g., Berge S.M. et al., "Pharmaceutical Salts," J. Pharm. Sci., 1977; 66: 1-19, incorporated herein by reference).

"Subjects" to whom the administration is carried out include, but are not limited to: humans (i.e., males or females of any age group, such as paediatric subjects (e.g., infants, children and adolescents) or adult subjects (e.g., young adults, middle-aged adults or elderly adults)), and/or non-human animals, for example, mammals, such as primates (e.g., cynomolgus monkeys and rhesus monkeys), cattle, pigs, horses, sheep, goats, rodents, cats and/or dogs. In some embodiments, the subject is a human. In some embodiments, the subject is a non-human animal. The terms "human", "patient" and "subject" are used interchangeably herein.

The terms "disease", "disorder" and "condition" are used interchangeably herein.

Unless otherwise indicated, the term "treatment" as used herein includes the effects that occur when a subject has a specific disease, disorder or condition, which reduce the severity of the disease, disorder or condition, or delay or slow down the development of the disease, disorder or condition ("therapeutic treatment"), and also includes the effects

that occur before a subject develops a specific disease, disorder or condition ("prophylactic treatment").

Generally, an "effective amount" of a compound is an amount sufficient to elicit a desired biological response. As will be appreciated by those of ordinary skill in the art, the effective amount of the compounds of the present invention may vary depending on factors such as the biological target, the pharmacokinetics of the compound, the disease being treated, the mode of administration, and the age, health condition and symptoms of the subject. The effective amount includes a therapeutically effective amount and a prophylactically effective amount.

Unless otherwise indicated, the "therapeutically effective amount" of the compound as used herein is an amount sufficient to provide a therapeutic benefit in the treatment of a disease, disorder or condition, or an amount that delays or minimizes one or more symptoms associated with the disease, disorder or condition. The therapeutically effective amount of the compound refers to an amount of a therapeutic agent that, when used alone or in combination with other therapies, provides a therapeutic benefit in the treatment of a disease, disorder or condition. The term "therapeutically effective amount" may include an amount that improves the overall therapy, reduces or avoids the symptoms or causes of a disease or condition, or enhances the therapeutic effect of an additional therapeutic agent.

Unless otherwise indicated, the "prophylactically effective amount" of the compound as used herein is an amount sufficient to prevent a disease, disorder or condition, or an amount sufficient to prevent one or more symptoms associated with the disease, disorder or condition, or an amount to prevent the recurrence of the disease, disorder or condition. The prophylactically effective amount of the compound refers to an amount of a therapeutic agent that, when used alone or in combination with other agents, provides a prophylactic benefit in the prevention of a disease, disorder or condition. The term "prophylactically effective amount" may include an amount that improves the overall prevention or enhances the prophylactic effect of other prophylactic agents.

"Combination" and related terms refer to the simultaneous or sequential administration of the compounds of the present invention and an additional therapeutic agent. For example, the compounds of the present invention may be administered simultaneously or sequentially with an additional therapeutic agent in separate unit dosage forms, or may be administered simultaneously with an additional therapeutic agent in a single unit dosage form.

## Brief Description of the Drawings

FIG. 1 shows the effect curve of the compounds of the present invention on PARP1/2-DNA trapping at 2 hours.

FIG. 2 shows a time-effect curve of the compounds of the present invention on PARP1/2-DNA trapping at different time points.

FIG. 3 shows the effect of the compounds of the present invention on the change of tumour volume in subcutaneous xenograft tumours of MDA-MB-436 mice.

FIG. 4 shows the induction of apoptosis in DLD-1 *BRCA2*$^{-/-}$ cells by the compounds of the present invention in three different experiments.

FIG. 5 shows the expression levels of cleaved Caspase-3 and Caspase-3 proteins in tumour tissues.

FIG. 6 shows the effect of the compound of Example 4 and the reference compound AZD5305 on the Cleaved Caspase-3 protein in tumour tissues at the same dosage (*, $p < 0.05$; by one-way ANOVA, there is no statistically significant difference among other groups).

## Detailed Description of Embodiments

Herein, "the compounds of the present invention" refer to compounds of formula (I), formula (II), etc., or pharmaceutically acceptable salts, isotopic variants, tautomers, stereoisomers, prodrugs, polymorphs, hydrates or solvates thereof.

Herein, the compounds are named using the standard nomenclature. For compounds having asymmetric centres, it should be understood that all optical isomers and mixtures thereof are intended to be encompassed, unless otherwise stated. Furthermore, unless otherwise specified, all isomeric compounds encompassed by the present invention may occur in both the Z and E forms of carbon-carbon double bonds. For compounds that exist in different tautomeric forms, a compound as described is not limited to any specific tautomer but is intended to encompass all tautomeric forms.

In one embodiment, the present invention relates to a compound of formula (I), or a pharmaceutically acceptable salt, an isotopic variant, a tautomer, a stereoisomer, a prodrug, a polymorph, a hydrate or a solvate thereof:

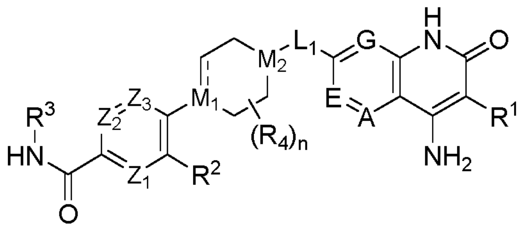

(I)

wherein

$L_1$ is selected from CRR', O, S, NH, -C(O)-, -S(O)- or -$S(O)_2$-;
R and R' are independently selected from H, D, halogen, $C_{1-6}$ alkyl or $C_{1-6}$ haloalkyl;
A is selected from $CR_A$ or N;
E is selected from $CR_E$ or N;
G is selected from $CR_G$ or N;
$R_A$, $R_E$ and $R_G$ are independently selected from H, D, halogen, CN, -L-$OR_a$, - L-$SR_a$, -L-$NR_bR_c$, $C_{1-6}$ alkyl, $C_{1-6}$ haloalkyl, $C_{2-6}$ alkenyl, $C_{2-6}$ alkynyl, -L-$C_{3-10}$ cycloalkyl or -L-3- to 10-membered heterocyclyl;
$R_1$ is selected from H, D, halogen, CN, -L-$OR_a$, -L-$SR_a$, -L-$NR_bR_c$, $C_{1-6}$ alkyl, $C_{1-6}$ haloalkyl, $C_{2-6}$ alkenyl, $C_{2-6}$ alkynyl, -L-$C_{3-10}$ cycloalkyl, -L-3- to 10-membered heterocyclyl, -L-$C_{6-10}$ aryl or -L-5- to 10-membered heteroaryl, which is optionally substituted with 1, 2 or 3 $R_{1s}$;
$R_{1s}$ is independently selected from H, D, halogen, CN, $OR_a$, $SR_a$, $NR_bR_c$, $C_{1-6}$ alkyl, $C_{1-6}$ haloalkyl, $C_{2-6}$ alkenyl, $C_{2-6}$ alkynyl, $C_{3-10}$ cycloalkyl, 3- to 10-membered heterocyclyl, $C_{6-10}$ aryl or 5- to 10-membered heteroaryl;
- - - represents a single bond or a double bond;
$M_1$ is selected from N, C or $CR_5$;
$M_2$ is N or $CR_6$;
$R_5$ and $R_6$ are independently selected from H, D, halogen, $C_{1-6}$ alkyl or $C_{1-6}$ haloalkyl;
$R_4$ is independently selected from H, D, halogen, CN, =O, $OR_a$, $SR_a$, $NR_bR_c$, $C_{1-6}$ alkyl, $C_{1-6}$ haloalkyl, $C_{3-7}$ cycloalkyl or 3- to 7-membered heterocyclyl, which is optionally substituted with 1, 2 or 3 $R_{4s}$;
alternatively, $R_4$ located at the ortho position of $M_1$ and $R_2$ together with the atom to which they are attached form $C_{5-7}$ cycloalkyl or 5- to 7-membered heterocyclyl; alternatively, $R_4$ located at the ortho position of $M_1$ and $Z_3$ together with the atom to which they are attached form $C_{5-7}$ cycloalkyl or 5- to 7-membered heterocyclyl, which is optionally substituted with 1, 2 or 3 $R_{4s}$;
$R_{4s}$ is independently selected from H, D, halogen, CN, $C_{1-6}$ alkyl, $C_{1-6}$ haloalkyl, $C_{3-7}$ cycloalkyl or 3- to 7-membered heterocyclyl;
n is 0, 1, 2, 3 or 4;
$Z_1$ is selected from $CR_7$ or N;
$Z_2$ is selected from $CR_8$ or N;
$Z_3$ is selected from $CR_9$ or N;
$R_2$, $R_7$, $R_8$ and $R_9$ are independently selected from H, D, halogen, CN, $OR_a$, $SR_a$, $NR_bR_c$, $C_{1-6}$ alkyl, $C_{1-6}$ haloalkyl, $C_{3-7}$ cycloalkyl or 3- to 7-membered heterocyclyl, which is optionally substituted with 1, 2 or 3 $R_{2s}$;
$R_{2s}$ is independently selected from H, D, halogen, CN, $C_{1-6}$ alkyl, $C_{1-6}$ haloalkyl, $C_{3-7}$ cycloalkyl or 3- to 7-membered heterocyclyl;
$R_3$ is selected from H, -L-$OR_a$, -L-$SR_a$, -L-$NR_bR_c$, $C_{1-6}$ alkyl, $C_{1-6}$ haloalkyl, $C_{2-6}$ alkenyl, $C_{2-6}$ alkynyl, -L-$C_{3-10}$ cycloalkyl, -L-3- to 10-membered heterocyclyl, -L-$C_{6-10}$ aryl or -L-5- to 10-membered heteroaryl, which is optionally substituted with 1, 2 or 3 $R_{3s}$;
$R_{3s}$ is independently selected from H, D, halogen, CN, $OR_a$, $SR_a$, $NR_bR_c$, $C_{1-6}$ alkyl, $C_{1-6}$ haloalkyl, $C_{2-6}$ alkenyl, $C_{2-6}$ alkynyl, $C_{3-10}$ cycloalkyl, 3- to 10-membered heterocyclyl, $C_{6-10}$ aryl or 5- to 10-membered heteroaryl;
L is a chemical bond, $C_{1-6}$ alkylene, $C_{2-6}$ alkenylene or $C_{2-6}$ alkynylene, which is optionally substituted with 1, 2 or 3 groups selected from H, D, halogen, $C_{1-6}$ alkyl, $C_{1-6}$ haloalkyl, $C_{2-6}$ alkenyl or $C_{2-6}$ alkynyl;
$R_a$, $R_b$ and $R_c$ are each independently selected from H, $C_{1-6}$ alkyl, $C_{1-6}$ haloalkyl, $C_{3-10}$ cycloalkyl or 3- to 10-membered heterocyclyl; alternatively, $R_b$ and $R_c$ together with the atom to which they are attached form 5- to 10-membered heterocyclyl;
wherein each of the above-defined groups is optionally deuterated, up to fully deuterated.

$L_1$

In one embodiment, $L_1$ is CRR', such as $CH_2$; in another embodiment, $L_1$ is O; in another embodiment, $L_1$ is S; in

another embodiment, $L_1$ is NH; in another embodiment, $L_1$ is -C(O)-; in another embodiment, $L_1$ is -S(O)-; in another embodiment, $L_1$ is $-S(O)_2-$.

In a more specific embodiment, $L_1$ is selected from CRR', O, S, NH or -C(O)-; in another more specific embodiment, $L_1$ is CRR' or -C(O)-; in another more specific embodiment, $L_1$ is CRR'; in another more specific embodiment, $L_1$ is $CH_2$.

R and R'

In one embodiment, R is H; in another embodiment, R is D; in another embodiment, R is halogen; in another embodiment, R is $C_{1-6}$ alkyl, such as $C_{1-3}$ alkyl; in another embodiment, R is $C_{1-6}$ haloalkyl, such as $C_{1-3}$ haloalkyl.

In one embodiment, R' is H; in another embodiment, R' is D; in another embodiment, R' is halogen; in another embodiment, R' is $C_{1-6}$ alkyl, such as $C_{1-3}$ alkyl; in another embodiment, R' is $C_{1-6}$ haloalkyl, such as $C_{1-3}$ haloalkyl.

In a more specific embodiment, R and R' are independently selected from H, D, $C_{1-6}$ alkyl or $C_{1-6}$ haloalkyl; in another more specific embodiment, R and R' are independently selected from H, D, $C_{1-3}$ alkyl or $C_{1-3}$ haloalkyl; in another more specific embodiment, R and R' are independently H or D; in another more specific embodiment, R and R' are H.

A, E and G

In one embodiment, A is $CR_A$; in another embodiment, A is N.

In one embodiment, E is $CR_E$; in another embodiment, E is N.

In one embodiment, G is $CR_G$; in another embodiment, G is N.

In a more specific embodiment, A is N; in another more specific embodiment, E is $CR_E$; in another more specific embodiment, G is $CR_G$; in another more specific embodiment,

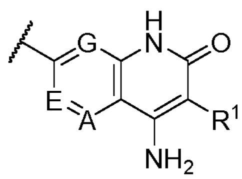
is
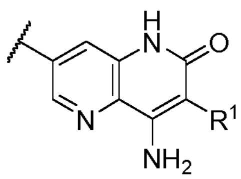
.

$R_A$, $R_E$ and $R_G$

In one embodiment, $R_A$ is H; in another embodiment, $R_A$ is D; in another embodiment, $R_A$ is halogen; in another embodiment, $R_A$ is CN; in another embodiment, $R_A$ is $-L-OR_a$, preferably $OR_a$; in another embodiment, $R_A$ is $-L-SR_a$, preferably $SR_a$; in another embodiment, $R_A$ is $-L-NR_bR_c$, preferably $NR_bR_c$; in another embodiment, $R_A$ is $C_{1-6}$ alkyl; in another embodiment, $R_A$ is $C_{1-6}$ haloalkyl; in another embodiment, $R_A$ is $C_{2-6}$ alkenyl; in another embodiment, $R_A$ is $C_{2-6}$ alkynyl; in another embodiment, $R_A$ is $-L-C_{3-10}$ cycloalkyl, preferably $C_{3-10}$ cycloalkyl, and preferably $C_{3-7}$ cycloalkyl; in another embodiment, $R_A$ is -L-3- to 10-membered heterocyclyl, preferably 3- to 10-membered heterocyclyl, and preferably 3- to 7-membered heterocyclyl.

In one embodiment, $R_E$ is H; in another embodiment, $R_E$ is D; in another embodiment, $R_E$ is halogen; in another embodiment, $R_E$ is CN; in another embodiment, $R_E$ is $-L-OR_a$, preferably $OR_a$; in another embodiment, $R_E$ is $-L-SR_a$, preferably $SR_a$; in another embodiment, $R_E$ is $-L-NR_bR_c$, preferably $NR_bR_c$; in another embodiment, $R_E$ is $C_{1-6}$ alkyl; in another embodiment, $R_E$ is $C_{1-6}$ haloalkyl; in another embodiment, $R_E$ is $C_{2-6}$ alkenyl; in another embodiment, $R_E$ is $C_{2-6}$ alkynyl; in another embodiment, $R_E$ is $-L-C_{3-10}$ cycloalkyl, preferably $C_{3-10}$ cycloalkyl, and preferably $C_{3-7}$ cycloalkyl; in another embodiment, $R_E$ is -L-3- to 10-membered heterocyclyl, preferably 3- to 10-membered heterocyclyl, and preferably 3- to 7-membered heterocyclyl.

In one embodiment, $R_G$ is H; in another embodiment, $R_G$ is D; in another embodiment, $R_G$ is halogen; in another embodiment, $R_G$ is CN; in another embodiment, $R_G$ is $-L-OR_a$, preferably $OR_a$; in another embodiment, $R_G$ is $-L-SR_a$, preferably $SR_a$; in another embodiment, $R_G$ is $-L-NR_bR_c$, preferably $NR_bR_c$; in another embodiment, $R_G$ is $C_{1-6}$ alkyl; in another embodiment, $R_G$ is $C_{1-6}$ haloalkyl; in another embodiment, $R_G$ is $C_{2-6}$ alkenyl; in another embodiment, $R_G$ is $C_{2-6}$ alkynyl; in another embodiment, $R_G$ is $-L-C_{3-10}$ cycloalkyl, preferably $C_{3-10}$ cycloalkyl, and preferably $C_{3-7}$ cycloalkyl; in another embodiment, $R_G$ is -L-3- to 10-membered heterocyclyl, preferably 3- to 10-membered heterocyclyl, and preferably 3- to 7-membered heterocyclyl.

In a more specific embodiment, $R_A$, $R_E$ and $R_G$ are independently selected from H, D, halogen, CN, $-L-OR_a$, $-L-SR_a$, $-L-NR_bR_c$, $C_{1-6}$ alkyl, $C_{1-6}$ haloalkyl, $-L-C_{3-7}$ cycloalkyl or -L-3- to 7-membered heterocyclyl; in another more specific embodiment, $R_A$, $R_E$ and $R_G$ are independently selected from H, D, halogen, CN, $OR_a$, $NR_bR_c$, $C_{1-6}$ alkyl, $C_{1-6}$ haloalkyl, $C_{3-7}$ cycloalkyl or 3- to 7-membered heterocyclyl; in another more specific embodiment, $R_A$, $R_E$ and $R_G$ are independently

selected from H, D, halogen, $C_{1-6}$ alkyl or $C_{1-6}$ haloalkyl; in another more specific embodiment, $R_A$, $R_E$ and $R_G$ are independently H or D; in another more specific embodiment, $R_A$, $R_E$ and $R_G$ are H.

$R_1$

In one embodiment, $R_1$ is H; in another embodiment, $R_1$ is D; in another embodiment, $R_1$ is halogen; in another embodiment, $R_1$ is CN; in another embodiment, $R_1$ is -L-$OR_a$, preferably $OR_a$; in another embodiment, $R_1$ is -L-$SR_a$, preferably $SR_a$; in another embodiment, $R_1$ is -L-$NR_bR_c$, preferably $NR_bR_c$; in another embodiment, $R_1$ is $C_{1-6}$ alkyl, such as Me, such as Et; in another embodiment, $R_1$ is $C_{1-6}$ haloalkyl; in another embodiment, $R_1$ is $C_{2-6}$ alkenyl; in another embodiment, $R_1$ is $C_{2-6}$ alkynyl; in another embodiment, $R_1$ is -L-$C_{3-10}$ cycloalkyl, preferably $C_{3-10}$ cycloalkyl, and preferably $C_{3-7}$ cycloalkyl; in another embodiment, $R_1$ is -L-3- to 10-membered heterocyclyl, preferably 3- to 10-membered heterocyclyl, and preferably 3- to 7-membered heterocyclyl; in another embodiment, $R_1$ is -L-$C_{6-10}$ aryl, preferably $C_{6-10}$ aryl, and preferably phenyl; in another embodiment, $R_1$ is - L-5- to 10-membered heteroaryl, preferably 5- to 10-membered heteroaryl, and preferably 5- to 6-membered heteroaryl; in another embodiment, $R_1$ is optionally substituted with 1, 2 or 3 $R_{1s}$.

In a more specific embodiment, $R_1$ is selected from H, D, halogen, CN, -L-$OR_a$, -L-$SR_a$, -L-$NR_bR_c$, $C_{1-6}$ alkyl, $C_{1-6}$ haloalkyl, -L-$C_{3-10}$ cycloalkyl, -L-3- to 10-membered heterocyclyl, -L-$C_{6-10}$ aryl or -L-5- to 10-membered heteroaryl; in another more specific embodiment, $R_1$ is selected from H, D, halogen, CN, $OR_a$, $NR_bR_c$, $C_{1-6}$ alkyl, $C_{1-6}$ haloalkyl, $C_{3-10}$ cycloalkyl, 3- to 10-membered heterocyclyl, $C_{6-10}$ aryl or 5- to 10-membered heteroaryl; in another more specific embodiment, $R_1$ is selected from H, D, halogen, CN, $OR_a$, $C_{1-6}$ alkyl, $C_{1-6}$ haloalkyl, $C_{3-7}$ cycloalkyl or 3- to 7-membered heterocyclyl; in another more specific embodiment, $R_1$ is selected from H, D, halogen, $C_{1-6}$ alkyl or $C_{1-6}$ haloalkyl, preferably selected from H, D, $C_{1-6}$ alkyl or $C_{1-6}$ haloalkyl; in another more specific embodiment, $R_1$ is $C_{1-6}$ alkyl or $C_{1-6}$ haloalkyl, preferably Et.

$R_{1s}$

In one embodiment, $R_{1s}$ is H; in another embodiment, $R_{1s}$ is D; in another embodiment, $R_{1s}$ is halogen; in another embodiment, $R_{1s}$ is CN; in another embodiment, $R_{1s}$ is $OR_a$; in another embodiment, $R_{1s}$ is $SR_a$; in another embodiment, $R_{1s}$ is $NR_bR_c$; in another embodiment, $R_{1s}$ is $C_{1-6}$ alkyl; in another embodiment, $R_{1s}$ is $C_{1-6}$ haloalkyl; in another embodiment, $R_{1s}$ is $C_{2-6}$ alkenyl; in another embodiment, $R_{1s}$ is $C_{2-6}$ alkynyl; in another embodiment, $R_{1s}$ is $C_{3-10}$ cycloalkyl, preferably $C_{3-7}$ cycloalkyl; in another embodiment, $R_{1s}$ is 3- to 10-membered heterocyclyl, preferably 3- to 7-membered heterocyclyl; in another embodiment, $R_{1s}$ is $C_{6-10}$ aryl, preferably phenyl; in another embodiment, $R_{1s}$ is 5- to 10-membered heteroaryl, preferably 5- to 6-membered heteroaryl.

In a more specific embodiment, $R_{1s}$ is independently selected from H, D, halogen, CN, $OR_a$, $NR_bR_c$, $C_{1-6}$ alkyl, $C_{1-6}$ haloalkyl, $C_{3-10}$ cycloalkyl, 3- to 10-membered heterocyclyl, $C_{6-10}$ aryl or 5- to 10-membered heteroaryl; in another more specific embodiment, $R_{1s}$ is independently selected from H, D, halogen, CN, $C_{1-6}$ alkyl, $C_{1-6}$ haloalkyl, $C_{3-7}$ cycloalkyl or 3- to 7-membered heterocyclyl; in another more specific embodiment, $R_{1s}$ is independently selected from H, D, halogen, $C_{1-6}$ alkyl or $C_{1-6}$ haloalkyl; in another more specific embodiment, $R_{1s}$ is independently selected from H, D, $C_{1-6}$ alkyl or $C_{1-6}$ haloalkyl.

In one embodiment, - - - is a single bond; in another embodiment, - - - is a double bond.

$M_1$ and $M_2$

In one embodiment, $M_1$ is N; in another embodiment, $M_1$ is C; in another embodiment, $M_1$ is $CR_5$.

In one embodiment, $M_2$ is N; in another embodiment, $M_2$ is $CR_6$.

In a more specific embodiment, $M_1$ is selected from N or $CR_5$; in another more specific embodiment, $M_1$ is N; in another more specific embodiment, $M_2$ is N; in another more specific embodiment,

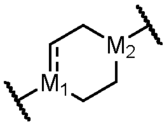 is 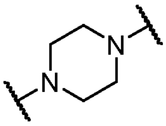.

$R_5$ and $R_6$

In one embodiment, $R_5$ is H; in another embodiment, $R_5$ is D; in another embodiment, $R_5$ is halogen; in another embodiment, $R_5$ is $C_{1-6}$ alkyl; in another embodiment, $R_5$ is $C_{1-6}$ haloalkyl.

In one embodiment, $R_6$ is H; in another embodiment, $R_6$ is D; in another embodiment, $R_6$ is halogen; in another embodiment, $R_6$ is $C_{1-6}$ alkyl; in another embodiment, $R_6$ is $C_{1-6}$ haloalkyl.

In a more specific embodiment, $R_5$ and $R_6$ are independently selected from H, D, $C_{1-6}$ alkyl or $C_{1-6}$ haloalkyl; in another more specific embodiment, $R_5$ and $R_6$ are H or D; in another more specific embodiment, $R_5$ and $R_6$ are H.

$R_4$

In one embodiment, $R_4$ is H; In another embodiment, $R_4$ is D; in another embodiment, $R_4$ is halogen; in another embodiment, $R_4$ is CN; in another embodiment, $R_4$ is =O; in another embodiment, $R_4$ is $OR_a$; in another embodiment, $R_4$ is $SR_a$; in another embodiment, $R_4$ is $NR_bR_c$; in another embodiment, $R_4$ is $C_{1-6}$ alkyl; in another embodiment, $R_4$ is $C_{1-6}$ haloalkyl; in another embodiment, $R_4$ is $C_{3-7}$ cycloalkyl; in another embodiment, $R_4$ is 3- to 7-membered heterocyclyl; in another embodiment, $R_4$ is optionally substituted with 1, 2 or 3 $R_{4s}$.

In one embodiment, $R_4$ located at the ortho position of $M_1$ and $R_2$ together with the atom to which they are attached form $C_{5-7}$ cycloalkyl; in another embodiment, $R_4$ located at the ortho position of $M_1$ and $R_2$ together with the atom to which they are attached form 5- to 7-membered heterocyclyl; in another embodiment, $R_4$ located at the ortho position of $M_1$ and $Z_3$ together with the atom to which they are attached form $C_{5-7}$ cycloalkyl; in another embodiment, $R_4$ located at the ortho position of $M_1$ and $Z_3$ together with the atom to which they are attached form 5- to 7-membered heterocyclyl; in another embodiment, the ring structure formed by $R_4$ and $R_2$ or $Z_3$ is optionally substituted with 1, 2 or 3 $R_{4s}$.

In a more specific embodiment, $R_4$ is independently selected from H, D, halogen, CN, $OR_a$, $NR_bR_c$, $C_{1-6}$ alkyl, $C_{1-6}$ haloalkyl, $C_{3-7}$ cycloalkyl or 3- to 7-membered heterocyclyl; in another more specific embodiment, $R_4$ is independently selected from H, D, halogen, CN, $C_{1-6}$ alkyl, $C_{1-6}$ haloalkyl, $C_{3-7}$ cycloalkyl or 3- to 7-membered heterocyclyl; in another more specific embodiment, $R_4$ is independently selected from H, D, halogen, $C_{1-6}$ alkyl or $C_{1-6}$ haloalkyl; in another more specific embodiment, $R_4$ is independently selected from H, D, $C_{1-6}$ alkyl or $C_{1-6}$ haloalkyl; in another more specific embodiment, $R_4$ is independently H or D; in another more specific embodiment, $R_4$ is H.

In a more specific embodiment, $R_4$ located at the ortho position of $M_1$ and $R_2$ together with the atom to which they are attached form 5- to 7-membered heterocyclyl; alternatively, $R_4$ located at the ortho position of $M_1$ and $Z_3$ together with the atom to which they are attached form 5- to 7-membered heterocyclyl; in another more specific embodiment, $R_4$ located at the ortho position of $M_1$ and $R_2$ together with the atom to which they are attached form 5- to 7-membered heterocyclyl.

$R_{4s}$

In one embodiment, $R_{4s}$ is H; in another embodiment, $R_{4s}$ is D; in another embodiment, $R_{4s}$ is halogen; in another embodiment, $R_{4s}$ is CN; in another embodiment, $R_{4s}$ is $C_{1-6}$ alkyl; in another embodiment, $R_{4s}$ is $C_{1-6}$ haloalkyl; in another embodiment, $R_{4s}$ is $C_{3-7}$ cycloalkyl; in another embodiment, $R_{4s}$ is 3- to 7-membered heterocyclyl.

In a more specific embodiment, $R_{4s}$ is independently selected from H, D, halogen, $C_{1-6}$ alkyl or $C_{1-6}$ haloalkyl.

n

In one embodiment, n is 0; in another embodiment, n is 1; in another embodiment, n is 2; in another embodiment, n is 3; in another embodiment, n is 4.

In a more specific embodiment, n is selected from 0, 1 or 2; in another more specific embodiment, n is 0.

$Z_1$, $Z_2$ and $Z_3$

In one embodiment, $Z_1$ is $CR_7$; in another embodiment, $Z_1$ is N.

In one embodiment, $Z_2$ is $CR_8$; in another embodiment, $Z_2$ is N;
in one embodiment, $Z_3$ is $CR_9$; in another embodiment, $Z_3$ is N.

In a more specific embodiment, $Z_1$ is N; in another more specific embodiment, $Z_2$ is $CR_8$; in another more specific embodiment, $Z_3$ is $CR_9$; in another more specific embodiment,

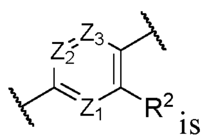 is 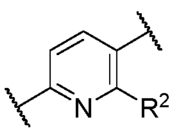 .

$R_7$, $R_8$ and $R_9$

In one embodiment, $R_7$ is H; in another embodiment, $R_7$ is D; in another embodiment, $R_7$ is halogen; in another embodiment, $R_7$ is CN; in another embodiment, $R_7$ is $OR_a$; in another embodiment, $R_7$ is $SR_a$; in another embodiment, $R_7$ is $NR_bR_c$; in another embodiment, $R_7$ is $C_{1-6}$ alkyl; in another embodiment, $R_7$ is $C_{1-6}$ haloalkyl; in another embodiment, $R_7$ is $C_{3-7}$ cycloalkyl; in another embodiment, $R_7$ is 3- to 7-membered heterocyclyl, which is optionally substituted with 1, 2 or 3 $R_{2s}$.

In one embodiment, $R_8$ is H; in another embodiment, $R_8$ is D; in another embodiment, $R_8$ is halogen; in another embodiment, $R_8$ is CN; in another embodiment, $R_8$ is $OR_a$; in another embodiment, $R_8$ is $SR_a$; in another embodiment, $R_8$ is $NR_bR_c$; in another embodiment, $R_8$ is $C_{1-6}$ alkyl; in another embodiment, $R_8$ is $C_{1-6}$ haloalkyl; in another embodiment, $R_8$ is $C_{3-7}$ cycloalkyl; in another embodiment, $R_8$ is 3- to 7-membered heterocyclyl, which is optionally substituted with 1, 2 or 3 $R_{2s}$.

In one embodiment, $R_9$ is H; in another embodiment, $R_9$ is D; in another embodiment, $R_9$ is halogen; in another embodiment, $R_9$ is CN; in another embodiment, $R_9$ is $OR_a$; in another embodiment, $R_9$ is $SR_a$; in another embodiment, $R_9$ is $NR_bR_c$; in another embodiment, $R_9$ is $C_{1-6}$ alkyl; in another embodiment, $R_9$ is $C_{1-6}$ haloalkyl; in another embodiment, $R_9$ is $C_{3-7}$ cycloalkyl; in another embodiment, $R_9$ is 3- to 7-membered heterocyclyl, which is optionally substituted with 1, 2 or 3 $R_{2s}$.

In a more specific embodiment, $R_7$, $R_8$ and $R_9$ are independently selected from H, D, halogen, CN, $C_{1-6}$ alkyl, $C_{1-6}$ haloalkyl, $C_{3-7}$ cycloalkyl or 3- to 7-membered heterocyclyl; in another more specific embodiment, $R_7$, $R_8$ and $R_9$ are independently selected from H, D, halogen, $C_{1-6}$ alkyl or $C_{1-6}$ haloalkyl; in another more specific embodiment, $R_7$, $R_8$ and $R_9$ are independently selected from H, D, $C_{1-6}$ alkyl or $C_{1-6}$ haloalkyl; in another more specific embodiment, $R_7$, $R_8$ and $R_9$ are independently H or D; in another more specific embodiment, $R_7$, $R_8$ and $R_9$ are H.

$R_2$

In one embodiment, $R_2$ is H; in another embodiment, $R_2$ is D; in another embodiment, $R_2$ is halogen, such as F; in another embodiment, $R_2$ is CN; in another embodiment, $R_2$ is $OR_a$; in another embodiment, $R_2$ is $SR_a$; in another embodiment, $R_2$ is $NR_bR_c$; in another embodiment, $R_2$ is $C_{1-6}$ alkyl; in another embodiment, $R_2$ is $C_{1-6}$ haloalkyl; in another embodiment, $R_2$ is $C_{3-7}$ cycloalkyl; in another embodiment, $R_2$ is 3- to 7-membered heterocyclyl, which is optionally substituted with 1, 2 or 3 $R_{2s}$.

In a more specific embodiment, $R_2$ is selected from H, D, halogen, CN, $C_{1-6}$ alkyl, $C_{1-6}$ haloalkyl, $C_{3-7}$ cycloalkyl or 3- to 7-membered heterocyclyl; in another more specific embodiment, $R_2$ is selected from H, D, halogen, CN, $C_{1-6}$ alkyl or $C_{1-6}$ haloalkyl; in another more specific embodiment, $R_2$ is selected from H, D, halogen, $C_{1-6}$ alkyl or $C_{1-6}$ haloalkyl; in another more specific embodiment, $R_2$ is selected from H, D or halogen; in another more specific embodiment, $R_2$ is H or F.

In a more specific embodiment, $R_2$ is halogen, preferably F, Cl or Br, and preferably F.

$R_{2s}$

In one embodiment, $R_{2s}$ is H; in another embodiment, $R_{2s}$ is D; in another embodiment, $R_{2s}$ is halogen; in another embodiment, $R_{2s}$ is CN; in another embodiment, $R_{2s}$ is $C_{1-6}$ alkyl; in another embodiment, $R_{2s}$ is $C_{1-6}$ haloalkyl; in another embodiment, $R_{2s}$ is $C_{3-7}$ cycloalkyl; in another embodiment, $R_{2s}$ is 3- to 7-membered heterocyclyl.

In a more specific embodiment, $R_{2s}$ is independently selected from H, D, halogen, $C_{1-6}$ alkyl or $C_{1-6}$ haloalkyl.

$R_3$

In one embodiment, $R_3$ is H; in another embodiment, $R_3$ is -L-$OR_a$, preferably $OR_a$, such as OMe; in another embodiment, $R_3$ is -L-$SR_a$, preferably $SR_a$; in another embodiment, $R_3$ is -L-$NR_bR_c$, preferably $NR_bR_c$; in another embodiment, $R_3$ is $C_{1-6}$ alkyl, such as Me; in another embodiment, $R_3$ is $C_{1-6}$ haloalkyl; in another embodiment, $R_3$ is $C_{2-6}$ alkenyl; in another embodiment, $R_3$ is $C_{2-6}$ alkynyl; in another embodiment, $R_3$ is -L-$C_{3-10}$ cycloalkyl, preferably $C_{3-10}$ cycloalkyl, and preferably $C_{3-7}$ cycloalkyl; in another embodiment, $R_3$ is -L-3- to 10-membered heterocyclyl, preferably 3- to 10-membered heterocyclyl, and preferably 3- to 7-membered heterocyclyl; in another embodiment, $R_3$ is -L-$C_{6-10}$ aryl, preferably $C_{6-10}$ aryl, and preferably phenyl; in another embodiment, $R_3$ is -L-5- to 10-membered heteroaryl, preferably 5- to 10-membered heteroaryl, and preferably 5- to 6-membered heteroaryl; in another embodiment, $R_3$ is optionally substituted with 1, 2 or 3 $R_{3s}$, for example, $R_3$ is $C_{1-6}$ deuterated alkyl, such as $CD_3$.

In a more specific embodiment, $R_3$ is selected from H, -L-$OR_a$, -L-$SR_a$, -L-$NR_bR_c$, $C_{1-6}$ alkyl, $C_{1-6}$ haloalkyl, -L-$C_{3-10}$ cycloalkyl, -L-3- to 10-membered heterocyclyl, -L-$C_{6-10}$ aryl or -L-5- to 10-membered heteroaryl; in another more specific embodiment, $R_3$ is selected from H, $OR_a$, $NR_bR_c$, $C_{1-6}$ alkyl, $C_{1-6}$ haloalkyl, $C_{3-10}$ cycloalkyl, 3- to 10-membered heterocyclyl, $C_{6-10}$ aryl or 5- to 10-membered heteroaryl; in another more specific embodiment, $R_3$ is selected from H, $OR_a$,

$C_{1-6}$ alkyl, $C_{1-6}$ haloalkyl, $C_{3-7}$ cycloalkyl or 3- to 7-membered heterocyclyl; in another more specific embodiment, $R_3$ is selected from H, $OR_a$, $C_{1-6}$ alkyl, $C_{1-6}$ haloalkyl or $C_{1-6}$ deuterated alkyl; in another more specific embodiment, $R_3$ is selected from $OR_a$, $C_{1-6}$ alkyl, $C_{1-6}$ haloalkyl or $C_{1-6}$ deuterated alkyl; in another more specific embodiment, $R_3$ is selected from Me, $CD_3$ or OMe.

In a more specific embodiment, $R_3$ is selected from $C_{1-6}$ alkyl, $C_{1-6}$ haloalkyl and $C_{1-6}$ deuterated alkyl; in another more specific embodiment, $R_3$ is $C_{1-6}$ deuterated alkyl; in another more specific embodiment, $R_3$ is selected from Me and $CD_3$; in another more specific embodiment, $R_3$ is $CD_3$.

$R_{3s}$

In one embodiment, $R_{3s}$ is H; in another embodiment, $R_{3s}$ is D; in another embodiment, $R_{3s}$ is halogen; in another embodiment, $R_{3s}$ is CN; in another embodiment, $R_{3s}$ is $OR_a$; in another embodiment, $R_{3s}$ is $SR_a$; in another embodiment, $R_{3s}$ is $NR_bR_c$; in another embodiment, $R_{3s}$ is $C_{1-6}$ alkyl; in another embodiment, $R_{3s}$ is $C_{1-6}$ haloalkyl; in another embodiment, $R_{3s}$ is $C_{2-6}$ alkenyl; in another embodiment, $R_{3s}$ is $C_{2-6}$ alkynyl; in another embodiment, $R_{3s}$ is $C_{3-10}$ cycloalkyl, preferably $C_{3-7}$ cycloalkyl; in another embodiment, $R_{3s}$ is 3- to 10-membered heterocyclyl, preferably 3- to 7-membered heterocyclyl; in another embodiment, $R_{3s}$ is $C_{6-10}$ aryl, preferably phenyl; in another embodiment, $R_{3s}$ is 5- to 10-membered heteroaryl, preferably 5- to 6-membered heteroaryl.

In a more specific embodiment, $R_{3s}$ is independently selected from H, D, halogen, CN, $OR_a$, $NR_bR_c$, $C_{1-6}$ alkyl, $C_{1-6}$ haloalkyl, $C_{3-10}$ cycloalkyl, 3- to 10-membered heterocyclyl, $C_{6-10}$ aryl or 5- to 10-membered heteroaryl; in another more specific embodiment, $R_{3s}$ is independently selected from H, D, halogen, CN, $C_{1-6}$ alkyl, $C_{1-6}$ haloalkyl, $C_{3-7}$ cycloalkyl or 3- to 7-membered heterocyclyl; in another more specific embodiment, $R_{3s}$ is independently selected from H, D, halogen, $C_{1-6}$ alkyl or $C_{1-6}$ haloalkyl; in another more specific embodiment, $R_{3s}$ is independently selected from H, D, $C_{1-6}$ alkyl or $C_{1-6}$ haloalkyl.

L

In one embodiment, L is a chemical bond; in another embodiment, L is $C_{1-6}$ alkylene; in another embodiment, L is $C_{2-6}$ alkenylene; in another embodiment, L is $C_{2-6}$ alkynylene; in another embodiment, L is optionally substituted with 1, 2 or 3 groups selected from H, D, halogen, $C_{1-6}$ alkyl, $C_{1-6}$ haloalkyl, $C_{2-6}$ alkenyl or $C_{2-6}$ alkynyl; in another embodiment, L is optionally substituted with 1, 2 or 3 groups selected from H, D, halogen, $C_{1-6}$ alkyl or $C_{1-6}$ haloalkyl.

In a more specific embodiment, L is independently selected from a chemical bond or $C_{1-6}$ alkylene, which is optionally substituted with 1, 2 or 3 groups selected from H, D, halogen, $C_{1-6}$ alkyl or $C_{1-6}$ haloalkyl.

$R_a$, $R_b$ and $R_c$

In one embodiment, $R_a$, $R_b$ and $R_c$ are independently H; in another embodiment, $R_a$, $R_b$ and $R_c$ are independently $C_{1-6}$ alkyl, such as Me; in another embodiment, $R_a$, $R_b$ and $R_c$ are independently $C_{1-6}$ haloalkyl; in another embodiment, $R_a$, $R_b$ and $R_c$ are independently $C_{3-10}$ cycloalkyl, preferably $C_{3-7}$ cycloalkyl; in another embodiment, $R_a$, $R_b$ and $R_c$ are independently 3- to 10-membered heterocyclyl, preferably 3- to 7-membered heterocyclyl; in another embodiment, $R_b$ and $R_c$ together with the atom to which they are attached form 5- to 10-membered heterocyclyl, preferably 5- to 7-membered heterocyclyl.

In a more specific embodiment, $R_a$, $R_b$ and $R_c$ are each independently selected from H, $C_{1-6}$ alkyl, $C_{1-6}$ haloalkyl, $C_{3-7}$ cycloalkyl or 3- to 7-membered heterocyclyl; in another more specific embodiment, $R_a$, $R_b$ and $R_c$ are each independently selected from H, $C_{1-6}$ alkyl or $C_{1-6}$ haloalkyl; in another more specific embodiment, $R_a$, $R_b$ and $R_c$ are H or Me; in another more specific embodiment, $R_a$, $R_b$ and $R_c$ are Me; in another more specific embodiment, $R_b$ and $R_c$ together with the atom to which they are attached form 5- to 7-membered heterocyclyl.

Any technical solution in any of the above specific embodiments or any combination thereof may be combined with any technical solution in other specific embodiments or any combination thereof. For example, any technical solution of ring A or any combination thereof may be combined with any technical solution of $L_1$, R, R', A, E, G, $R_A$, $R_E$, $R_G$, $R_1$, $R_{1s}$, $M_1$, $M_2$, $R_5$, $R_6$, $R_4$, $R_{4s}$, n, $Z_1$, $Z_2$, $Z_3$, $R_2$, $R_7$, $R_8$, $R_9$, $R_{2s}$, $R_3$, $R_{3s}$, L, $R_a$, $R_b$, $R_c$, etc. or any combination thereof. The present invention is intended to include combinations of all these technical solutions, which are not listed one by one due to space limitations.

In more specific embodiments, the present invention provides a compound of formula (I), or a pharmaceutically acceptable salt, an isotopic variant, a tautomer, a stereoisomer, a prodrug, a polymorph, a hydrate or a solvate thereof:

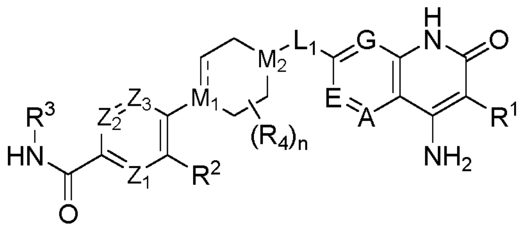

(I)

wherein

$L_1$ is selected from CRR', O, S, NH, -C(O)-, -S(O)- or $-S(O)_2-$;
R and R' are independently selected from H, D, halogen, $C_{1-6}$ alkyl or $C_{1-6}$ haloalkyl;
A is selected from $CR_A$ or N;
E is selected from $CR_E$ or N;
G is selected from $CR_G$ or N;
$R_A$, $R_E$ and $R_G$ are independently selected from H, D, halogen, CN, $-L-OR_a$, - $L-SR_a$, $-L-NR_bR_c$, $C_{1-6}$ alkyl, $C_{1-6}$ haloalkyl, $C_{2-6}$ alkenyl, $C_{2-6}$ alkynyl, $-L-C_{3-10}$ cycloalkyl or -L-3- to 10-membered heterocyclyl;
$R_1$ is selected from H, D, halogen, CN, $-L-OR_a$, $-L-SR_a$, $-L-NR_bR_c$, $C_{1-6}$ alkyl, $C_{1-6}$ haloalkyl, $C_{2-6}$ alkenyl, $C_{2-6}$ alkynyl, $-L-C_{3-10}$ cycloalkyl, -L-3- to 10-membered heterocyclyl, $-L-C_{6-10}$ aryl or -L-5- to 10-membered heteroaryl, which is optionally substituted with 1, 2 or 3 $R_{1s}$;
$R_{1s}$ is independently selected from H, D, halogen, CN, $OR_a$, $SR_a$, $NR_bR_c$, $C_{1-6}$ alkyl, $C_{1-6}$ haloalkyl, $C_{2-6}$ alkenyl, $C_{2-6}$ alkynyl, $C_{3-10}$ cycloalkyl, 3- to 10-membered heterocyclyl, $C_{6-10}$ aryl or 5- to 10-membered heteroaryl;
- - - represents a single bond or a double bond;
$M_1$ is selected from N, C or $CR_5$;
$M_2$ is N or $CR_6$;
$R_5$ and $R_6$ are independently selected from H, D, halogen, $C_{1-6}$ alkyl or $C_{1-6}$ haloalkyl;
$R_4$ is independently selected from H, D, halogen, CN, =O, $OR_a$, $SR_a$, $NR_bR_c$, $C_{1-6}$ alkyl, $C_{1-6}$ haloalkyl, $C_{3-7}$ cycloalkyl or 3- to 7-membered heterocyclyl, which is optionally substituted with 1, 2 or 3 $R_{4s}$;
alternatively, $R_4$ located at the ortho position of $M_1$ and $R_2$ together with the atom to which they are attached form $C_{5-7}$ cycloalkyl or 5- to 7-membered heterocyclyl; alternatively, $R_4$ located at the ortho position of $M_1$ and $Z_3$ together with the atom to which they are attached form $C_{5-7}$ cycloalkyl or 5- to 7-membered heterocyclyl, which is optionally substituted with 1, 2 or 3 $R_{4s}$;
$R_{4s}$ is independently selected from H, D, halogen, CN, $C_{1-6}$ alkyl, $C_{1-6}$ haloalkyl, $C_{3-7}$ cycloalkyl or 3- to 7-membered heterocyclyl;
n is 0, 1, 2, 3 or 4;
$Z_1$ is selected from $CR_7$ or N;
$Z_2$ is selected from $CR_8$ or N;
$Z_3$ is selected from $CR_9$ or N;
$R_2$, $R_7$, $R_8$ and $R_9$ are independently selected from H, D, halogen, CN, $OR_a$, $SR_a$, $NR_bR_c$, $C_{1-6}$ alkyl, $C_{1-6}$ haloalkyl, $C_{3-7}$ cycloalkyl or 3- to 7-membered heterocyclyl, which is optionally substituted with 1, 2 or 3 $R_{2s}$;
$R_{2s}$ is independently selected from H, D, halogen, CN, $C_{1-6}$ alkyl, $C_{1-6}$ haloalkyl, $C_{3-7}$ cycloalkyl or 3- to 7-membered heterocyclyl;
$R_3$ is selected from H, $-L-OR_a$, $-L-SR_a$, $-L-NR_bR_c$, $C_{1-6}$ alkyl, $C_{1-6}$ haloalkyl, $C_{2-6}$ alkenyl, $C_{2-6}$ alkynyl, $-L-C_{3-10}$ cycloalkyl, -L-3- to 10-membered heterocyclyl, $-L-C_{6-10}$ aryl or -L-5- to 10-membered heteroaryl, which is optionally substituted with 1, 2 or 3 $R_{3s}$;
$R_{3s}$ is independently selected from H, D, halogen, CN, $OR_a$, $SR_a$, $NR_bR_c$, $C_{1-6}$ alkyl, $C_{1-6}$ haloalkyl, $C_{2-6}$ alkenyl, $C_{2-6}$ alkynyl, $C_{3-10}$ cycloalkyl, 3- to 10-membered heterocyclyl, $C_{6-10}$ aryl or 5- to 10-membered heteroaryl;
L is a chemical bond, $C_{1-6}$ alkylene, $C_{2-6}$ alkenylene or $C_{2-6}$ alkynylene, which is optionally substituted with 1, 2 or 3 groups selected from H, D, halogen, $C_{1-6}$ alkyl, $C_{1-6}$ haloalkyl, $C_{2-6}$ alkenyl or $C_{2-6}$ alkynyl;
$R_a$, $R_b$ and $R_c$ are each independently selected from H, $C_{1-6}$ alkyl, $C_{1-6}$ haloalkyl, $C_{3-10}$ cycloalkyl or 3- to 10-membered heterocyclyl; alternatively, $R_b$ and $R_c$ together with the atom to which they are attached form 5- to 10-membered heterocyclyl;
wherein each of the above-defined groups is optionally deuterated, up to fully deuterated.

In more specific embodiments, the present invention provides the compound of formula (I) as described above, or the pharmaceutically acceptable salt, the isotopic variant, the tautomer, the stereoisomer, the prodrug, the polymorph, the hydrate or the solvate thereof, wherein $L_1$ is selected from CRR', O, S, NH or -C(O)-, preferably CRR' or -C(O)-, preferably

CRR', and preferably $CH_2$.

In more specific embodiments, the present invention provides the compound of formula (I) as described above, or the pharmaceutically acceptable salt, the isotopic variant, the tautomer, the stereoisomer, the prodrug, the polymorph, the hydrate or the solvate thereof, wherein R and R' are independently selected from H, D, $C_{1-6}$ alkyl or $C_{1-6}$ haloalkyl, preferably selected from H, D, $C_{1-3}$ alkyl or $C_{1-3}$ haloalkyl, preferably H or D, and preferably H.

In more specific embodiments, the present invention provides the compound of formula (I) as described above, or the pharmaceutically acceptable salt, the isotopic variant, the tautomer, the stereoisomer, the prodrug, the polymorph, the hydrate or the solvate thereof, wherein A is N;

preferably, E is $CR_E$;
preferably, G is $CR_G$;
preferably,

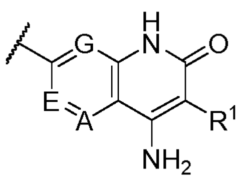 is 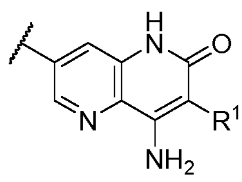 .

In more specific embodiments, the present invention provides the compound of formula (I) as described above, or the pharmaceutically acceptable salt, the isotopic variant, the tautomer, the stereoisomer, the prodrug, the polymorph, the hydrate or the solvate thereof, wherein $R_A$, $R_E$ and $R_G$ are independently selected from H, D, halogen, CN, -L-$OR_a$, -L-$SR_a$, -L-$NR_bR_c$, $C_{1-6}$ alkyl, $C_{1-6}$ haloalkyl, -L-$C_{3-7}$ cycloalkyl or -L-3- to 7-membered heterocyclyl, preferably selected from H, D, halogen, CN, $OR_a$, $NR_bR_c$, $C_{1-6}$ alkyl, $C_{1-6}$ haloalkyl, $C_{3-7}$ cycloalkyl or 3- to 7-membered heterocyclyl, preferably selected from H, D, halogen, $C_{1-6}$ alkyl or $C_{1-6}$ haloalkyl, preferably H or D, and preferably H.

In more specific embodiments, the present invention provides the compound of formula (I) as described above, or the pharmaceutically acceptable salt, the isotopic variant, the tautomer, the stereoisomer, the prodrug, the polymorph, the hydrate or the solvate thereof, wherein $R_1$ is selected from H, D, halogen, CN, -L-$OR_a$, -L-$SR_a$, -L-$NR_bR_c$, $C_{1-6}$ alkyl, $C_{1-6}$ haloalkyl, -L-$C_{3-10}$ cycloalkyl, -L-3- to 10-membered heterocyclyl, -L-$C_{6-10}$ aryl or -L-5- to 10-membered heteroaryl, preferably selected from H, D, halogen, CN, $OR_a$, $NR_bR_c$, $C_{1-6}$ alkyl, $C_{1-6}$ haloalkyl, $C_{3-10}$ cycloalkyl, 3-to 10-membered heterocyclyl, $C_{6-10}$ aryl or 5- to 10-membered heteroaryl, preferably selected from H, D, halogen, CN, $OR_a$, $C_{1-6}$ alkyl, $C_{1-6}$ haloalkyl, $C_{3-7}$ cycloalkyl or 3- to 7-membered heterocyclyl, preferably selected from H, D, halogen, $C_{1-6}$ alkyl or $C_{1-6}$ haloalkyl, preferably selected from H, D, $C_{1-6}$ alkyl or $C_{1-6}$ haloalkyl; preferably $C_{1-6}$ alkyl or $C_{1-6}$ haloalkyl, preferably Et.

In more specific embodiments, the present invention provides the compound of formula (I) as described above, or the pharmaceutically acceptable salt, the isotopic variant, the tautomer, the stereoisomer, the prodrug, the polymorph, the hydrate or the solvate thereof, wherein $R_{1s}$ is independently selected from H, D, halogen, CN, $OR_a$, $NR_bR_c$, $C_{1-6}$ alkyl, $C_{1-6}$ haloalkyl, $C_{3-10}$ cycloalkyl, 3- to 10-membered heterocyclyl, $C_{6-10}$ aryl or 5- to 10-membered heteroaryl, preferably selected from H, D, halogen, CN, $C_{1-6}$ alkyl, $C_{1-6}$ haloalkyl, $C_{3-7}$ cycloalkyl or 3- to 7-membered heterocyclyl, preferably selected from H, D, halogen, $C_{1-6}$ alkyl or $C_{1-6}$ haloalkyl, and preferably selected from H, D, $C_{1-6}$ alkyl or $C_{1-6}$ haloalkyl.

In more specific embodiments, the present invention provides the compound of formula (I) as described above, or the pharmaceutically acceptable salt, the isotopic variant, the tautomer, the stereoisomer, the prodrug, the polymorph, the hydrate or the solvate thereof, wherein $M_1$ is selected from N or $CR_5$, preferably N;

preferably, $M_2$ is N;

preferably,

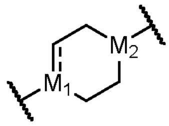 is 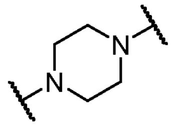 .

In more specific embodiments, the present invention provides the compound of formula (I) as described above, or the pharmaceutically acceptable salt, the isotopic variant, the tautomer, the stereoisomer, the prodrug, the polymorph, the hydrate or the solvate thereof, wherein $R_5$ and $R_6$ are independently selected from H, D, $C_{1-6}$ alkyl or $C_{1-6}$ haloalkyl, preferably H or D, and preferably H.

In more specific embodiments, the present invention provides the compound of formula (I) as described above, or

the pharmaceutically acceptable salt, the isotopic variant, the tautomer, the stereoisomer, the prodrug, the polymorph, the hydrate or the solvate thereof, wherein $R_4$ is independently selected from H, D, halogen, CN, $OR_a$, $NR_bR_c$, $C_{1-6}$ alkyl, $C_{1-6}$ haloalkyl, $C_{3-7}$ cycloalkyl or 3- to 7-membered heterocyclyl, preferably selected from H, D, halogen, CN, $C_{1-6}$ alkyl, $C_{1-6}$ haloalkyl, $C_{3-7}$ cycloalkyl or 3- to 7-membered heterocyclyl, preferably selected from H, D, halogen, $C_{1-6}$ alkyl or $C_{1-6}$ haloalkyl, preferably selected from H, D, $C_{1-6}$ alkyl or $C_{1-6}$ haloalkyl; preferably H or D, and preferably H;
preferably, n is 0, 1 or 2, preferably 0.

In more specific embodiments, the present invention provides the compound of formula (I) as described above, or the pharmaceutically acceptable salt, the isotopic variant, the tautomer, the stereoisomer, the prodrug, the polymorph, the hydrate or the solvate thereof, wherein $R_4$ located at the ortho position of $M_1$ and $R_2$ together with the atom to which they are attached form 5- to 7-membered heterocyclyl; alternatively, $R_4$ located at the ortho position of $M_1$ and $Z_3$ together with the atom to which they are attached form 5- to 7-membered heterocyclyl;
preferably, $R_4$ located at the ortho position of $M_1$ and $R_2$ together with the atom to which they are attached form 5- to 7-membered heterocyclyl.

In more specific embodiments, the present invention provides the compound of formula (I) as described above, or the pharmaceutically acceptable salt, the isotopic variant, the tautomer, the stereoisomer, the prodrug, the polymorph, the hydrate or the solvate thereof, wherein $R_{4s}$ is independently selected from H, D, halogen, $C_{1-6}$ alkyl or $C_{1-6}$ haloalkyl.

In more specific embodiments, the present invention provides the compound of formula (I) as described above, or the pharmaceutically acceptable salt, the isotopic variant, the tautomer, the stereoisomer, the prodrug, the polymorph, the hydrate or the solvate thereof, wherein $Z_1$ is N;

preferably, $Z_2$ is $CR_8$;
preferably, $Z_3$ is $CR_9$;
preferably,

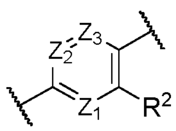 is 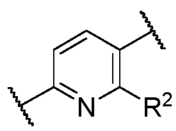.

In more specific embodiments, the present invention provides the compound of formula (I) as described above, or the pharmaceutically acceptable salt, the isotopic variant, the tautomer, the stereoisomer, the prodrug, the polymorph, the hydrate or the solvate thereof, wherein $R_7$, $R_8$ and $R_9$ are independently selected from H, D, halogen, CN, $C_{1-6}$ alkyl, $C_{1-6}$ haloalkyl, $C_{3-7}$ cycloalkyl or 3- to 7-membered heterocyclyl, preferably selected from H, D, halogen, $C_{1-6}$ alkyl or $C_{1-6}$ haloalkyl, preferably selected from H, D, $C_{1-6}$ alkyl or $C_{1-6}$ haloalkyl; preferably H or D, and preferably H.

In more specific embodiments, the present invention provides the compound of formula (I) as described above, or the pharmaceutically acceptable salt, the isotopic variant, the tautomer, the stereoisomer, the prodrug, the polymorph, the hydrate or the solvate thereof, wherein $R_2$ is selected from H, D, halogen, CN, $C_{1-6}$ alkyl, $C_{1-6}$ haloalkyl, $C_{3-7}$ cycloalkyl or 3- to 7-membered heterocyclyl, preferably selected from H, D, halogen, CN, $C_{1-6}$ alkyl or $C_{1-6}$ haloalkyl, preferably selected from H, D, halogen, $C_{1-6}$ alkyl or $C_{1-6}$ haloalkyl, preferably selected from H, D or halogen, and preferably H or F.

In more specific embodiments, the present invention provides the compound of formula (I) as described above, or the pharmaceutically acceptable salt, the isotopic variant, the tautomer, the stereoisomer, the prodrug, the polymorph, the hydrate or the solvate thereof, wherein $R_{2s}$ is independently selected from H, D, halogen, $C_{1-6}$ alkyl or $C_{1-6}$ haloalkyl.

In more specific embodiments, the present invention provides the compound of formula (I) as described above, or the pharmaceutically acceptable salt, the isotopic variant, the tautomer, the stereoisomer, the prodrug, the polymorph, the hydrate or the solvate thereof, wherein $R_3$ is selected from H, $-L-OR_a$, $-L-SR_a$, $-L-NR_bR_c$, $C_{1-6}$ alkyl, $C_{1-6}$ haloalkyl, $-L-C_{3-10}$ cycloalkyl, -L-3- to 10-membered heterocyclyl, $-L-C_{6-10}$ aryl or -L-5- to 10-membered heteroaryl, preferably selected from H, $OR_a$, $NR_bR_c$, $C_{1-6}$ alkyl, $C_{1-6}$ haloalkyl, $C_{3-10}$ cycloalkyl, 3- to 10-membered heterocyclyl, $C_{6-10}$ aryl or 5- to 10-membered heteroaryl, preferably selected from H, $OR_a$, $C_{1-6}$ alkyl, $C_{1-6}$ haloalkyl, $C_{3-7}$ cycloalkyl or 3- to 7-membered heterocyclyl, preferably selected from H, $OR_a$, $C_{1-6}$ alkyl, $C_{1-6}$ haloalkyl or $C_{1-6}$ deuterated alkyl, preferably selected from $OR_a$, $C_{1-6}$ alkyl, $C_{1-6}$ haloalkyl or $C_{1-6}$ deuterated alkyl, and preferably selected from Me, $CD_3$ or OMe.

In more specific embodiments, the present invention provides the compound of formula (I) as described above, or the pharmaceutically acceptable salt, the isotopic variant, the tautomer, the stereoisomer, the prodrug, the polymorph, the hydrate or the solvate thereof, wherein $R_{3s}$ is independently selected from H, D, halogen, CN, $OR_a$, $NR_bR_c$, $C_{1-6}$ alkyl, $C_{1-6}$ haloalkyl, $C_{3-10}$ cycloalkyl, 3- to 10-membered heterocyclyl, $C_{6-10}$ aryl or 5- to 10-membered heteroaryl, preferably selected from H, D, halogen, CN, $C_{1-6}$ alkyl, $C_{1-6}$ haloalkyl, $C_{3-7}$ cycloalkyl or 3- to 7-membered heterocyclyl, preferably selected from H, D, halogen, $C_{1-6}$ alkyl or $C_{1-6}$ haloalkyl, and preferably selected from H, D, $C_{1-6}$ alkyl or $C_{1-6}$ haloalkyl.

In more specific embodiments, the present invention provides the compound of formula (I) as described above, or the pharmaceutically acceptable salt, the isotopic variant, the tautomer, the stereoisomer, the prodrug, the polymorph, the

hydrate or the solvate thereof, wherein L is independently selected from a chemical bond or $C_{1-6}$ alkylene, which is optionally substituted with 1, 2 or 3 groups selected from H, D, halogen, $C_{1-6}$ alkyl or $C_{1-6}$ haloalkyl.

In more specific embodiments, the present invention provides the compound of formula (I) as described above, or the pharmaceutically acceptable salt, the isotopic variant, the tautomer, the stereoisomer, the prodrug, the polymorph, the hydrate or the solvate thereof, wherein $R_a$, $R_b$ and $R_c$ are each independently selected from H, $C_{1-6}$ alkyl, $C_{1-6}$ haloalkyl, $C_{3-7}$ cycloalkyl or 3- to 7-membered heterocyclyl, preferably selected from H, $C_{1-6}$ alkyl or $C_{1-6}$ haloalkyl, preferably $C_{1-6}$ alkyl or $C_{1-6}$ haloalkyl, preferably H or Me, and more preferably Me;

alternatively, $R_b$ and $R_c$ together with the atom to which they are attached form 5- to 7-membered heterocyclyl.

In more specific embodiments, the present invention provides the compound of formula (I) as described above, or the pharmaceutically acceptable salt, the isotopic variant, the tautomer, the stereoisomer, the prodrug, the polymorph, the hydrate or the solvate thereof, having the following structural formula:

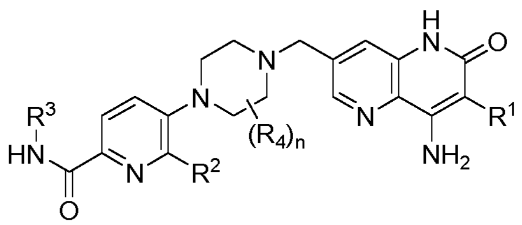

(II)

wherein each group is as defined above.

In more specific embodiments, the present invention provides the compound of formula (II) as described above, or the pharmaceutically acceptable salt, the isotopic variant, the tautomer, the stereoisomer, the prodrug, the polymorph, the hydrate or the solvate thereof, wherein

$R_1$ is selected from H, D, halogen, CN, $OR_a$, $NR_bR_c$, $C_{1-6}$ alkyl, $C_{1-6}$ haloalkyl, $C_{3-10}$ cycloalkyl, 3- to 10-membered heterocyclyl, $C_{6-10}$ aryl or 5- to 10-membered heteroaryl, which is optionally substituted with 1, 2 or 3 $R_{1s}$;
$R_2$ is selected from H, D, halogen, CN, $C_{1-6}$ alkyl, $C_{1-6}$ haloalkyl, $C_{3-7}$ cycloalkyl or 3- to 7-membered heterocyclyl, which is optionally substituted with 1, 2 or 3 $R_{2s}$;
$R_3$ is selected from H, $OR_a$, $NR_bR_c$, $C_{1-6}$ alkyl, $C_{1-6}$ haloalkyl, $C_{3-10}$ cycloalkyl, 3- to 10-membered heterocyclyl, $C_{6-10}$ aryl or 5- to 10-membered heteroaryl, which is optionally substituted with 1, 2 or 3 $R_{3s}$;
$R_4$ is selected from H, D, halogen, CN, $C_{1-6}$ alkyl, $C_{1-6}$ haloalkyl, $C_{3-7}$ cycloalkyl or 3- to 7-membered heterocyclyl, which is optionally substituted with 1, 2 or 3 $R_{4s}$;
alternatively, $R_4$ located at the ortho position of N attached to pyridine and $R_2$ together with the atom to which they are attached form 5- to 7-membered heterocyclyl;
$R_{1s}$ and $R_{3s}$ are each independently selected from H, D, halogen, CN, $OR_a$, $NR_bR_c$, $C_{1-6}$ alkyl, $C_{1-6}$ haloalkyl, $C_{3-10}$ cycloalkyl, 3- to 10-membered heterocyclyl, $C_{6-10}$ aryl or 5- to 10-membered heteroaryl;
$R_{2s}$ and $R_{4s}$ are each independently selected from H, D, halogen, $C_{1-6}$ alkyl or $C_{1-6}$ haloalkyl;
n is 0, 1, 2, 3 or 4;
$R_a$, $R_b$ and $R_c$ are each independently selected from H, $C_{1-6}$ alkyl, $C_{1-6}$ haloalkyl, $C_{3-7}$ cycloalkyl or 3- to 7-membered heterocyclyl; alternatively, $R_b$ and $R_c$ together with the atom to which they are attached form 5- to 7-membered heterocyclyl;
wherein each of the above-defined groups is optionally deuterated, up to fully deuterated.

In more specific embodiments, the present invention provides the compound of formula (II) as described above, or the pharmaceutically acceptable salt, the isotopic variant, the tautomer, the stereoisomer, the prodrug, the polymorph, the hydrate or the solvate thereof, wherein

$R_1$ is selected from H, D, halogen, CN, $OR_a$, $C_{1-6}$ alkyl, $C_{1-6}$ haloalkyl, $C_{3-7}$ cycloalkyl or 3- to 7-membered heterocyclyl, which is optionally substituted with 1, 2 or 3 $R_{1s}$;
$R_2$ is selected from H, D, halogen, CN, $C_{1-6}$ alkyl or $C_{1-6}$ haloalkyl, which is optionally substituted with 1, 2 or 3 $R_{2s}$;
$R_3$ is selected from H, $OR_a$, $C_{1-6}$ alkyl, $C_{1-6}$ haloalkyl, $C_{3-7}$ cycloalkyl or 3- to 7-membered heterocyclyl, which is optionally substituted with 1, 2 or 3 $R_{3s}$;
$R_4$ is selected from H, D, halogen, $C_{1-6}$ alkyl or $C_{1-6}$ haloalkyl, which is optionally substituted with 1, 2 or 3 $R_{4s}$;
$R_{1s}$ and $R_{3s}$ are each independently selected from H, D, halogen, CN, $C_{1-6}$ alkyl, $C_{1-6}$ haloalkyl, $C_{3-7}$ cycloalkyl or 3- to 7-membered heterocyclyl;
$R_{2s}$ and $R_{4s}$ are each independently selected from H, D, halogen, $C_{1-6}$ alkyl or $C_{1-6}$ haloalkyl;

n is 0, 1, 2, 3 or 4;
$R_a$ is independently selected from H, $C_{1-6}$ alkyl or $C_{1-6}$ haloalkyl.

In more specific embodiments, the present invention provides the compound of formula (II) as described above, or the pharmaceutically acceptable salt, the isotopic variant, the tautomer, the stereoisomer, the prodrug, the polymorph, the hydrate or the solvate thereof, wherein

$R_1$ is selected from H, D, $C_{1-6}$ alkyl or $C_{1-6}$ haloalkyl;
$R_2$ is selected from H, D, halogen, $C_{1-6}$ alkyl or $C_{1-6}$ haloalkyl;
$R_3$ is selected from H, $OR_a$, $C_{1-6}$ alkyl, $C_{1-6}$ haloalkyl or $C_{1-6}$ deuterated alkyl;
$R_4$ is selected from H, D, $C_{1-6}$ alkyl or $C_{1-6}$ haloalkyl;
n is 0, 1, 2, 3 or 4;
$R_a$ is selected from H, $C_{1-6}$ alkyl or $C_{1-6}$ haloalkyl.

In more specific embodiments, the present invention provides the compound of formula (II) as described above, or the pharmaceutically acceptable salt, the isotopic variant, the tautomer, the stereoisomer, the prodrug, the polymorph, the hydrate or the solvate thereof, wherein

$R_1$ is $C_{1-6}$ alkyl or $C_{1-6}$ haloalkyl, preferably Et;
$R_2$ is selected from H, D or halogen, preferably H or F;
$R_3$ is selected from $OR_a$, $C_{1-6}$ alkyl, $C_{1-6}$ haloalkyl or $C_{1-6}$ deuterated alkyl, preferably selected from Me, $CD_3$ or OMe;
$R_4$ is H or D, preferably H;
n is 0, 1 or 2, preferably 0;
$R_a$ is $C_{1-6}$ alkyl or $C_{1-6}$ haloalkyl, preferably Me;
preferably, $R_3$ is selected from $C_{1-6}$ alkyl, $C_{1-6}$ haloalkyl and $C_{1-6}$ deuterated alkyl, preferably $C_{1-6}$ deuterated alkyl, preferably selected from Me and $CD_3$, and preferably $CD_3$;
preferably, $R_2$ is halogen, preferably F, Cl or Br, and preferably F.

In more specific embodiments, the present invention provides the compound of formula (I) as described above, or the pharmaceutically acceptable salt, the isotopic variant, the tautomer, the stereoisomer, the prodrug, the polymorph, the hydrate or the solvate thereof, wherein the compound is selected from the following:

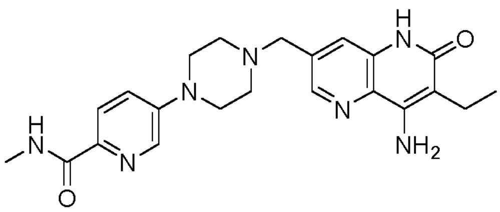
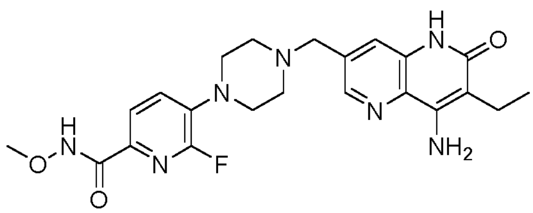
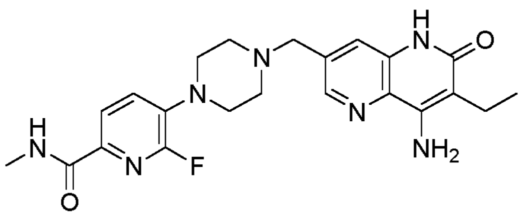
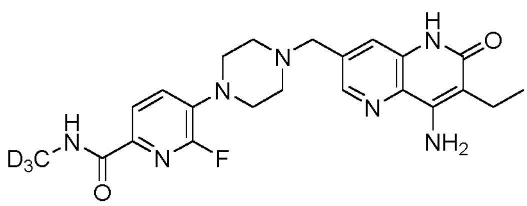
.

The compounds of the present invention may contain one or more asymmetric centres and may therefore exist in various stereoisomeric forms, for example, enantiomeric and/or diastereomeric forms. For example, the compounds of the present invention can be individual enantiomers, diastereomers or geometric isomers (such as cis and trans isomers), or can be in the form of mixtures of stereoisomers, including racemic mixtures and mixtures enriched in one or more stereoisomers. Isomers may be separated from mixtures by methods known to those skilled in the art, including: chiral high pressure liquid chromatography (HPLC), as well as the formation and crystallization of chiral salts; alternatively, preferred isomers may be prepared by asymmetric synthesis.

The compounds of the present invention may exist in tautomeric forms. Tautomers are functional group isomers produced by the rapid movement of an atom in a molecule at two positions. Tautomers are a special type of functional group isomers. A pair of tautomers can be converted into each other, but usually a more stable isomer exists predominantly. The

most prominent examples are the enol and keto tautomers.

Those skilled in the art will appreciate that an organic compound may form a complex with a solvent in which it reacts or from which it precipitates or crystallizes. These complexes are called "solvates". When the solvent is water, the complex is called a "hydrate". The present invention encompasses all solvates of the compounds of the present invention.

The term "solvate" refers to the form of a compound or a salt thereof in association with a solvent, usually formed by a solvolysis reaction. This physical association may involve hydrogen bonding. Conventional solvents include water, methanol, ethanol, acetic acid, DMSO, THF, diethyl ether, etc. The compounds described herein can be prepared, for example, in a crystalline form, and can be solvated. Suitable solvates include pharmaceutically acceptable solvates and further include stoichiometric solvates and non-stoichiometric solvates. In some cases, the solvate will be capable of isolation, for example, when one or more solvent molecules are incorporated in the crystal lattice of the crystalline solid. The "solvate" includes both solution-phase and isolatable solvates. Representative solvates include hydrates, ethanolates and methanolates.

The term "hydrate" refers to a compound in combination with water. Usually, the ratio of the number of water molecules contained in a hydrate of a compound to the number of molecules of the compound in the hydrate is determined. Therefore, the hydrate of a compound can be represented, for example, by general formula $R \cdot x\ H_2O$, wherein R is the compound, and x is a number greater than 0. A given compound may form more than one type of hydrate, including, for example, a monohydrate (x is 1), a lower hydrate (x is a number greater than 0 and less than 1, for example, a hemihydrate ($R \cdot 0.5\ H_2O$)) and a polyhydrate (x is a number greater than 1, for example, a dihydrate ($R \cdot 2\ H_2O$) and a hexahydrate ($R \cdot 6\ H_2O$)).

The compounds of the present invention may be in amorphous or crystalline forms (polymorphs). Furthermore, the compounds of the present invention may exist in one or more crystalline forms. Therefore, the present invention includes within its scope all amorphous or crystalline forms of the compounds of the present invention. The term "polymorph" refers to a crystalline form of a compound (or a salt, hydrate, or solvate thereof) in a specific crystal packing arrangement. All polymorphs have the same elemental composition. Different crystalline forms usually have different X-ray diffraction patterns, infrared spectra, melting points, density, hardness, crystal shape, optoelectronic properties, stability and solubility. The recrystallization solvent, crystallization rate, storage temperature and other factors may cause one crystalline form to predominate. Various polymorphs of a compound can be prepared by crystallization under different conditions.

The present invention further includes isotopically labelled compounds (isotopic variants) which are identical to those described in formula (I), but have one or more atoms replaced with an atom having an atomic mass or mass number different from the atomic mass or mass number usually found in nature. Examples of isotopes that can be incorporated into the compounds of the present invention include isotopes of hydrogen, carbon, nitrogen, oxygen, phosphorus, sulphur, fluorine and chlorine, such as $^{2}H$, $^{3}H$, $^{13}C$, $^{11}C$, $^{14}C$, $^{15}N$, $^{18}O$, $^{17}O$, $^{31}P$, $^{32}P$, $^{35}S$, $^{18}F$ and $^{36}Cl$, respectively. The compounds of the present invention, the prodrugs thereof, and the pharmaceutically acceptable salts of the compounds or the prodrugs containing the above-mentioned isotopes and/or other isotopes of other atoms all fall within the scope of the present invention. Certain isotopically labelled compounds of the present invention, for example those into which radioactive isotopes such as $^{3}H$ and $^{14}C$ are incorporated, are useful in drug and/or substrate tissue distribution assays. Tritium (i.e., $^{3}H$) and carbon-14 (i.e., $^{14}C$) isotopes are particularly preferred for their ease of preparation and detectability. Further, substitution with heavier isotopes, such as deuterium (i.e., $^{2}H$), can provide therapeutic benefits due to higher metabolic stability, such as an extended in vivo half-life or a reduced dosage requirement, and may thus be preferred in some cases. The isotopically labelled compounds of formula (I) of the present invention and the prodrugs thereof can generally be prepared by replacing non-isotopically labelled reagents with readily available isotopically labelled reagents when carrying out the processes disclosed in the following procedures and/or examples and preparation examples.

Furthermore, prodrugs are also included within the context of the present invention. As used herein, the term "prodrug" refers to a compound that is converted in vivo by hydrolysis, for example, in the blood, to its active form that has a medical effect. Pharmaceutically acceptable prodrugs are described in T. Higuchi and V. Stella, Prodrugs as Novel Delivery Systems, A.C.S. Symposium Series, Vol. 14; Edward B. Roche, ed., Bioreversible Carriers in Drug Design, American Pharmaceutical Association and Pergamon Press, 1987; and D. Fleisher, S. Ramon and H. Barbra, "Improved oral drug delivery: solubility limitations overcome by the use of prodrugs", Advanced Drug Delivery Reviews (1996) 19(2) 115-130, each of which is incorporated herein by reference.

A prodrug is any covalently bonded compound of the present invention which, when administered to a patient, releases the parent compound in vivo. Prodrugs are generally prepared by modifying functional groups in such a way that the modification can be made by conventional manipulation or cleavage in vivo to produce the parent compound. Prodrugs include, for example, the compounds of the present invention in which a hydroxyl, amino or sulfhydryl group is bonded to any group that can be cleaved to form the hydroxyl, amino or sulfhydryl group when the compounds are administered to a patient. Thus, representative examples of prodrugs include, but are not limited to, acetate/amide, formate/amide and benzoate/amide derivatives of hydroxyl, sulfhydryl and amino functional groups of the compound of formula (I). In addition, in the case of carboxylic acid (-COOH), esters such as methyl ester and ethyl ester can be used. The ester itself can be

active and/or can be hydrolysed under the conditions in the human body. Suitable pharmaceutically acceptable in vivo hydrolysable ester groups include those that break down readily in the human body to release the parent acid or a salt thereof.

The present invention further provides a pharmaceutical preparation comprising a therapeutically effective amount of a compound of formula (I) or a therapeutically acceptable salt thereof, and a pharmaceutically acceptable carrier, diluent or excipient thereof. All these forms fall within the scope of the present invention.

Pharmaceutical composition and kit

In another aspect, the present invention provides a pharmaceutical composition comprising the compound of the present invention (also referred to as the "active ingredient") and a pharmaceutically acceptable excipient. In some embodiments, the pharmaceutical composition comprises an effective amount of the compound of the present invention. In some embodiments, the pharmaceutical composition comprises a therapeutically effective amount of the compound of the present invention. In some embodiments, the pharmaceutical composition comprises a prophylactically effective amount of the compound of the present invention.

Pharmaceutically acceptable excipients for use in the present invention refer to non-toxic carriers, adjuvants or vehicles that do not destroy the pharmacological activity of the compounds formulated together. Pharmaceutically acceptable carriers, adjuvants or vehicles that can be used in the compositions of the present invention include, but are not limited to, ion exchangers, aluminium oxide, aluminium stearate, lecithin, serum proteins (such as human serum albumin), buffering substances (such as phosphates), glycine, sorbic acid, potassium sorbate, mixtures of partial glycerides of saturated vegetable fatty acids, water, salts or electrolytes (such as protamine sulfate), disodium hydrogen phosphate, potassium hydrogen phosphate, sodium chloride, zinc salts, silica gel, magnesium trisilicate, polyvinylpyrrolidone, cellulose-based substances, polyethylene glycol, sodium carboxymethyl cellulose, polyacrylates, waxes, polyethylene-polyoxypropylene-block polymers, polyethylene glycol and lanolin.

Suitable preparations for administering the compounds of the present invention will be apparent to those of ordinary skill in the art and include, for example, tablets, pills, capsules, suppositories, troches, lozenges, solutions (particularly solutions for injection (subcutaneous, intravenous, and intramuscular) and infusion (injectables)), elixirs, syrups, cachets, emulsions, inhalants or dispersible powders. The content of the pharmaceutically active compound(s) should be in the range of 0.1 to 90 wt%, preferably 0.5 to 50 wt%, of the composition as a whole, i.e., in an amount sufficient to achieve the dosage range specified below. If necessary, the specified dose may be administered several times a day.

The present invention further comprises a kit (e.g., a pharmaceutical package). The provided kit may include the compounds of the present invention, an additional therapeutic agent, as well as first and second containers (such as vials, ampoules, bottles, syringes and/or dispersible packages or other suitable containers) containing the compounds of the present invention and an additional therapeutic agent. In some embodiments, the provided kit may optionally further include a third container containing a pharmaceutical excipient for diluting or suspending the compounds of the present invention and/or an additional therapeutic agent. In some embodiments, the compounds of the present invention and an additional therapeutic agent provided in the first container and the second container are combined to form a unit dosage form.

Administration

The pharmaceutical composition provided by the present invention can be administered by many routes, including but not limited to: oral administration, parenteral administration, inhalation administration, topical administration, rectal administration, nasal administration, buccal administration, vaginal administration, administration via implants or other administration methods. For example, parenteral administration as used herein includes subcutaneous administration, intradermal administration, intravenous administration, intramuscular administration, intra-articular administration, intra-arterial administration, intrasynovial administration, intrasternal administration, intrathecal administration, intralesional administration, and intracranial injection or infusion techniques.

Typically, an effective amount of the compound provided herein is administered. The actual amount of the compound administered can be determined by a physician according to the relevant circumstances, including the condition being treated, the route of administration chosen, the compound actually administered, the age, weight and response of an individual patient, the severity of the patient's symptoms, etc.

When used to prevent the condition of the present invention, the compound provided herein is administered to a subject at risk of developing the condition, typically based on the advice and under the supervision of a physician, at dosage levels as described above. Subjects at risk of developing a specific condition generally include those with a family history of the condition or those identified through genetic testing or screening as being particularly susceptible to developing the condition.

The pharmaceutical composition provided herein can also be administered chronically ("chronic administration"). Chronic administration refers to administration of a compound or pharmaceutical composition thereof over an extended period of time, e.g., 3 months, 6 months, 1 year, 2 years, 3 years, 5 years, and so on, or may continue indefinitely, e.g., for the remainder of the subject's life. **In** some embodiments, chronic administration is intended to provide a constant level of the compound in the blood over an extended period of time, e.g., within a therapeutic window.

Various methods of administration may be used to further deliver the pharmaceutical compositions of the present invention. For example, in some embodiments, the pharmaceutical composition can be administered by bolus, for instance, in order to increase the concentration of the compound in the blood to an effective level. The bolus dose depends on the target systemic level of the active ingredient in the body. For example, an intramuscular or subcutaneous bolus dose results in a slow release of the active ingredient, while a bolus administered directly into a vein (e.g., via an intravenous (IV) drip) enables a faster delivery, causing the concentration of the active ingredient in the blood to rise rapidly to an effective level. In other embodiments, the pharmaceutical composition may be administered as a continuous infusion, for example, via an intravenous (IV) drip, so as to provide a steady-state concentration of the active ingredient in the subject's body. Furthermore, in other embodiments, a bolus dose of the pharmaceutical composition may be administered first, followed by a continuous infusion.

Oral compositions may take the form of bulk liquid solutions or suspensions, or bulk powders. More typically, however, for the convenience of precise dosing, the composition is provided in unit dosage form. The term "unit dosage form" refers to physically discrete units suitable as unitary dosages for human patients and other mammals, each unit containing a predetermined amount of active material suitable to produce the desired therapeutic effect, in association with a suitable pharmaceutical excipient. Typical unit dosage forms include pre-filled and premeasured ampoules or syringes for liquid compositions, or pills, tablets, capsules and the like in the case of solid compositions. In such compositions, the compound typically constitutes a minor component (about 0.1 to about 50% by weight, or preferably about 1 to about 40% by weight), with the remainder consisting of various carriers or excipients and processing aids useful for forming the desired administration form.

For oral dosing, a representative regimen is one to five oral doses, especially two to four oral doses, typically three oral doses per day. Using these dosing regimens, each dose provides about 0.01 to about 20 mg/kg of the compound of the present invention, preferably each dose provides about 0.1 to about 10 mg/kg, especially about 1 to about 5 mg/kg.

In order to provide blood levels similar to those achieved with an injection dose, or blood levels lower than those with an injection dose, a transdermal dose is typically selected in an amount of about 0.01 to about 20% by weight, preferably about 0.1 to about 20% by weight, preferably about 0.1 to about 10% by weight, and more preferably about 0.5 to about 15% by weight.

The injection dosage level ranges from about 0.1 mg/kg/hour to at least 10 mg/kg/hour from about 1 to about 120 hours, especially from 24 to 96 hours. To achieve adequate steady-state levels, a pre-loading bolus of about 0.1 mg/kg to about 10 mg/kg or more may also be administered. For a human patient weighing 40 to 80 kg, the maximum total dose should not exceed approximately 2 g/day.

Liquid forms suitable for oral administration may include suitable aqueous or non-aqueous carriers, as well as buffering agents, suspending agents and dispersing agents, colouring agents, flavouring agents, and the like. Solid forms may include, for example, any of the following components, or compounds with similar properties: binders, such as microcrystalline cellulose, tragacanth gum or gelatine; excipients, such as starch or lactose; disintegrants, such as alginic acid, Primogel or corn starch; lubricants, such as magnesium stearate; glidants, such as colloidal silica; sweeteners, such as sucrose or saccharin; or flavouring agents, such as peppermint, methyl salicylate, or orange flavouring agents.

Injectable compositions are typically based on injectable sterile saline or phosphate-buffered saline, or other injectable excipients known in the art. As described above, in such compositions, the active compound typically constitutes a minor component, usually about 0.05 to 10% by weight, with the remainder consisting of injectable excipients and the like.

Transdermal compositions are typically formulated as a topical ointment or cream containing the active ingredient. When formulated as an ointment, the active ingredient is typically combined with paraffin or water-miscible ointment bases. Alternatively, the active ingredient may be formulated as a cream with, for example, an oil-in-water cream base. Such a transdermal preparation is well known in the art and generally includes other components for enhancing the stable skin penetration of the active ingredient or the preparation. All such known transdermal preparations and components are included within the scope provided by the present invention.

The compounds of the present invention may also be administered via transdermal devices. Thus, transdermal administration may be accomplished using patches of the reservoir or porous membrane type, or a variety of solid matrices.

The above-mentioned components of the compositions for oral administration, injection or topical administration are merely representative. Additional materials and processing techniques are described in Section 8 of Remington's Pharmaceutical Sciences, 17th edition, 1985, Mack Publishing Company, Easton, Pennsylvania, which is incorporated herein by reference.

The compounds of the present invention may also be administered in sustained release form or from a sustained

release drug delivery system. Descriptions of representative sustained release materials can be found in Remington's Pharmaceutical Sciences.

The present invention further relates to pharmaceutically acceptable preparations of the compounds of the present invention. In one embodiment, the preparation comprises water. In another embodiment, the preparation comprises a cyclodextrin derivative. The most common cyclodextrins are α-, β-, and γ-cyclodextrins composed of 6, 7, and 8 α-1,4-linked glucose units, respectively, which optionally include one or more substituents on the linked sugar moieties, including but not limited to: methylated, hydroxyalkylated, acylated, and sulfoalkyl ether substitutions. In some embodiments, the cyclodextrin is a sulfoalkyl ether β-cyclodextrin, such as sulfobutyl ether β-cyclodextrin, also known as Captisol. See, e.g., U.S. 5,376,645. In some embodiments, the preparation includes hexapropyl-β-cyclodextrin (e.g., 10-50% in water).

**Indication**

For tumours lacking the HR-dependent DNA DSB repair pathway or other DNA repair mechanisms, the development of highly selective PARP1 inhibitors can provide therapeutic benefits to a large number of cancer patients. The compounds of the present invention exert therapeutic effects by negatively regulating the activity of PARP1 within tumour cells in a highly selective manner, especially on tumour cells lacking the HR-dependent DNA DSB repair pathway or other DNA repair mechanisms, or on various tumour cells with a BRCA1- or BRCA2-deficient phenotype.

In some embodiments, the PARP1 inhibitors of the present invention can treat various cancers, ischemic diseases and neurodegenerative diseases.

More specifically, these compounds are useful in the treatment of: heart: sarcomas (such as angiosarcoma, fibrosarcoma, rhabdomyosarcoma, and liposarcoma), myxoma, rhabdomyoma, fibroma, lipoma, and teratoma; lung: bronchogenic carcinoma (such as squamous cell, undifferentiated small cell, undifferentiated large cell, and adenocarcinoma), alveolar (bronchiolar) carcinoma, bronchial adenoma, sarcoma, lymphoma, chondroma, hamartoma, and mesothelioma; gastrointestinal tract: oesophagus (such as squamous cell carcinoma, adenocarcinoma, leiomyosarcoma, and lymphoma), stomach (such as tumour, lymphoma, and leiomyosarcoma), pancreas (such as ductal adenocarcinoma, insulinoma, glucagonoma, gastrinoma, carcinoid tumour, and haemangioma), small intestine (such as adenocarcinoma, lymphoma, carcinoid tumour, Kaposi's sarcoma, leiomyoma, haemangioma, lipoma, neurofibroma, and fibroma), and large intestine (such as adenocarcinoma, tubular adenoma, villous adenoma, hamartoma, and leiomyoma); urogenital tract: kidney (such as adenocarcinoma and nephroblastoma), bladder and urethra (such as squamous cell carcinoma, transitional cell carcinoma, and adenocarcinoma), prostate (such as adenocarcinoma and sarcoma), and testis (such as seminoma, teratoma, embryonal carcinoma, teratocarcinoma, choriocarcinoma, sarcoma, stromal cell carcinoma, fibroma, fibroadenoma, adenomatous tumour, and lipoma); liver: hepatoma, cholangiocarcinoma, hepatoblastoma, angiosarcoma, hepatocellular adenoma, and haemangioma; biliary tract: gallbladder cancer, ampullary cancer, bile duct cancer, etc.; bone: osteosarcoma, fibrosarcoma, malignant fibrous histiocytoma, chondrosarcoma, Ewing's sarcoma, malignant lymphoma (such as reticular cell sarcoma), multiple myeloma, malignant giant cell chordoma, osteochondroma (osteocartilaginous exostoses), benign chondroma, chondroblastoma, chondromyxoid fibroma, osteoid osteoma and giant cell tumour of bone; nervous system: skull (such as osteoma, haemangioma, granuloma, xanthoma, and osteitis deformans), meninges (such as meningioma, meningosarcoma, and glioma), and brain (such as astrocytoma, medulloblastoma, glioma, ependymoma, germinoma (such as pinealoma), glioblastoma multiforme, oligodendroglioma, schwannoma, retinoblastoma, congenital tumour, spinal neurofibroma, meningioma, glioma, and sarcoma); gynaecology: uterus (such as endometrial cancer), cervix (such as cervical cancer and preneoplastic cervical dysplasia), ovary (such as ovarian cancer, serous cystadenocarcinoma, mucinous cystadenocarcinoma, unclassified cancer, granulosa cell tumour, Sertoli-Leydig cell tumour, dysgerminoma, and malignant teratoma), vulva (such as squamous cell carcinoma, intraepithelial carcinoma, adenocarcinoma, fibrosarcoma, and melanoma), vagina (such as clear cell carcinoma, squamous cell carcinoma, and botryoid sarcoma (such as embryonal rhabdomyosarcoma)), fallopian tube cancer, etc.; haematology: blood (such as myeloid leukaemia (acute and chronic), acute lymphocytic leukaemia, chronic lymphocytic leukaemia, diffuse large B-cell lymphoma, mantle cell lymphoma (MCL), follicular lymphoma, myeloproliferative disorders, multiple myeloma, and myelodysplastic syndrome), Hodgkin's disease, non-Hodgkin's lymphoma (malignant lymphoma), etc.; skin: malignant melanoma, basal cell carcinoma, squamous cell carcinoma, Kaposi's sarcoma, nevus dysplasia, lipoma, haemangioma, dermatofibroma, keloid, psoriasis, etc.; and adrenal glands: neuroblastoma, etc.

More specifically, these compounds are useful in the treatment of: breast cancer, ovarian cancer, pancreatic cancer, prostate cancer, blood cancer, gastrointestinal cancer (such as gastric cancer and colourectal cancer) and lung cancer.

**Combined administration**

The PARP1 inhibitor of the present invention can be used in combination with other drugs for the treatment of

cancer, including at least one target drug/cell activity modulator, including CDK4/6 inhibitors, MAT2A inhibitors, MAPK1/-MAPK3 inhibitors, Type I PRMT inhibitors, EGFR inhibitors, SHP2 inhibitors, pan-KRAS inhibitors, KRASG12C inhibitors, RAF inhibitors, MEK inhibitors, ERK inhibitors, Bcl-2 inhibitors, SOS1 inhibitors, PARP inhibitors, MALT1 inhibitors, MALT2 inhibitors, BTK inhibitors, PI3K inhibitors, AKT inhibitors, FGFR inhibitors, DNA methyltransferase (DNMT) inhibitors, EZH1/2 inhibitors, EZH2 inhibitors, Menin-MLL inhibitors, IDH1 inhibitors, IDH2 inhibitors, IDH1/2 inhibitors, chemotherapeutic drugs (such as carboplatin), radiotherapy, STING agonists or immune checkpoint inhibitors/modulators, etc.

**Example**

The raw materials or reagents used herein are commercially available or prepared by synthetic methods generally known in the art.

**Example 1**

**5-(4-((8-Amino-7-ethyl-6-oxo-5,6-dihydro-1,5-naphthyridin-3-yl)methyl)piperazin-1-yl)-N-methylpicolinamide**

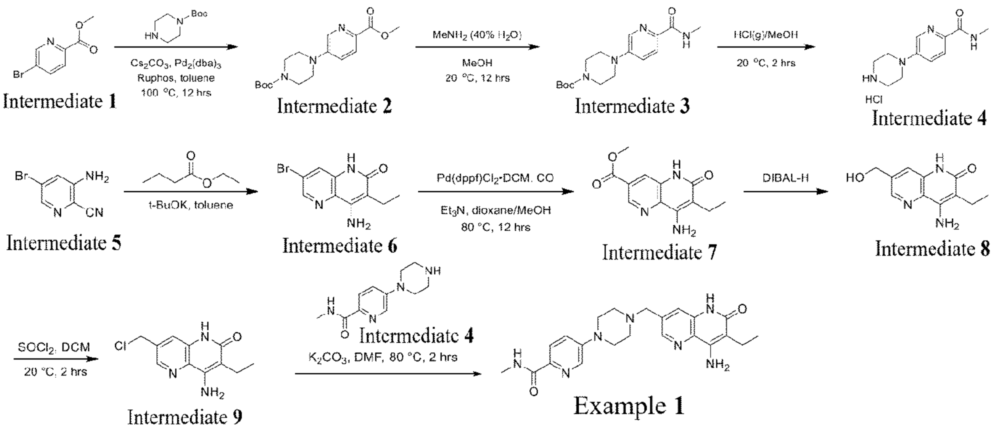


**Step 1**

Intermediate **1** (2.00 g) was dissolved in toluene (14.0 mL), and 1-Bocpiperazine (1.72 g), cesium carbonate (9.05 g), 2-dicyclohexylphosphino-2,6-diisopropoxy-1,1-biphenyl (432 mg) and bis(dibenzylideneacetone)palladium (133 mg) were added. After the addition was completed, the reaction liquid was stirred at 100°C for 12 hours. LCMS (RT = 1.700 min) showed that the raw materials were completely consumed. The reaction liquid was cooled and then filtered through diatomaceous earth. Water (15.0 mL) and ethyl acetate (20.0 mL, 10.0 mL) were added to the filtrate for extraction. The organic phase was washed with saturated sodium chloride solution (10.0 mL), dried over anhydrous sodium sulfate, filtered, and concentrated under reduced pressure to obtain intermediate **2** (3.00 g, crude) as a yellow oil.

LCMS (ESI) m/z: 322.0 $[M+H]^+$.

**Step 2**

Intermediate **2** (3.00 g) was dissolved in methanol (21.0 mL), and methylamine solution (7.25 g, purity: 40%) was added. After the addition was completed, the reaction liquid was stirred at 20°C for 4 hours. LCMS (RT = 1.376 min) showed that the raw materials were completely consumed. Dilute hydrochloric acid (20.0 mL, 2M) was added to the reaction liquid to quench the reaction, and dichloromethane (20.0 mL, 10.0 mL) was added for extraction. The organic phase was washed with saturated sodium chloride solution (20.0 mL), dried over anhydrous sodium sulfate, filtered, and concentrated under reduced pressure to obtain intermediate **3** (2.90 g, crude) as a yellow oil.

LCMS (ESI) m/z: 321.2 $[M+H]^+$.

**Step 3**

Intermediate **3** (2.90 g) was dissolved in hydrochloric acid/methanol (21.0 mL, 4M). After the addition was

completed, the reaction liquid was stirred at 20°C for 2 hours. TLC (petroleum ether/ethyl acetate = 0/1, product: $R_f$ = 0.02, raw material: $R_f$ = 0.43) showed that the raw materials were completely consumed. The reaction liquid was concentrated under reduced pressure to obtain a crude product, which was then slurried in methyl tert-butyl ether (4.00 mL) for purification to obtain intermediate **4** (2.00 g, yield: 86.0%, HCl) as a yellow oil.

**Step 4**

Intermediate **5** (5.00 g) was dissolved in toluene (35.0 mL), and ethyl butyrate (5.87 g, 6.74 mL) and potassium tert-butoxide (8.50 g) were added. After the addition was completed, the reaction liquid was stirred at 50°C for 2 hours. LCMS (RT = 1.355 min) showed that the raw materials were completely consumed. The reaction liquid was cooled, and then water (15.0 mL) and ethyl acetate (20.0 mL, 15.0 mL) were added to the reaction liquid for extraction. The organic phase was washed with saturated sodium chloride solution (15.0 mL), dried over anhydrous sodium sulfate, filtered, and concentrated under reduced pressure to obtain intermediate **6** (3.00 g, yield: 31.2%) as a yellow oil.

LCMS (ESI) m/z: 268.0 $[M+H]^+$.

**Step 5**

Intermediate **6** (3.00 g) was dissolved in dioxane (18.0 mL) and methanol (3.00 mL), and triethylamine (3.40 g, 4.67 mL) and [1,1-bis(diphenylphosphino)ferrocene]dichloropalladium-dichloromethane (456 mg) were added. After the addition was completed, the reaction liquid was stirred at 80°C for 12 hours under a carbon monoxide atmosphere (50 Psi). LCMS (RT = 1.213 min) showed that the raw materials were completely consumed. The reaction liquid was cooled and then filtered through diatomaceous earth. Water (10.0 mL) and ethyl acetate (10.0 mL, 8.00 mL) were added to the filtrate for extraction. The organic phase was washed with saturated sodium chloride solution (5.00 mL), dried over anhydrous sodium sulfate, filtered, and concentrated under reduced pressure to obtain intermediate 7 (2.00 g, crude) as a yellow solid.

LCMS (ESI) m/z: 248.1 $[M+H]^+$.

**Step 6**

Intermediate **7** (2.30 g) was dissolved in dichloromethane (15.0 mL), and diisobutylaluminium hydride (13.9 mL, 1M) was added at -78°C. After the addition was completed, the reaction liquid was stirred at 20°C for 1 hour. LCMS (RT = 0.58 min) showed that the raw materials were completely consumed. Water (10.0 mL) and dichloromethane (10.0 mL, 8.00 mL) were added to the reaction liquid for extraction. The organic phase was washed with saturated sodium chloride solution (5.00 mL), dried over anhydrous sodium sulfate, filtered, and concentrated under reduced pressure to obtain a crude product. The crude product was purified by high performance liquid chromatography (chromatographic column: Phenomenex C18 250 * 50 mm * 10 um; mobile phase: [water (ammonia water) - acetonitrile]; B%: 3% - 30%, 20 min) to obtain intermediate **8** (300 mg, yield: 14.7%) as a yellow solid.

LCMS (ESI) m/z: 220.1 $[M+H]^+$.

**Step 7**

Intermediate **8** (80.0 mg) was dissolved in dichloromethane (2.00 mL), and thionyl chloride (217 mg, 132 μL) was added at 0°C. After the addition was completed, the reaction liquid was stirred at 20°C for 2 hours. LCMS (RT = 1.43 min) showed that the raw materials were completely consumed. The reaction liquid was concentrated under reduced pressure to obtain intermediate **9** (80.0 mg, yield: 92.2%) as a yellow solid.

LCMS (ESI) m/z: 238.0 $[M+H]^+$.

**Step 8**

Intermediate **9** (80.0 mg) and intermediate **4** (111 mg, HCl) were dissolved in N,N-dimethylformamide (1.00 mL), and potassium carbonate (139 mg) was added. After the addition was completed, the reaction liquid was stirred at 60°C for 2 hours. LCMS (RT = 1.287 min) showed that the raw materials were completely consumed. The reaction liquid was cooled, and then water (3.00 mL) and ethyl acetate (5.00 mL, 3.00 mL) were added to the reaction liquid for extraction. The organic phase was washed with saturated sodium chloride solution (5.00 mL), dried over anhydrous sodium sulfate, filtered, and concentrated under reduced pressure to obtain a crude product. The crude product was purified by high performance liquid chromatography (chromatographic column: Phenomenex luna C18 80 * 40 mm * 3 um; mobile phase: [water (hydrochloric acid) - acetonitrile]; B%: 5% - 35%, 7 min) to obtain the compound of **Example 1** (74.3 mg, purity: 100%, HCl) as a white solid.

LCMS (ESI) m/z: 422.2 [M+H]$^+$;

$^1$H NMR (400 MHz, DMSO-d6) δ ppm 1.01 (t, *J* = 7.32 Hz, 3 H), 2.53 - 2.59 (m, 2 H), 2.77 - 2.87 (m, 3 H), 3.17 - 3.55 (m, 6 H), 4.02 - 4.18 (m, 2 H), 4.52 (br s, 2 H),, 7.47 - 7.58 (m, 1 H) 7.73 (d, *J* = 1.60 Hz, 1 H), 7.86 - 7.99 (m, 1 H), 8.29 - 8.41 (m, 1 H), 8.50 (br d, *J* = 4.80 Hz, 1 H), 8.61 - 8.70 (m, 1 H), 11.18 - 11.30 (m, 1 H), 11.32 - 11.51 (m, 1 H).

**Example 2**

**5-(4-((8-Amino-7-ethyl-6-oxo-5,6-dihydro-1,5-naphthyridin-3-yl)methyl)piperazin-1-yl)-6-fluoro-N-methoxypicolinamide**

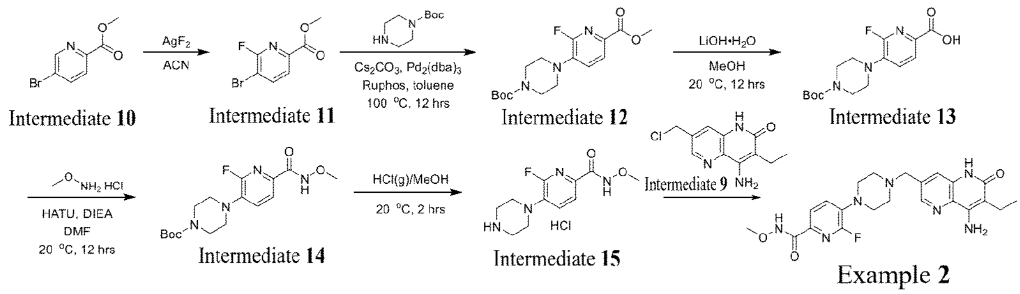


**Step 1**

Intermediate **10** (14.0 g) was dissolved in acetonitrile (140 mL), and silver (II) fluoride (24.6 g) was added. After the addition was completed, the reaction liquid was stirred at 20°C for 12 hours. TLC (petroleum ether/ethyl acetate = 2/1, product: $R_f$ = 0.54, raw material: $R_f$ = 0.43) showed that the raw materials were completely consumed. The reaction liquid was filtered through diatomaceous earth, and the filtrate was concentrated under reduced pressure to obtain a crude product. The crude product was purified by column chromatography (silica, petroleum ether/ethyl acetate = 50/1 - 1/1) to obtain intermediate **11** (9.30 g, yield: 61.3%) as a yellow solid.

**Step 2**

Intermediate **11** (4.30 g) was dissolved in toluene (43 mL), and 1-tert-butoxycarbonyl-piperazine (4.11 g), cesium carbonate (19.4 g), 2-dicyclohexylphosphino-2,6-diisopropoxy-1,1-biphenyl (857 mg) and bis(dibenzylideneacetone) palladium (504 mg) were added. After the addition was completed, the reaction liquid was stirred at 100°C for 12 hours. TLC (petroleum ether/ethyl acetate = 2/1, product: $R_f$ = 0.24, raw material: $R_f$ = 0.54) showed that the raw materials were completely consumed. The reaction liquid was cooled and then filtered through diatomaceous earth. Water (50.0 mL) and ethyl acetate (50.0 mL, 30.0 mL) were added to the filtrate for extraction. The organic phase was washed with saturated sodium chloride solution (30.0 mL), dried over anhydrous sodium sulfate, filtered, and concentrated under reduced pressure to obtain a crude product. The crude product was purified by column chromatography (silica, petroleum ether/ethyl acetate = 50/1 - 0/1) to obtain intermediate **12** (2.70 g, yield: 43.3%) as a yellow solid.

$^1$H NMR (400 MHz, $CDCl_3$) δ ppm 1.49 (s, 9 H) 3.15 - 3.26 (m, 4 H) 3.56 - 3.67 (m, 4 H) 3.96 (s, 3 H) 7.23 - 7.27 (m, 1 H) 7.97 (dd, *J* = 8.00, 1.06 Hz, 1 H).

**Step 3**

Intermediate 12 (2.70 g) was dissolved in tetrahydrofuran (14.0 mL), methanol (2.70 mL) and water (7.00 mL), and lithium hydroxide (700 mg) was added. After the addition was completed, the reaction liquid was stirred at 20°C for 12 hours. TLC (petroleum ether/ethyl acetate = 1/1, product: $R_f$ = 0.02, raw material: $R_f$ = 0.43) showed that the raw materials were completely consumed. Dilute hydrochloric acid (10.0 mL, 2M) was added to the reaction liquid to quench the reaction, and ethyl acetate (30.0 mL, 20.0 mL) was added for extraction. The organic phase was washed with saturated sodium chloride solution (20.0 mL), dried over anhydrous sodium sulfate, filtered, and concentrated under reduced pressure to obtain intermediate **13** (2.10 g, yield: 81.1%) as a yellow oil.

$^1$H NMR (400 MHz, $CDCl_3$) δ ppm 1.35 - 1.60 (m, 9 H) 2.63 - 3.13 (m, 4 H) 3.28 - 3.68 (m, 4 H) 6.81 - 7.16 (m, 1 H) 7.65 - 8.00 (m, 1 H).

**Step 4**

Intermediate **13** (700 mg) was dissolved in N,N-dimethylformamide (7.00 mL), and *N,N*-diisopropylethylamine (556 mg, 749 μL), methoxyamine hydrochloride (179 mg) and O-(7-azabenzotriazol-1-yl)-N,N,N',N'-tetramethyluronium hexafluorophosphonate (818 mg) were added. After the addition was completed, the reaction liquid was stirred at 20°C for 12 hours. TLC (petroleum ether/ethyl acetate = 1/1, product: $R_f$ = 0.24, raw material: $R_f$ = 0.02) showed that the raw materials were completely consumed. Water (10.0 mL) and ethyl acetate (10.0 mL, 8.00 mL) were added to the reaction liquid for extraction. The organic phase was washed with saturated sodium chloride solution (10.0 mL), dried over anhydrous sodium sulfate, filtered, and concentrated under reduced pressure to obtain intermediate **14** (500 mg, yield: 65.5%) as a yellow solid.

**Step 5**

Intermediate **14** (100 mg) was dissolved in hydrochloric acid/methanol (2.00 mL, 4 M). After the addition was completed, the reaction liquid was stirred at 20°C for 2 hours. TLC (petroleum ether/ethyl acetate = 1/1, product: $R_f$ = 0.02, raw material: $R_f$ = 0.43) showed that the raw materials were completely consumed. The reaction liquid was concentrated under reduced pressure to obtain intermediate 15 (50.0 mg, yield: 69.6%, HCl) as a yellow solid.

**Step 6**

Intermediate **9** (50.0 mg) and intermediate **15** (56.3 mg, HCl) were dissolved in *N,N*-dimethylformamide (1.00 mL), and *N,N*-diisopropylethylamine (81.5 mg) was added. After the addition was completed, the reaction liquid was stirred at 60°C for 2 hours. LCMS (RT = 0.892 min) showed that the raw materials were completely consumed. The reaction liquid was cooled, and then water (3.00 mL) and ethyl acetate (5.00 mL, 3.00 mL) were added to the reaction liquid for extraction. The organic phase was washed with saturated sodium chloride solution (5.00 mL), dried over anhydrous sodium sulfate, filtered, and concentrated under reduced pressure to obtain a crude product. The crude product was purified by high performance liquid chromatography (chromatographic column: Phenomenex luna C18 80 * 30 mm * 3 um; mobile phase: [water (hydrochloric acid) - acetonitrile]; B%: 5% - 35%, 8 min) to obtain the compound of **Example 2** (13.0 mg, purity: 96.4%, HCl) as a white solid.

LCMS (ESI) m/z: 456.1 $[M+H]^+$;
$^1$H NMR (400 MHz, DMSO-d6) δ ppm 1.01 (t, *J* = 7.20 Hz, 3 H) 2.56 (br. s, 2 H) 3.13 - 3.33 (m, 6 H) 3.67 (s, 3 H) 3.73 (br. d, *J* = 12.0 Hz, 2 H) 4.54 (br. s, 2 H) 6.36 - 6.50 (m, 1 H) 7.65 - 7.74 (m, 2 H) 7.88 (d, *J* = 8.00 Hz, 1 H) 8.52 (br. s, 1 H) 10.51 (br. d, *J* = 3.60 Hz, 1 H) 11.24 (br. s, 1 H) 11.82 (s, 1 H).

**Examples 3 and 4 were synthesized using the same experimental method as that of Examples 1 and 2.**

**Example 3**

**5-(4-((8-Amino-7-ethyl-6-oxo-5,6-dihydro-1,5-naphthyridin-3-yl)methyl)piperazin-1-yl)-6-fluoro-N-methylpicolinamide**

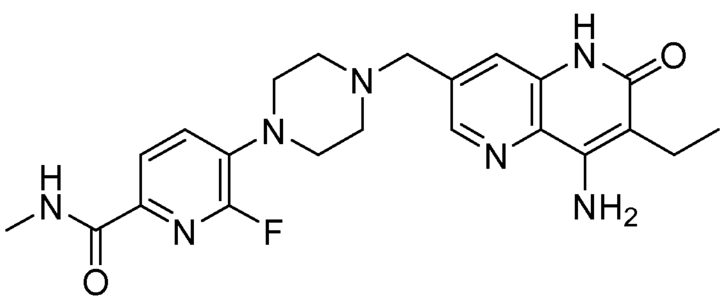

LCMS (ESI) m/z: 440.1 $[M+H]^+$;
$^1$H NMR (400 MHz, DMSO-d6) δ ppm 1.01 (t, *J* = 7.20 Hz, 3 H) 2.54 - 2.58 (m, 2 H) 2.74 - 2.82 (m, 3 H) 3.14 - 3.38 (m, 6 H) 3.72 (br. d, *J* = 12.0 Hz, 2 H) 4.54 (br. s, 2 H) 6.31 - 6.53 (m, 1 H) 7.65 - 7.73 (m, 2 H) 7.89 (d, *J* = 7.80 Hz, 1 H) 8.43 -

8.48 (m, 1 H) 8.50 - 8.55 (m, 1 H) 10.28 - 10.68 (m, 1 H) 11.25 (br. s, 1 H).

**Example 4**

**5-(4-((8-Amino-7-ethyl-6-oxo-5,6-dihydro-1,5-naphthyridin-3-yl)methyl)piperazin-1-yl)-6-fluoro-N-(methyl-d3)picolinamide**

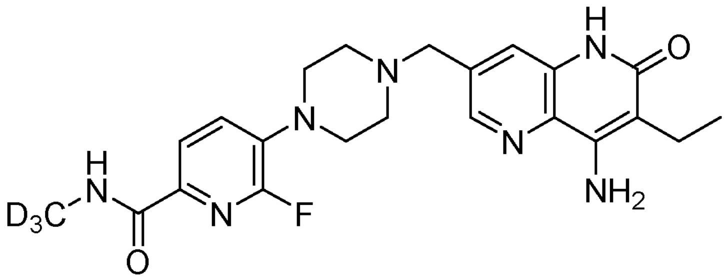

LCMS (ESI) m/z: 443.1 $[M+H]^+$;
$^1$H NMR (400 MHz, DMSO-d6) δ ppm 0.96 - 1.05 (m, 3 H) 2.29 - 2.41 (m, 4 H) 2.54 (br. s, 2 H) 3.11 - 3.25 (m, 4 H) 3.63 - 3.80 (m, 2 H) 4.54 (br. s, 2 H) 6.42 (br. s, 2 H) 7.68 - 7.72 (m, 1 H) 7.89 (d, *J*= 8.00 Hz, 1 H) 8.42 (s, 1 H) 8.51 (br. s, 1 H) 10.28 - 10.54 (m, 1 H) 11.24 (br. s, 1 H).

**Experimental example 1 Inhibitory effect of compounds on PARP1/PARP2 enzyme activity**

The PARP1/PARP2 enzyme activity was detected by chemiluminescence. First, histone (Active Motif, 81126) was incubated on a 384-well plate for 2 hours, and different dilutions of the example compounds and PARP1 working solution (Abcam, ab279663) or PARP2 working solution (BPS, 80502) were added. Only PARP1 working solution or PARP2 working solution was added to the Max control well, and only assay buffer was added to the Min control well. The plate was incubated at room temperature for 15 minutes, and the biotinylated substrate $NAD^+$ (BPS, 80610) was added. The plate was incubated at room temperature for 2 hours. After the substrate was catalysed by the enzyme, the ADP-ribose group was bound to the histone and still remained biotinylated. Streptavidin-HRP solution (Abcam, ab7403) was added to develop colour via the biotin, and the values were read on the EnSight (PE) instrument. The inhibition rate was calculated using the fluorescence values of the Max and Min wells, and the dose-response curves were fitted using the analysis software GraphPad Prism 5 to obtain the $IC_{50}$ value of each compound for the enzyme activity.

The results are as shown in Table 1 below. Examples 1, 2, 3, and 4 had a dose-dependent inhibitory effect on the PARP1 enzyme, with significant inhibitory activities reaching the picomolar concentration level, showing a similar activity to the reference compound AZD5305 and Olaparib. In terms of selectivity for PARP2, Example 1 had a 3-fold superior selectivity to the reference compound AZD5305, and Examples 2, 3, and 4 had a similar selectivity to that of the reference compound AZD5305. In terms of selectivity for PARP2, Example 1 had a 3-fold superior selectivity to the control AZD5305, and Examples 2, 3, and 4 had a similar selectivity to that of the positive control. Compared with the currently marketed PARPi Olaparib, Examples 1, 2, 3, and 4 had a significantly improved selectivity for PARP2 while maintaining the inhibitory activity against the PARP1 enzyme, with the selectivity increased by 16- to 83-fold.

Table 1 Inhibition of PARP1/PARP2 enzyme activity by compounds

| No. | Compound No. | PARP1 $IC_{50}$ (nM) | PARP2 $IC_{50}$ (nM) | PARP1/2 selectivity fold |
|---|---|---|---|---|
| 1 | Example 1 | 0.49 | 52.8 | 108 |
| 2 | Example 2 | 0.39 | 17.0 | 44 |
| 3 | Example 3 | 0.41 | 8.7 | 21 |
| 4 | Example 4 | 0.48 | 18.6 | 39 |
| 5 | AZD5305 | 0.42 | 13.6 | 32 |
| 6 | Olaparib | 0.50 | 0.65 | 1.3 |

**Experimental example 2 DNA trapping capability of PARP1/PARP2 induced by compound**

The DNA trapping capability of PARP1/PARP2 was detected using homogeneous time-resolved fluorescence (HTRF). First, PARP1 (BPS, 80501) or PARP2 (BPS, 80502) was labelled with Mab anti GST-Tb crypate (cisbio, 61GSTTLA), and a damaged DNA labelling probe (Generay) was added. Different concentrations of the example compounds were added, 50 μM AZD2281 was added to the Max control well, and medium buffer was added to the Min well. The mixture was incubated at room temperature for 1 hour, and the substrate $NAD^+$ (Sigma, 10127965001) was added and incubated for 10 minutes. When PARP exerted its enzymatic activity, it caused the release of PARP itself from the damaged DNA, at which point only the fluorescent signal with an emission wavelength of 615 nm could be detected. When PARP was inhibited, it induced PARP to bind to the damaged DNA, triggering energy transfer. At that point, two fluorescence signals with different emission wavelengths could be detected: one from the damaged DNA probe itself with an emission wavelength of 615 nm, and the other from the energy transfer that occurred after PARP bound to the damaged DNA with an emission wavelength of 665 nm. The fluorescence ratio of 665 nm to 615 nm was calculated to represent the amount of the complex formed by PARP trapping DNA. The trapping capability induced by each compound was calculated using the fluorescence values from the Max and Min wells, and the dose-response curve was fitted using the analysis software GraphPad Prism 5 to obtain the $EC_{50}$ value (the concentration required to achieve 50% of the maximum trapping) of the DNA trapping capability of PARP enzymes induced by each compound.

The results are as shown in Table 2 below. Examples 1, 2, 3, and 4 induced PARP to trapping DNA at single-digit nanomolar concentrations, with significant trapping capability, showing a similar activity to the reference compound AZD53305 and a superior activity to Olaparib. In terms of selectivity for PARP2, Example 3 achieved a 64-fold selectivity, similar to the reference compound AZD5305; Examples 1, 2, and 4 achieved approximately 100-fold selectivity, better than the reference compound AZD5305. Compared with the currently marketed PARPi Olaparib, Examples 1, 2, 3, and 4 had a significantly improved activity against PARP1 and selectivity for PARP2, with the activity increased by 3- to 7-fold and the selectivity by 213- to 333-fold.

Table 2 DNA trapping capability of PARP induced by compound

| No. | Compound No. | PARP1 $EC_{50}$ (nM) | PARP2 $EC_{50}$ (nM) | PARP1/2 selectivity fold |
|---|---|---|---|---|
| 1 | Example 1 | 1.78 | 167.8 | 94 |
| 2 | Example 2 | 3.73 | 337.8 | 91 |
| 3 | Example 3 | 3.87 | 248.5 | 64 |
| 4 | Example 4 | 3.80 | 382.4 | 100 |
| 5 | AZD5305 | 1.74 | 130.5 | 75 |
| 6 | Olaparib | 11.49 | 3.8 | 0.3 |

**Experimental example 3 Inhibitory effect of compounds on PARP1/PARP2 enzyme activity at the cellular level**

The inhibitory effect of the compounds on PARP1/PARP2 enzyme activity at the cellular level was detected using high-content imaging. A549 WT, *PARP1*-KO and *PARP2-KO* cell lines were established in-house, and the cells were revived. After the cells were stable, a sufficient number of cells were collected and 100 μL of the cell suspension was seeded into a 96-well plate. The next day, the example compounds with serially diluted concentrations were added. Only buffer was added to the Max control well, and 500 nM AZD5305 (A549 WT and *PARP2-KO* cell lines) or 1 μM AZD2281 (*PARP1*-KO cell line) was added to the Min control well. The plate was incubated for 1.5 hours (A549 WT and *PARP2-KO* cell lines) or 2 hours (*PARP1*-KO cell line). After removing the supernatant, 0.4 mM $H_2O_2$ was added and incubation was carried out for 10 minutes (A549 WT and *PARP2-KO* cell lines) or 1.5 mM $H_2O_2$ was added and incubation was carried out for 15 minutes (*PARP1*-KO cell line) to induce extensive cellular DNA damage. After removing the supernatant, the cells were fixed with 4% paraformaldehyde for 20 minutes, followed by treatment with 0.5% Triton X-100 for 20 minutes to increase the permeability of the cell membrane. After that, the cells were incubated with 3% BSA for 1 hour to prevent non-specific binding. A 1: 500 diluted primary antibody, Poly (ADP-ribose) monoclonal antibody (CST, 83732S), was added and incubated overnight, and then a 1: 500 diluted secondary antibody, Goat anti-Rabbit IgG, Alexa Fluor™ 488 (invitrogen, A-11034), was added and incubated for 1 hour. The cell nuclei were stained for 30 minutes by adding 50 μL DAPI (invitrogen, R37606). After washing twice with PBS, images were acquired on OPERETTA CLS™ (PE) by scanning the cells under a 20x water objective lens in non-confocal mode, with 5 fields of view captured per well. When analysing the data, the DAPI-stained nucleus population was selected to distinguish the nucleus from the cytoplasm, and the average intensity value of Alexa 488 in the nucleus was calculated for each well. The inhibition rate was calculated using the

fluorescence values from the Max and Min wells, and the dose-response curve was fitted using the analysis software GraphPad Prism 5 to obtain the $IC_{50}$ value of each compound for the enzyme activity.

The results are as shown in Table 3. The example compounds exhibited dose-dependent inhibition of enzyme activity in both A549 WT and *PARP2-KO* cell lines, with significant inhibitory activity. Example 4 achieved single-digit nanomolar concentrations and showed comparable inhibitory activity between the two cell lines, indicating that the enzyme activity at the cellular level was primarily attributed to PARP1. Compared with the reference compound AZD5305, Examples 1, 2, 3, and 4 had similar inhibitory activities against the PARP1 enzyme. In A549 *PARP1*-KO cell lines, the example compounds all showed markedly weak inhibitory activities, wherein Examples 2, 3, and 4 showed no detectable inhibition on the enzyme activity even at the highest concentration of 40 μM, indicating that after *PARP1* knockout, the example compounds showed negligible inhibition on PARylation at the cellular level, and the compounds had non-obvious inhibitory effects on the activity of enzymes other than PARP1. Examples 1 and 2 had a similar selectivity to the reference compound AZD5305. Examples 3 and 4 had a selectivity increased by more than 3- to 6-fold compared with the reference compound AZD5305, showing better selectivity.

Table 3 Inhibition of PARP1/PARP2 enzyme-mediated PARylation activity by compounds at the cellular level

| Compound No. | A549 WT | *PARP2*-KO | *PARP1*-KO | *PARP1* KO/*PARP2* KO selectivity fold |
|---|---|---|---|---|
| | $IC_{50}$ (nM) | $IC_{50}$ (nM) | $IC_{50}$ (nM) | |
| Example 1 | 14.4 | 72.2 | 47650.7 | 660 |
| Example 2 | 10.5 | 61.7 | >40000 | >648 |
| Example 3 | 5.9 | 16.2 | >40000 | >2468 |
| Example 4 | 5.2 | 7.7 | >40000 | >5222 |
| AZD5305 | 2.2 | 6.7 | 5760.8 | 861 |

**Experimental example 4 Anti-proliferative activity test of compounds on tumour cells**

The anti-proliferation experiment of the compounds on tumour cells adopted the most widely used ATP concentration-based detection method at present. MDA-MB-231 and MDA-MB-436 cell lines were obtained from ATCC, DLD-1 human colourectal adenocarcinoma epithelial cells and DLD-1 *BRCA2*$^{-/-}$ cells were obtained from Horizon, and the cells were revived. After the cells were stable, the cells were collected. When the viable cell count exceeded 90%, 450-500 cells were seeded into a 384-well plate. The next day, the example compounds with serially diluted concentrations were added. Only buffer was added to the Max well, and 50 μM AZD2281 was added to the Min well. The plate was incubated for 7 days, and on the eighth day, CellTiter-Glo® reagent (Promega, G7573) was added. The plate was incubated at room temperature for 30 minutes, and the values were read on Envision (PE). The inhibition rate was calculated using the fluorescence values from the Max and Min wells, and the dose-response curve was fitted using the analysis software GraphPad Prism 5 to obtain the $IC_{50}$ value of each compound for the enzyme activity.

The results are as shown in Table 4 below. In the *BRCA1*-mutated MDA-MB-436 human triple-negative breast cancer cell line and the *BRCA1*-normal MDA-MB-231 human triple-negative breast cancer cell line, the example compounds showed dose-dependent anti-proliferative activity against the *BRCA1*-mutated MDA-MB-436 cell line, and Examples 2, 3, and 4 could inhibit the proliferation of these cancer cells at single-digit nanomolar concentrations, which was similar to the reference compound AZD5305. For the *BRCA1*-normal MDA-MB-231 cell line, Examples 1, 2, and 4 showed no detectable inhibition on cell proliferation even at the highest concentration of 10 μM, which was consistent with the reference compound AZD5305, and the $IC_{50}$ of Example 3 was 7.4 μM, indicating that Examples 1, 2, 3, and 4 had obvious selectivity for the *BRCA1*-mutated cell line and the *BRCA1*-normal cells, with the selectivity reaching hundreds or thousands of folds. Compared with the marketed PARPi Olaparib, Examples 1, 2, 3, and 4 had an increased anti-proliferative activity against the *BRCA1*-mutated MDA-MB-436 cell line by 18- to 235-fold, and selectivity for PARP2 by 55- to 530-fold, significantly improving the inhibitory activity against PARP1 and the selectivity for PARP2.

As shown in Table 5, in the *BRCA2*-mutated DLD-1 *BRCA2*$^{-/-}$ and *BRCA2*-normal DLD-1 human colourectal adenocarcinoma epithelial cell lines, the example compounds all showed dose-dependent anti-proliferative activity against the BRCA2-mutated cell line, and Examples 2, 3, and 4 could inhibit the proliferation of these cancer cells at single-digit nanomolar concentrations, which was similar to the reference compound AZD5305. For the BRCA2-normal DLD-1 cell line, Examples 1, 2, 3, and 4 showed no detectable inhibition on cell proliferation even at the highest concentration of 10 μM, which was consistent with the reference compound AZD5305, indicating that Examples 1, 2, 3, and 4 had obvious selectivity for the BRCA2-mutated cell line and the BRCA2-normal cells, with the selectivity reaching hundreds or thousands of folds.

The anti-proliferative activity of the example compounds on the *BRCA1*-mutated and normal, and *BRCA2*-mutated and normal cancer cell lines indicated that the example compounds had strong anti-proliferative activity on target cells, and were targeted and safe when used in clinical treatment.

Table 4 Anti-proliferative capability of compounds against *BRCA1*-mutated and *BRCA1*-normal cancer cells

| No. | Compound No. | MDA-MB-436 $IC_{50}$ (nM) | MDA-MB-231 $IC_{50}$ (nM) | *BRCA1* mutation/WT selectivity fold |
|---|---|---|---|---|
| 1 | Example 1 | 16.45 | >10,000 | >608 |
| 2 | Example 2 | 5.74 | >10,000 | >1,742 |
| 3 | Example 3 | 1.27 | 7,402 | 5,828 |
| 4 | Example 4 | 1.93 | >10,000 | >5,181 |
| 5 | AZD5305 | 1.94 | >10,000 | >5,154 |
| 6 | Olaparib | 298.48 | 9521.25 | 11 |

Table 5 Anti-proliferative capability of compounds against *BRCA2*-mutated and *BRCA2*-normal cancer cells

| No. | Compound No. | **DLD-1** *BRCA2*$^{-/-}$ $IC_{50}$ (nM) | DLD-1 WT $IC_{50}$ (nM) | *BRCA2* mutation/WT selectivity fold |
|---|---|---|---|---|
| 1 | Example 1 | 18.6 | >10,000 | >538 |
| 2 | Example 2 | 8.62 | >10,000 | >1,162 |
| 3 | Example 3 | 5.07 | >10,000 | >1,972 |
| 4 | Example 4 | 4.59 | >10,000 | >2,178 |
| 5 | AZD5305 | 2.12 | >10,000 | >4,716 |

Taking into account the enzymatic activity at both molecular and cellular levels, the induced damaged DNA trapping capability of PARP, and the anti-proliferative activity against cancer cells, Examples 1, 2, 3, and 4 had excellent PARP1-selective inhibitory activity and anti-proliferative capability against target cells. They were significantly superior to the marketed PARPi Olaparib in terms of both PARP1 activity and selectivity for PARP2, and had a significantly improved selectivity for PARP1 at the cellular level compared with the reference compound AZD5305.

**Experimental example 5 Pharmacokinetic study of single administration in mice**

Male CD-1 mice were used as test animals. The plasma drug concentrations of the compounds were determined and the pharmacokinetic parameters were evaluated after a single administration. Healthy adult male CD-1 mice were selected. The candidate compound was added to 10% DMSO, vortexed and sonicated. After complete dissolution, 30% PEG400 was added, followed by vortexing and sonication, and 60% double-distilled water was added to prepare a clear solution for later use. After the mice were intravenously injected at a dose of 2 mg/kg and orally administered at a dose of 10 mg/kg, whole blood was collected at 5 minutes, 15 minutes, 30 minutes, 1 hour, 2 hours, 4 hours, 8 hours, and 24 hours after administration to prepare plasma. After sample treatment, the drug concentration was analysed by LC-MS/MS method, and the pharmacokinetic parameters were calculated.

The results are as shown in Table 6 below, indicating that the compound of Example 1 has good bioavailability and good pharmacokinetic properties.

Table 6 Pharmacokinetic parameters of compounds in CD-1 mice

| Parameter | Example 1 | |
|---|---|---|
| Route of administration | IV | PO |
| Clearance (mL/min/kg) | 0.872 | -- |
| Half-life (h) | 2.81 | 3.51 |
| Time to peak (h) | -- | 2 |
| Peak concentration (ng/mL) | -- | 15700 |
| Exposure last (h*ng/mL) | 38128 | 189560 |

(continued)

| Parameter | Example 1 | |
|---|---|---|
| Route of administration | IV | PO |
| Exposure inf (h*ng/mL) | 38222 | 191246 |
| Apparent volume of distribution (L/kg) | 0.218 | -- |
| Bioavailability (%) | -- | 100 |

It could be seen from the above experiment that the compounds of the present invention had high selectivity for the inhibition of PARP1 enzyme, and the inhibitory activity against PARP1 enzyme and at the cellular level was 10- to 100-fold or higher than that against PARP2 enzyme. Higher selectivity is expected to bring greater clinical safety and fewer toxic and side effects, making it more acceptable to patients and thus more valuable in medical applications.

**Experimental example 6 Selectivity of compounds for PARP family PARP3, 5a, 6, 7, and 11**

The enzyme activity of PARP family was detected by chemiluminescence. First, histone (Active Motif, 81126) was incubated on a 384-well plate for 2 hours.

Different dilutions of the example compounds and PARP3 working solution (BPS, 80503) were added. Only PARP working solution was added to the Max control well, and only assay buffer was added to the Min control well. The plate was incubated at room temperature for 15 minutes, and the biotinylated substrate $NAD^+$ (BPS, 80610) and PARP3 activating DNA (Generay) were added. The plate was incubated at room temperature for 2 hours. After the substrate was catalysed by the enzyme, the ADP-ribose group was bound to the histone and still remained biotinylated. Streptavidin-HRP solution (Abcam, ab7403) was added to develop colour via the biotin, and the values were read on the EnSight (PE) instrument.

Different dilutions of the example compounds and PARP5a working solution (BPS, 80504) were added. Only PARP working solution was added to the Max control well, and only assay buffer was added to the Min control well. The plate was incubated at room temperature for 15 minutes, and the biotinylated substrate $NAD^+$ (BPS, 80610) was added. The plate was incubated at room temperature for 2 hours. After the substrate was catalysed by the enzyme, the ADP-ribose group was bound to the histone and still remained biotinylated. Streptavidin-HRP solution (Abcam, ab7403) was added to develop colour via the biotin, and the values were read on the EnSight (PE) instrument.

Different dilutions of the example compounds and PARP6 working solution (BPS, 80506) were added. Only PARP working solution was added to the Max control well, and only assay buffer was added to the Min control well. The plate was incubated at room temperature for 15 minutes, and the biotinylated substrate $NAD^+$ (BPS, 80610) was added. The plate was incubated at room temperature for 2 hours. After the substrate was catalysed by the enzyme, the ADP-ribose group was bound to the histone and still remained biotinylated. Streptavidin-HRP solution (Abcam, ab7403) was added to develop colour via the biotin, and the values were read on the EnSight (PE) instrument.

PARP7 Chemiluminescent assay kit (BPS, 79729) was used, and different dilutions of the example compounds and PARP7 working solution (BPS, 80527) were added. Only PARP working solution was added to the Max control well, and only assay buffer was added to the Min control well. The plate was incubated at room temperature for 15 minutes, and the substrate mixture provided by the assay kit (BPS, 78371) was added. The plate was incubated at room temperature for 1 hour. After the substrate was catalysed by the enzyme, the ADP-ribose group was bound to the histone and still remained biotinylated. Streptavidin-HRP solution (BPS, 80611) was added to develop colour via the biotin, and the values were read on the EnSight (PE) instrument.

PARP11 Chemiluminescent assay kit (BPS, 80561) was used, and different dilutions of the example compounds and PARP11 working solution (BPS, 80511) were added. Only PARP working solution was added to the Max control well, and only assay buffer was added to the Min control well. The plate was incubated at room temperature for 15 minutes, and the substrate mixture provided by the assay kit (BPS, 78371) was added. The plate was incubated at room temperature for 1 hour. After the substrate was catalysed by the enzyme, the ADP-ribose group was bound to the histone and still remained biotinylated. Streptavidin-HRP solution (BPS, 80611) was added to develop colour via the biotin, and the values were read on the EnSight (PE) instrument.

The inhibition rate was calculated using the fluorescence values from the Max and Min wells, and the dose-response curve was fitted using the analysis software GraphPad Prism 5 to obtain the $IC_{50}$ value of each compound for the enzyme activity.

The results are as shown in Table 7 below. The inhibitory effect of Example 4 on each PARP enzyme was weaker than its inhibitory effect on the PARP1 enzyme activity; the values in brackets in Table 7 represented the ratios relative to the PARP1 enzyme activity, indicating that compared with the marketed PARPi Olaparib, Example 4 had a significantly

improved selectivity for other enzymes, except that its selectivity for PARP11 was slightly weaker; and compared with the reference compound AZD5305, Example 4 had a significantly improved selectivity for the tested PARP enzymes.

It indicated that Example 4 was a specific selective inhibitor of PARP1. Its selectivity for PARP3, PARP5a, PARP6, PARP7 and PARP11 was superior to that of AZD5305, with a particularly significant selective advantage especially for PARP11; and its selectivity for PARP3, PARP5a, PARP6 and PARP7 was superior to that of Olaparib, with a particularly significant selective advantage especially for PARP3.

Table 7 Inhibition of PARP3/PARP5a/PARP6/PARP7/PARP11 enzyme activity by compounds (nM)

| PARP family | Olaparib (multiple vs PARP1) | AZD5305 (multiple vs PARP1) | Example 4 (multiple vs PARP1) |
|---|---|---|---|
| PARP1 (Experimental example 1) | 0.50 | 0.42 | 0.48 |
| PARP3 | 23.5 (47) | 669.6 (1,594) | 7,646.0 (15,929) |
| PARP5a | 1,150.0 (2,300) | 2,903.0 (6,912) | 4,064.0 (8,467) |
| PARP6 | 152 (304) | 8,660 (20,619) | >10,000 (∞) |
| PARP7 | 904.4 (1,809) | 1558 (3,710) | >10,000 (∞) |
| PARP11 | 2,640 (5,280) | 19.4 (46) | 422.2 (880) |

**Experimental example 7 Effects of compounds on inducing PARP1/2-DNA trapping in FP experiments**

In order to further explore the effect of Example 4 on the PARP 1-DNA trapping and PARP2-DNA trapping, as well as the difference between the two, the fluorescence polarization (FP) method was used for further evaluation. The PARP1/2 enzymes bound to the fluorescently labelled DNA to form a larger complex, leading to slower rotation of the fluorescently labelled DNA and thus higher polarization value. Upon $NAD^+$ addition, the PARP1/2 enzymes autoribosylated and accumulated negative charges. When the negative charges accumulated to a certain extent, the fluorescently labelled DNA dissociated, causing faster rotation of the fluorescently labelled DNA and thus lower polarization value. Adding PARP inhibitors affected the PARP-DNA trapping, and the extent of the effect could be detected by changes in the intensity of polarized light. First, 25 nL of compounds at different concentrations with a final concentration of 1000-fold were transferred to a 384-well plate. 25 nL of 100% DMSO was added to the Min well and the Max well, respectively. 5 μL of enzyme solution containing 10 nM PARP1 (BPS, 80501) and 1 nM FAM-PARP1-DNA (Generay, customized) or 10 nM PARP2 (BPS, 80502) and 1 nM FAM-PARP2-DNA (Generay, customized) was added. The plate was centrifuged at 1000 rpm for 1 minute, and then incubated at room temperature for 30 minutes. 5 μL of 1 mM substrate $NAD^+$ (MCE, HY-B0445) was added, 5 μL of 1 mM substrate $NAD^+$ was added to the Min well, and assay buffer was added to the Max well. The plate was centrifuged at 1000 rpm for 1 minute and reacted at 25°C. The following different time points were used for the assay.

For PARP1 trapping assay, the values were read on the plate reader (Envision) at 0.25 hours, 0.5 hours, 1 hour, 2 hours, 4 hours, 8 hours, 24 hours, 30 hours, 48 hours and 54 hours.

For PARP2 trapping assay, the values were read on the plate reader at 0.25 hours, 0.5 hours, 1 hour, 2 hours, 4 hours, 8 hours, 24 hours and 48 hours.

The trapping capability induced by each compound was calculated using the fluorescence values from the Max and Min wells, and the dose-response curve was fitted using the analysis software GraphPad Prism 5 to obtain the $EC_{50}$ value of the DNA trapping capability of PARP enzymes induced by each compound.

The results are as shown in Table 8 and FIG. 1. Taking the 2-hour time point as an example, the capability of Example 4 to induce PARP1-DNA trapping was 48-fold higher than that of the marketed PARPi Olaparib, and was similar to that of the reference compound AZD5305. Example 4 had almost no capability to trapping PARP2-DNA, which was similar to the reference compound AZD5305. The marketed PARPi Olaparib showed a similar level of trapping for PARP2-DNA as it did for PARP1-DNA, indicating no selectivity.

This indicated that Example 4 had much higher selectivity for PARP1 than PARP2. It was expected to significantly reduce the haematotoxicity caused by PARP2.

As shown in Table 8 and FIG. 2, by further comparing the capability of Example 4 and the reference compound AZD5305 to trapping PARP1-DNA, it was found that over time, the activity of Example 4 could be maintained for more than 54 hours, and its activity was 14-fold stronger than that of the reference compound AZD5305 at the 54-hour time point. This suggested that it had better anti-tumour proliferation activity.

Table 8 Effects of compounds on PARP1/2-DNA trapping

| Target | Compound | Time (hour) | Bottom | Top | Hillslope | $EC_{50}$ (nM) |
|---|---|---|---|---|---|---|
| PARP1 | Olaparib | 0.25 | = 0.0 | = 100.0 | 0.9 | 13.9 |
| | | 0.5 | -1.8 | 110.9 | 0.7 | 41.4 |
| | | 1 | 1.5 | 105.6 | 1.3 | 137.2 |
| | | 2 | 1.0 | 90.55 | 1.3 | 288.8 |
| | | 4 | -0.3 | 97.61 | 0.8 | 1013.0 |
| | | 8 | -5.2 | = 100.0 | 0.6 | 4146.0 |
| | | 24 | -0.1 | = 100.0 | 0.7 | >10000 |
| | | 30 | -4.7 | = 100.0 | 1.0 | >10000 |
| | | 48 | -1.2 | = 100.0 | 1.2 | >10000 |
| | | 54 | -3.1 | = 100.0 | 0.8 | >10000 |
| | AZD5305 | 0.25 | -8.7 | 108.7 | 2.8 | 2.01 |
| | | 0.5 | -4.5 | 103.5 | 2.9 | 2.27 |
| | | 1 | -7.6 | 104.1 | 3.1 | 2.57 |
| | | 2 | -5.7 | 100.3 | 2.1 | 3.12 |
| | | 4 | -3.9 | 99.2 | 1.8 | 4.66 |
| | | 8 | -7.3 | 93.9 | 1.5 | 5.87 |
| | | 24 | -3.9 | 42.3 | 1.6 | 10.36 |
| | | 30 | -12.7 | 52.7 | 0.8 | 13.31 |
| | | 48 | -7.1 | 34.6 | 1.1 | 42.05 |
| | | 54 | -3.6 | 54.4 | 1.1 | 134.50 |
| | Example 4 | 0.25 | -8.7 | 108.7 | 2.8 | 3.11 |
| | | 0.5 | -6.3 | 105.6 | 2.9 | 2.26 |
| | | 1 | -4.2 | 109.1 | 3.1 | 2.58 |
| | | 2 | -0.8 | 105.3 | 3.0 | 2.86 |
| | | 4 | -5.6 | 106.8 | 2.4 | 2.99 |
| | | 8 | -6.8 | 107.2 | 2.6 | 3.51 |
| | | 24 | -4.4 | 79.2 | 1.5 | 3.97 |
| | | 30 | -8.8 | 79.1 | 1.2 | 5.46 |
| | | 48 | -5.6 | 51.0 | 2.0 | 4.58 |
| | | 54 | -12.8 | 55.9 | 0.8 | 9.54 |

(continued)

| Target | Compound | Time (hour) | Bottom | Top | Hillslope | $EC_{50}$ (nM) |
|---|---|---|---|---|---|---|
| PARP2 | Olaparib | 0.25 | -5.4 | 107.8 | 1.2 | 10.3 |
| | | 0.5 | -2.7 | 109.9 | 1.4 | 23.99 |
| | | 1 | 2.8 | 109.4 | 2.0 | 48.78 |
| | | 2 | -2.0 | 107.9 | 2.2 | 82.05 |
| | | 4 | -1.4 | 107.2 | 1.9 | 147.6 |
| | | 8 | 1.8 | 106.5 | 2.1 | 274.7 |
| | | 24 | 0.3 | 109.8 | 2.0 | 711.4 |
| | | 48 | -1.6 | 110.6 | 1.8 | 1181 |
| | AZD5305 | 0.25 | 1.5 | = 100.0 | 1.6 | 1911 |
| | | 0.5 | -2.8 | = 100.0 | 1.5 | 5081 |
| | | 1 | 0.3 | = 100.0 | 1.9 | >10000 |
| | | 2 | - | - | - | >10000 |
| | | 4 | - | - | - | >10000 |
| | | 8 | - | - | - | >10000 |
| | | 24 | - | - | - | >10000 |
| | | 48 | - | - | - | >10000 |
| | Example 4 | 0.25 | 4.3 | = 100.0 | 1.4 | 2327 |
| | | 0.5 | 1.0 | = 100.0 | 1.9 | 5469 |
| | | 1 | 0.8 | = 100.0 | 2.1 | >10000 |
| | | 2 | - | - | - | >10000 |
| | | 4 | - | - | - | >10000 |
| | | 8 | - | - | - | >10000 |
| | | 24 | - | - | - | >10000 |
| | | 48 | - | - | - | >10000 |

**Experimental example 8 Anti-tumour effects of compounds on human breast cancer cell MDA-MB-436 xenograft tumour mouse model**

Example 4 was further verified in vivo. A subcutaneous xenograft tumour mouse model of the *BRCA1*-mutated MDA-MB-436 cell line was selected. Human breast cancer MDA-MB-436 cells (ATCC, HTB-130) were cultured in vitro in a monolayer, with the culture conditions of L-15 medium supplemented with 10% foetal bovine serum, 1% penicillin-streptomycin solution, and 0.01 mg/mL bovine insulin, and cultured at 37°C in a CO2-free incubator. The cells were routinely digested and passaged twice a week using trypsin-EDTA. When the cell confluency reached 80%-90%, the cells were harvested and counted, and 0.2 mL of $1 \times 10^7$ MDA-MB-436 cells were subcutaneously inoculated on the right back of each mouse (PBS : Matrigel = 1 : 1). When the mean tumour volume reached 152 $mm^3$, the mice were grouped for drug administration. The day of drug administration was set as Day 0. The tumour volume and the body weight of the mice were monitored twice a week, and the drug treatment lasted for 28 days.

The diameter of the tumour was measured with a vernier calliper. The tumour volume is calculated according to the following equation: $V = 0.5a \times b^2$, where a and b represent the long and short diameters of the tumour, respectively. The tumour inhibition effect of the compound was evaluated by relative tumour shrinkage rate Reg%. Reg% reflects the rate of tumour volume reduction after treatment. Reg% = $(V_0-V_t)/V_0 \times 100\%$, where $V_0$ is the tumour volume measured at the time of grouping for drug administration (i.e., $d_0$), and $V_t$ is the tumour volume measured at a certain time point.

The criteria for compound response in the mouse model were adapted from mRECIST (Modified Response Evaluation Criteria in Solid Tumours) (Gao et al, 2015), and are specifically defined as follows:

CR (Complete Response): BestResponse < - 95% and BestAvgResponse < - 40%;

PR (Partial Response):

$$\text{BestResponse} < -50\% \text{ and BestAvgResponse} < -20\%;$$

SD (Stable Disease):

$$\text{BestResponse} < 35\% \text{ and BestAvgResponse} < 30\%;$$

PD (Progressive Disease): other cases;
ORR% is the sum of the proportions of Complete Response (CR) and Partial Response (PR).

Statistical analysis was performed using Prism software, including the mean value (Mean) and standard error of the mean (SEM) of the tumour volume at each time point for each group. For the statistical analysis of TV, the original TV data from each measurement were used to compare the differences between groups. Two-way ANOVA was applied to include the two factors, i.e., drug administration and time for analysis, followed by the Tukey's multiple comparisons test, wherein $p < 0.05$ was considered statistically significant.
The results are as shown in FIG. 3. At the same dose, both Example 4 and the reference compound AZD5305 promoted tumour regression. The degree of tumour regression in Example 4 was better than that in the reference compound AZD5305, with an ORR of 60%, while the ORR of the reference compound was 20%.
This indicated that in the subcutaneous xenograft tumour mouse model of human breast cancer MDA-MB-436, the anti-tumour growth effect of Example 4 was superior to that of the reference compound AZD5305.

**Experimental example 9 Pro-apoptotic effects of compounds on *BRCA2*-deficient cell lines**

The mechanism of the anti-proliferative effect of the compounds on *BRCA*-mutated tumours was analysed, and the impact of the compounds on apoptosis in the *BRCA2*-mutated cell line was analysed by flow cytometry. *BRCA2*-deficient human colon cancer DLD-1 cells (ATCC, HTB-130) were adherently cultured, with the culture conditions of RPMI1640 medium supplemented with 1% foetal bovine serum and 100 μg/mL hygromycin B. Once entering the logarithmic growth phase, the cells were collected and placed in a 6-well plate with 600,000 cells per well. The cells were allowed to adhere to the plate in a 37°C/5% $CO_2$ incubator overnight. Different concentrations of compounds were added to each well and incubated in the incubator. After 7 days, the cells were collected and placed in a 96-well plate. 195 μL of Annexin V-FITC binding buffer was added to each well to resuspend the cells. Then 5 μL of Annexin V-FITC (beyotime, C1052) was added and gently mixed. The cells were incubated at room temperature for 30 minutes, washed twice with PBS, and centrifuged at 300 g for 5 minutes. 500 μL of PBS was added to each well to resuspend the cells, and then 5 μL of PI (beyotime, C1052) was added and gently mixed. The cells were stained at room temperature for 30 minutes, washed once with PBS, and centrifuged at 300 g for 5 minutes. 500 μL of PBS was added to resuspend the cells, and 300 μL of the cells were transferred into a flow tube for flow cytometry analysis (BD bioscience, FACSVerse). The data acquired from the flow cytometer were analysed using FlowJo software.
For apoptosis analysis, the apoptosis samples were dragged into FlowJo, the raw data were double-clicked to open the graph window, and FSC-A was selected for the X-axis and SSC-A for the Y-axis. For the analysis of apoptotic cells, the cell population shown in the above plot was selected for analysis. Cell debris was excluded from analysis. The "analysed cell population" was double-clicked in the SSC/FSC plot. FITC was selected for the X-axis to represent AnnexinV-FITC, and PerCP was selected for the Y-axis to represent PI. The quadrant gate tool was used to define viable cells and apoptotic cells. Annexin V-positive cells were apoptotic cells, Annexin V-positive/PI-negative cells were early apoptotic cells, and Annexin V-positive/PI-positive cells were late apoptotic cells. Graphical analysis was performed using PRISM. For statistical analysis, two-way ANOVA was applied to include the two factors, i.e., compound type and concentration for analysis, followed by the Tukey's multiple comparisons test, wherein $p < 0.05$ was considered statistically significant.
The results are as shown in FIG. 4. The experiment was repeated three times, and it was found that Example 4 induced a higher proportion of apoptotic cells at different concentrations than the reference compound AZD5305, with some concentrations showing statistically significant differences. This suggested that Example 4 was superior to the reference compound AZD5305 in inducing apoptosis of *BRCA2*-deficient human colon cancer DLD-1 cells.

**Experimental example 10 Pro-apoptotic effects of compounds on *BRCA1*-mutated human breast cancer xenograft tumour**

The pro-apoptotic effect of the compounds was further verified in vivo. The changes of cleaved caspase-3 in subcutaneous xenograft tumour tissues of MDA-MB-436 mice were detected by immunoblotting. After 10 days of treatment with different compounds, the animals were euthanized 0.25 hours and 24 hours after the last administration, and the tumour tissues were obtained for analysis. The rapidly frozen tumour tissues were placed on dry ice, 350 μL of complete cell lysis buffer (containing 1% protease inhibitors and phosphatase inhibitors) was added, the tissues were disrupted using Tissue grinder for 5 minutes, and the tissue lysate was placed on ice for 30 minutes. Centrifugation was performed at 12,000 rpm and 4°C for 10 minutes, the supernatant was placed in a new 1.5 mL centrifuge tube, and protein quantification was carried out using a BCA quantification kit. According to the quantitative results, the sample protein concentration was uniformly adjusted to 2 μg/μL, LDS loading buffer (4X) and sample reducing agent (10X) were added, and the samples were heated at a constant temperature of 100°C for 10 minutes. For Western blotting, 10 μL of sample was loaded into each well of an SDS-PAGE gel, and electrophoresis was performed at 80 V for 30 minutes, followed by 120 V for 90 minutes. The proteins were transferred to the membrane using the iBlot2 transfer kit and a transfer apparatus for 7 minutes. The membrane was then cut according to the molecular weights of the proteins to be detected. The membrane was washed 3 times with 1xTBST, 5 minutes each time. The primary antibodies Cleaved Caspase-3 (Asp175) (5A1E) Rabbit mAb (CST, 9664), Caspase-3 Antibody (CST, 9662), and β-Actin Antibody (CST, 4967) were added and incubated overnight at 4°C. The membrane was then washed 3 times with 1xTBST, 10 minutes each time. The secondary antibody Goat anti-Rabbit IgG-HRP (Thermo fisher, 31462) was added and incubated at room temperature for 1 hour. The membrane was then washed 3 times with 1xTBST, 10 minutes each time. The HRP substrate from the West Femto Ultra-Sensitive Chemiluminescent Kit was added for chemiluminescence, and the chemiluminescent signals were detected on a Tanon 5200 Multi machine, with images acquired and saved. For quantitative analysis, Alpha View software was used to perform relative quantification of the density and intensity of immunoblot luminescent bands. β-Actin is a housekeeping protein used to monitor the consistency of sample loading in immunoblotting detection. The density and intensity of the Cleaved caspase-3 band were normalized to those of the total caspase-3 band. Using the relative density and intensity of Cleaved caspase-3 in the Vehicle control group as 1, the relative expression levels of cleaved caspase-3 in each treatment group were calculated and plotted. Prism software was used to plot and analyse the values, including the mean value and standard error of the mean (SEM) of the relative expression levels of cleaved caspase-3 for each group at each time point, and differences among groups were compared. For multiple group comparisons, one-way ANOVA was performed for analysis. If heterogeneity of variance was observed (significant difference in F value), the Games-Howell test was applied for analysis. If there was no significant difference in F value, Tukey's multiple comparisons test was applied for analysis. $p < 0.05$ was considered statistically significant.

The results are as shown in FIG. 5 and 6. Example 4 induced an increase in cleaved caspase-3 in subcutaneous xenograft tumour tissues of MDA-MB-436 mice. At 0.25 hours and 24 hours after the last administration, the degree of increase was superior to that of the reference compound AZD5305. This suggested that in *BRCA1*-mutated MDA-MB-436 tumours in vivo, Example 4 exhibited a better effect in promoting tumour cell apoptosis than the reference compound AZD5305, which was consistent with its superior tumour inhibition effect.

In conclusion, the compound of the present invention was a highly selective PARP1 inhibitor, which exhibited superior tumour growth inhibition compared with AZD5305 in the *BRCA1* MDA-MB-436 human breast cancer tumour model. This advantage was further confirmed by comparisons in promoting PARP1-DNA trapping and inducing cell apoptosis. Compared with the marketed PAPR inhibitors, the compound of the present invention had a significantly improved selectivity for PARP2, and compared with the reference compound AZD5305, it had a significantly improved selectivity for other PARP families. This suggested that the compounds of the present invention, such as Example 4, were expected to become safer and more effective highly selective PARP1 inhibitors, and could provide clinical patients with higher quality treatments.

Although the embodiments of the present invention have been shown and described, it can be understood by those of ordinary skill in the art that various changes, modifications, replacements and variations can be made to these embodiments without departing from the principles and spirits of the present invention, and the scope of the present invention is defined by the appended claims and their equivalents.

## Claims

1. A compound of formula (I), or a pharmaceutically acceptable salt, an isotopic variant, a tautomer, a stereoisomer, a prodrug, a polymorph, a hydrate or a solvate thereof:

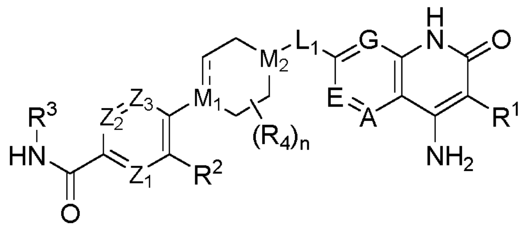

(I)

characterized in that$L_1$ is selected from CRR', O, S, NH, -C(O)-, -S(O)- or - $S(O)_2$-;
R and R' are independently selected from H, D, halogen, $C_{1-6}$ alkyl or $C_{1-6}$ haloalkyl;
A is selected from $CR_A$ or N;
E is selected from $CR_E$ or N;
G is selected from $CR_G$ or N;
$R_A$, $R_E$ and $R_G$ are independently selected from H, D, halogen, CN, -L-$OR_a$, - L-$SR_a$, -L-$NR_bR_c$, $C_{1-6}$ alkyl, $C_{1-6}$ haloalkyl, $C_{2-6}$ alkenyl, $C_{2-6}$ alkynyl, -L-$C_{3-10}$ cycloalkyl or -L-3- to 10-membered heterocyclyl;
$R_1$ is selected from H, D, halogen, CN, -L-$OR_a$, -L-$SR_a$, -L-$NR_bR_c$, $C_{1-6}$ alkyl, $C_{1-6}$ haloalkyl, $C_{2-6}$ alkenyl, $C_{2-6}$ alkynyl, -L-$C_{3-10}$ cycloalkyl, -L-3- to 10-membered heterocyclyl, -L-$C_{6-10}$ aryl or -L-5- to 10-membered heteroaryl, which is optionally substituted with 1, 2 or 3 $R_{1s}$;
$R_{1s}$ is independently selected from H, D, halogen, CN, $OR_a$, $SR_a$, $NR_bR_c$, $C_{1-6}$ alkyl, $C_{1-6}$ haloalkyl, $C_{2-6}$ alkenyl, $C_{2-6}$ alkynyl, $C_{3-10}$ cycloalkyl, 3- to 10-membered heterocyclyl, $C_{6-10}$ aryl or 5- to 10-membered heteroaryl;
- - - represents a single bond or a double bond;
$M_1$ is selected from N, C or $CR_5$;
$M_2$ is N or $CR_6$;
$R_5$ and $R_6$ are independently selected from H, D, halogen, $C_{1-6}$ alkyl or $C_{1-6}$ haloalkyl;
$R_4$ is independently selected from H, D, halogen, CN, =O, $OR_a$, $SR_a$, $NR_bR_c$, $C_{1-6}$ alkyl, $C_{1-6}$ haloalkyl, $C_{3-7}$ cycloalkyl or 3- to 7-membered heterocyclyl, which is optionally substituted with 1, 2 or 3 $R_{4s}$;
alternatively, $R_4$ located at the ortho position of $M_1$ and $R_2$ together with the atom to which they are attached form $C_{5-7}$ cycloalkyl or 5- to 7-membered heterocyclyl; alternatively, $R_4$ located at the ortho position of $M_1$ and $Z_3$ together with the atom to which they are attached form $C_{5-7}$ cycloalkyl or 5- to 7-membered heterocyclyl, which is optionally substituted with 1, 2 or 3 $R_{4s}$;
$R_{4s}$ is independently selected from H, D, halogen, CN, $C_{1-6}$ alkyl, $C_{1-6}$ haloalkyl, $C_{3-7}$ cycloalkyl or 3- to 7-membered heterocyclyl;
n is 0, 1, 2, 3 or 4;
$Z_1$ is selected from $CR_7$ or N;
$Z_2$ is selected from $CR_8$ or N;
$Z_3$ is selected from $CR_9$ or N;
$R_2$, $R_7$, $R_8$ and $R_9$ are independently selected from H, D, halogen, CN, $OR_a$, $SR_a$, $NR_bR_c$, $C_{1-6}$ alkyl, $C_{1-6}$ haloalkyl, $C_{3-7}$ cycloalkyl or 3- to 7-membered heterocyclyl, which is optionally substituted with 1, 2 or 3 $R_{2s}$;
$R_{2s}$ is independently selected from H, D, halogen, CN, $C_{1-6}$ alkyl, $C_{1-6}$ haloalkyl, $C_{3-7}$ cycloalkyl or 3- to 7-membered heterocyclyl;
$R_3$ is selected from H, -L-$OR_a$, -L-$SR_a$, -L-$NR_bR_c$, $C_{1-6}$ alkyl, $C_{1-6}$ haloalkyl, $C_{2-6}$ alkenyl, $C_{2-6}$ alkynyl, -L-$C_{3-10}$ cycloalkyl, -L-3- to 10-membered heterocyclyl, -L-$C_{6-10}$ aryl or -L-5- to 10-membered heteroaryl, which is optionally substituted with 1, 2 or 3 $R_{3s}$;
$R_{3s}$ is independently selected from H, D, halogen, CN, $OR_a$, $SR_a$, $NR_bR_c$, $C_{1-6}$ alkyl, $C_{1-6}$ haloalkyl, $C_{2-6}$ alkenyl, $C_{2-6}$ alkynyl, $C_{3-10}$ cycloalkyl, 3- to 10-membered heterocyclyl, $C_{6-10}$ aryl or 5- to 10-membered heteroaryl;
L is a chemical bond, $C_{1-6}$ alkylene, $C_{2-6}$ alkenylene or $C_{2-6}$ alkynylene, which is optionally substituted with 1, 2 or 3 groups selected from H, D, halogen, $C_{1-6}$ alkyl, $C_{1-6}$ haloalkyl, $C_{2-6}$ alkenyl or $C_{2-6}$ alkynyl;
$R_a$, $R_b$ and $R_c$ are each independently selected from H, $C_{1-6}$ alkyl, $C_{1-6}$ haloalkyl, $C_{3-10}$ cycloalkyl or 3- to 10-membered heterocyclyl; alternatively, $R_b$ and $R_c$ together with the atom to which they are attached form 5- to 10-membered heterocyclyl;
wherein each of the above-defined groups is optionally deuterated, up to fully deuterated.

2. The compound of formula (I), or the pharmaceutically acceptable salt, the isotopic variant, the tautomer, the stereoisomer, the prodrug, the polymorph, the hydrate or the solvate thereof according to claim 1, **characterized in that** $L_1$ is selected from CRR', O, S, NH or -C(O)-, preferably CRR' or -C(O)-, preferably CRR', and preferably $CH_2$.

3. The compound of formula (I), or the pharmaceutically acceptable salt, the isotopic variant, the tautomer, the

stereoisomer, the prodrug, the polymorph, the hydrate or the solvate thereof according to claim 1 or 2, **characterized in that** R and R' are independently selected from H, D, $C_{1-6}$ alkyl or $C_{1-6}$ haloalkyl, preferably selected from H, D, $C_{1-3}$ alkyl or $C_{1-3}$ haloalkyl, preferably H or D, and preferably H.

**4.** The compound of formula (I), or the pharmaceutically acceptable salt, the isotopic variant, the tautomer, the stereoisomer, the prodrug, the polymorph, the hydrate or the solvate thereof according to claims 1 to 3, **characterized in that** A is N;

preferably, E is $CR_E$;
preferably, G is $CR_G$;
preferably,

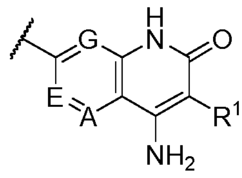 is 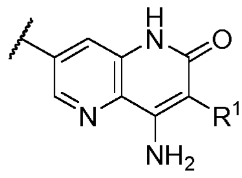.

**5.** The compound of formula (I), or the pharmaceutically acceptable salt, the isotopic variant, the tautomer, the stereoisomer, the prodrug, the polymorph, the hydrate or the solvate thereof according to any one of claims 1 to 4, **characterized in that** $R_A$, $R_E$ and $R_G$ are independently selected from H, D, halogen, CN, -L-$OR_a$, -L-$SR_a$, -L-$NR_bR_c$, $C_{1-6}$ alkyl, $C_{1-6}$ haloalkyl, -L-$C_{3-7}$ cycloalkyl or -L-3- to 7-membered heterocyclyl, preferably selected from H, D, halogen, CN, $OR_a$, $NR_bR_c$, $C_{1-6}$ alkyl, $C_{1-6}$ haloalkyl, $C_{3-7}$ cycloalkyl or 3- to 7-membered heterocyclyl, preferably selected from H, D, halogen, $C_{1-6}$ alkyl or $C_{1-6}$ haloalkyl, preferably H or D, and preferably H.

**6.** The compound of formula (I), or the pharmaceutically acceptable salt, the isotopic variant, the tautomer, the stereoisomer, the prodrug, the polymorph, the hydrate or the solvate thereof according to any one of claims 1 to 5, **characterized in that** $R_1$ is selected from H, D, halogen, CN, -L-$OR_a$, -L-$SR_a$, -L-$NR_bR_c$, $C_{1-6}$ alkyl, $C_{1-6}$ haloalkyl, -L-$C_{3-10}$ cycloalkyl, -L-3- to 10-membered heterocyclyl, -L-$C_{6-10}$ aryl or -L-5- to 10-membered heteroaryl, preferably selected from H, D, halogen, CN, $OR_a$, $NR_bR_c$, $C_{1-6}$ alkyl, $C_{1-6}$ haloalkyl, $C_{3-10}$ cycloalkyl, 3- to 10-membered heterocyclyl, $C_{6-10}$ aryl or 5- to 10-membered heteroaryl, preferably selected from H, D, halogen, CN, $OR_a$, $C_{1-6}$ alkyl, $C_{1-6}$ haloalkyl, $C_{3-7}$ cycloalkyl or 3- to 7-membered heterocyclyl, preferably selected from H, D, halogen, $C_{1-6}$ alkyl or $C_{1-6}$ haloalkyl, preferably selected from H, D, $C_{1-6}$ alkyl or $C_{1-6}$ haloalkyl; preferably $C_{1-6}$ alkyl or $C_{1-6}$ haloalkyl, preferably Et.

**7.** The compound of formula (I), or the pharmaceutically acceptable salt, the isotopic variant, the tautomer, the stereoisomer, the prodrug, the polymorph, the hydrate or the solvate thereof according to any one of claims 1 to 6, **characterized in that** $R_{1s}$ is independently selected from H, D, halogen, CN, $OR_a$, $NR_bR_c$, $C_{1-6}$ alkyl, $C_{1-6}$ haloalkyl, $C_{3-10}$ cycloalkyl, 3- to 10-membered heterocyclyl, $C_{6-10}$ aryl or 5- to 10-membered heteroaryl, preferably selected from H, D, halogen, CN, $C_{1-6}$ alkyl, $C_{1-6}$ haloalkyl, $C_{3-7}$ cycloalkyl or 3- to 7-membered heterocyclyl, preferably selected from H, D, halogen, $C_{1-6}$ alkyl or $C_{1-6}$ haloalkyl, and preferably selected from H, D, $C_{1-6}$ alkyl or $C_{1-6}$ haloalkyl.

**8.** The compound of formula (I), or the pharmaceutically acceptable salt, the isotopic variant, the tautomer, the stereoisomer, the prodrug, the polymorph, the hydrate or the solvate thereof according to any one of claims 1 to 7, **characterized in that** $M_1$ is selected from N or $CR_5$, preferably N;

preferably, $M_2$ is N;
preferably,

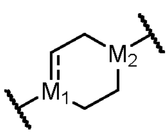 is 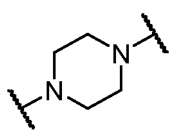.

**9.** The compound of formula (I), or the pharmaceutically acceptable salt, the isotopic variant, the tautomer, the stereoisomer, the prodrug, the polymorph, the hydrate or the solvate thereof according to any one of claims 1 to

8, **characterized in that** $R_5$ and $R_6$ are independently selected from H, D, $C_{1-6}$ alkyl or $C_{1-6}$ haloalkyl, preferably H or D, and preferably H.

**10.** The compound of formula (I), or the pharmaceutically acceptable salt, the isotopic variant, the tautomer, the stereoisomer, the prodrug, the polymorph, the hydrate or the solvate thereof according to any one of claims 1 to 9, **characterized in that** $R_4$ is independently selected from H, D, halogen, CN, $OR_a$, $NR_bR_c$, $C_{1-6}$ alkyl, $C_{1-6}$ haloalkyl, $C_{3-7}$ cycloalkyl or 3- to 7-membered heterocyclyl, preferably selected from H, D, halogen, CN, $C_{1-6}$ alkyl, $C_{1-6}$ haloalkyl, $C_{3-7}$ cycloalkyl or 3- to 7-membered heterocyclyl, preferably selected from H, D, halogen, $C_{1-6}$ alkyl or $C_{1-6}$ haloalkyl, preferably selected from H, D, $C_{1-6}$ alkyl or $C_{1-6}$ haloalkyl; preferably H or D, and preferably H; preferably, n is 0, 1 or 2, preferably 0.

**11.** The compound of formula (I), or the pharmaceutically acceptable salt, the isotopic variant, the tautomer, the stereoisomer, the prodrug, the polymorph, the hydrate or the solvate thereof according to any one of claims 1 to 10, **characterized in that** $R_4$ located at the ortho position of $M_1$ and $R_2$ together with the atom to which they are attached form 5- to 7-membered heterocyclyl; alternatively, $R_4$ located at the ortho position of $M_1$ and $Z_3$ together with the atom to which they are attached form 5- to 7-membered heterocyclyl;
preferably, $R_4$ located at the ortho position of $M_1$ and $R_2$ together with the atom to which they are attached form 5- to 7-membered heterocyclyl.

**12.** The compound of formula (I), or the pharmaceutically acceptable salt, the isotopic variant, the tautomer, the stereoisomer, the prodrug, the polymorph, the hydrate or the solvate thereof according to any one of claims 1 to 11, **characterized in that** $R_{4s}$ is independently selected from H, D, halogen, $C_{1-6}$ alkyl or $C_{1-6}$ haloalkyl.

**13.** The compound of formula (I), or the pharmaceutically acceptable salt, the isotopic variant, the tautomer, the stereoisomer, the prodrug, the polymorph, the hydrate or the solvate thereof according to any one of claims 1 to 12, **characterized in that** $Z_1$ is N;

preferably, $Z_2$ is $CR_8$;
preferably, $Z_3$ is $CR_9$;
preferably,

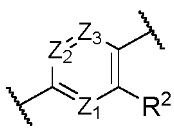
is
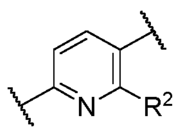
.

**14.** The compound of formula (I), or the pharmaceutically acceptable salt, the isotopic variant, the tautomer, the stereoisomer, the prodrug, the polymorph, the hydrate or the solvate thereof according to any one of claims 1 to 13, **characterized in that** $R_7$, $R_8$ and $R_9$ are independently selected from H, D, halogen, CN, $C_{1-6}$ alkyl, $C_{1-6}$ haloalkyl, $C_{3-7}$ cycloalkyl or 3- to 7-membered heterocyclyl, preferably selected from H, D, halogen, $C_{1-6}$ alkyl or $C_{1-6}$ haloalkyl, preferably selected from H, D, $C_{1-6}$ alkyl or $C_{1-6}$ haloalkyl; preferably H or D, and preferably H.

**15.** The compound of formula (I), or the pharmaceutically acceptable salt, the isotopic variant, the tautomer, the stereoisomer, the prodrug, the polymorph, the hydrate or the solvate thereof according to any one of claims 1 to 14, **characterized in that** $R_2$ is selected from H, D, halogen, CN, $C_{1-6}$ alkyl, $C_{1-6}$ haloalkyl, $C_{3-7}$ cycloalkyl or 3- to 7-membered heterocyclyl, preferably selected from H, D, halogen, CN, $C_{1-6}$ alkyl or $C_{1-6}$ haloalkyl, preferably selected from H, D, halogen, $C_{1-6}$ alkyl or $C_{1-6}$ haloalkyl, preferably selected from H, D or halogen, and preferably H or F.

**16.** The compound of formula (I), or the pharmaceutically acceptable salt, the isotopic variant, the tautomer, the stereoisomer, the prodrug, the polymorph, the hydrate or the solvate thereof according to any one of claims 1 to 15, **characterized in that** $R_{2s}$ is independently selected from H, D, halogen, $C_{1-6}$ alkyl or $C_{1-6}$ haloalkyl.

**17.** The compound of formula (I), or the pharmaceutically acceptable salt, the isotopic variant, the tautomer, the stereoisomer, the prodrug, the polymorph, the hydrate or the solvate thereof according to any one of claims 1 to 16, **characterized in that** $R_3$ is selected from H, $-L-OR_a$, $-L-SR_a$, $-L-NR_bR_c$, $C_{1-6}$ alkyl, $C_{1-6}$ haloalkyl, $-L-C_{3-10}$ cycloalkyl, -L-3- to 10-membered heterocyclyl, $-L-C_{6-10}$ aryl or -L-5- to 10-membered heteroaryl, preferably selected from H, $OR_a$, $NR_bR_c$, $C_{1-6}$ alkyl, $C_{1-6}$ haloalkyl, $C_{3-10}$ cycloalkyl, 3- to 10-membered heterocyclyl, $C_{6-10}$ aryl or 5- to 10-membered heteroaryl, preferably selected from H, $OR_a$, $C_{1-6}$ alkyl, $C_{1-6}$ haloalkyl, $C_{3-7}$ cycloalkyl or 3- to 7-membered heterocyclyl, preferably selected from H, $OR_a$, $C_{1-6}$ alkyl, $C_{1-6}$ haloalkyl or $C_{1-6}$ deuterated alkyl, preferably selected from $OR_a$, $C_{1-6}$ alkyl, $C_{1-6}$ haloalkyl or $C_{1-6}$ deuterated alkyl, and preferably selected from Me, $CD_3$ or OMe.

**18.** The compound of formula (I), or the pharmaceutically acceptable salt, the isotopic variant, the tautomer, the stereoisomer, the prodrug, the polymorph, the hydrate or the solvate thereof according to any one of claims 1 to 17, **characterized in that** $R_{3s}$ is independently selected from H, D, halogen, CN, $OR_a$, $NR_bR_c$, $C_{1-6}$ alkyl, $C_{1-6}$ haloalkyl, $C_{3-10}$ cycloalkyl, 3- to 10-membered heterocyclyl, $C_{6-10}$ aryl or 5- to 10-membered heteroaryl, preferably selected from H, D, halogen, CN, $C_{1-6}$ alkyl, $C_{1-6}$ haloalkyl, $C_{3-7}$ cycloalkyl or 3- to 7-membered heterocyclyl, preferably selected from H, D, halogen, $C_{1-6}$ alkyl or $C_{1-6}$ haloalkyl, and preferably selected from H, D, $C_{1-6}$ alkyl or $C_{1-6}$ haloalkyl.

**19.** The compound of formula (I), or the pharmaceutically acceptable salt, the isotopic variant, the tautomer, the stereoisomer, the prodrug, the polymorph, the hydrate or the solvate thereof according to any one of claims 1 to 18, **characterized in that** L is independently selected from a chemical bond or $C_{1-6}$ alkylene, which is optionally substituted with 1, 2 or 3 groups selected from H, D, halogen, $C_{1-6}$ alkyl or $C_{1-6}$ haloalkyl.

**20.** The compound of formula (I), or the pharmaceutically acceptable salt, the isotopic variant, the tautomer, the stereoisomer, the prodrug, the polymorph, the hydrate or the solvate thereof according to any one of claims 1 to 19, **characterized in that** $R_a$, $R_b$ and $R_c$ are each independently selected from H, $C_{1-6}$ alkyl, $C_{1-6}$ haloalkyl, $C_{3-7}$ cycloalkyl or 3- to 7-membered heterocyclyl, preferably selected from H, $C_{1-6}$ alkyl or $C_{1-6}$ haloalkyl, preferably $C_{1-6}$ alkyl or $C_{1-6}$ haloalkyl, preferably H or Me, and more preferably Me;
alternatively, $R_b$ and $R_c$ together with the atom to which they are attached form 5- to 7-membered heterocyclyl.

**21.** The compound of formula (I), or the pharmaceutically acceptable salt, the isotopic variant, the tautomer, the stereoisomer, the prodrug, the polymorph, the hydrate or the solvate thereof according to any one of claims 1 to 20, **characterized by** having the following structural formula:

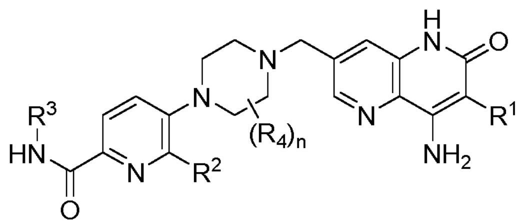

(II)

wherein each group is as defined in claims 1-20.

**22.** The compound of formula (II), or the pharmaceutically acceptable salt, the isotopic variant, the tautomer, the stereoisomer, the prodrug, the polymorph, the hydrate or the solvate thereof according to claim 21, **characterized in that**

$R_1$ is selected from H, D, halogen, CN, $OR_a$, $NR_bR_c$, $C_{1-6}$ alkyl, $C_{1-6}$ haloalkyl, $C_{3-10}$ cycloalkyl, 3- to 10-membered heterocyclyl, $C_{6-10}$ aryl or 5- to 10-membered heteroaryl, which is optionally substituted with 1, 2 or 3 $R_{1s}$;
$R_2$ is selected from H, D, halogen, CN, $C_{1-6}$ alkyl, $C_{1-6}$ haloalkyl, $C_{3-7}$ cycloalkyl or 3- to 7-membered heterocyclyl, which is optionally substituted with 1, 2 or 3 $R_{2s}$;
$R_3$ is selected from H, $OR_a$, $NR_bR_c$, $C_{1-6}$ alkyl, $C_{1-6}$ haloalkyl, $C_{3-10}$ cycloalkyl, 3- to 10-membered heterocyclyl, $C_{6-10}$ aryl or 5- to 10-membered heteroaryl, which is optionally substituted with 1, 2 or 3 $R_{3s}$;
$R_4$ is selected from H, D, halogen, CN, $C_{1-6}$ alkyl, $C_{1-6}$ haloalkyl, $C_{3-7}$ cycloalkyl or 3- to 7-membered heterocyclyl, which is optionally substituted with 1, 2 or 3 $R_{4s}$;
alternatively, $R_4$ located at the ortho position of N attached to pyridine and $R_2$ together with the atom to which they are attached form 5- to 7-membered heterocyclyl;
$R_{1s}$ and $R_{3s}$ are each independently selected from H, D, halogen, CN, $OR_a$, $NR_bR_c$, $C_{1-6}$ alkyl, $C_{1-6}$ haloalkyl, $C_{3-10}$ cycloalkyl, 3- to 10-membered heterocyclyl, $C_{6-10}$ aryl or 5- to 10-membered heteroaryl;
$R_{2s}$ and $R_{4s}$ are each independently selected from H, D, halogen, $C_{1-6}$ alkyl or $C_{1-6}$ haloalkyl;
n is 0, 1, 2, 3 or 4;
$R_a$, $R_b$ and $R_c$ are each independently selected from H, $C_{1-6}$ alkyl, $C_{1-6}$ haloalkyl, $C_{3-7}$ cycloalkyl or 3- to 7-membered heterocyclyl; alternatively, $R_b$ and $R_c$ together with the atom to which they are attached form 5- to 7-membered heterocyclyl;
wherein each of the above-defined groups is optionally deuterated, up to fully deuterated.

**23.** The compound of formula (II), or the pharmaceutically acceptable salt, the isotopic variant, the tautomer, the stereoisomer, the prodrug, the polymorph, the hydrate or the solvate thereof according to claim 21 or 22, **characterized in that**

$R_1$ is selected from H, D, halogen, CN, $OR_a$, $C_{1-6}$ alkyl, $C_{1-6}$ haloalkyl, $C_{3-7}$ cycloalkyl or 3- to 7-membered heterocyclyl, which is optionally substituted with 1, 2 or 3 $R_{1s}$;
$R_2$ is selected from H, D, halogen, CN, $C_{1-6}$ alkyl or $C_{1-6}$ haloalkyl, which is optionally substituted with 1, 2 or 3 $R_{2s}$;
$R_3$ is selected from H, $OR_a$, $C_{1-6}$ alkyl, $C_{1-6}$ haloalkyl, $C_{3-7}$ cycloalkyl or 3- to 7-membered heterocyclyl, which is optionally substituted with 1, 2 or 3 $R_{3s}$;
$R_4$ is selected from H, D, halogen, $C_{1-6}$ alkyl or $C_{1-6}$ haloalkyl, which is optionally substituted with 1, 2 or 3 $R_{4s}$;
$R_{1s}$ and $R_{3s}$ are each independently selected from H, D, halogen, CN, $C_{1-6}$ alkyl, $C_{1-6}$ haloalkyl, $C_{3-7}$ cycloalkyl or 3- to 7-membered heterocyclyl;
$R_{2s}$ and $R_{4s}$ are each independently selected from H, D, halogen, $C_{1-6}$ alkyl or $C_{1-6}$ haloalkyl;
n is 0, 1, 2, 3 or 4;
$R_a$ is independently selected from H, $C_{1-6}$ alkyl or $C_{1-6}$ haloalkyl.

**24.** The compound of formula (II), or the pharmaceutically acceptable salt, the isotopic variant, the tautomer, the stereoisomer, the prodrug, the polymorph, the hydrate or the solvate thereof according to any one of claims 21 to 23, **characterized in that**

$R_1$ is selected from H, D, $C_{1-6}$ alkyl or $C_{1-6}$ haloalkyl;
$R_2$ is selected from H, D, halogen, $C_{1-6}$ alkyl or $C_{1-6}$ haloalkyl;
$R_3$ is selected from H, $OR_a$, $C_{1-6}$ alkyl, $C_{1-6}$ haloalkyl or $C_{1-6}$ deuterated alkyl;
$R_4$ is selected from H, D, $C_{1-6}$ alkyl or $C_{1-6}$ haloalkyl;
n is 0, 1, 2, 3 or 4;
$R_a$ is selected from H, $C_{1-6}$ alkyl or $C_{1-6}$ haloalkyl.

**25.** The compound of formula (II), or the pharmaceutically acceptable salt, the isotopic variant, the tautomer, the stereoisomer, the prodrug, the polymorph, the hydrate or the solvate thereof according to any one of claims 21 to 24, **characterized in that**

$R_1$ is $C_{1-6}$ alkyl or $C_{1-6}$ haloalkyl, preferably Et;
$R_2$ is selected from H, D or halogen, preferably H or F;
$R_3$ is selected from $OR_a$, $C_{1-6}$ alkyl, $C_{1-6}$ haloalkyl or $C_{1-6}$ deuterated alkyl, preferably selected from Me, $CD_3$ or OMe;
$R_4$ is H or D, preferably H;
n is 0, 1 or 2, preferably 0;
$R_a$ is $C_{1-6}$ alkyl or $C_{1-6}$ haloalkyl, preferably Me;
preferably, $R_3$ is selected from $C_{1-6}$ alkyl, $C_{1-6}$ haloalkyl and $C_{1-6}$ deuterated alkyl, preferably $C_{1-6}$ deuterated alkyl, preferably selected from Me and $CD_3$, and preferably $CD_3$;
preferably, $R_2$ is halogen, preferably F, Cl or Br, and preferably F.

**26.** The compound of formula (I), or the pharmaceutically acceptable salt, the isotopic variant, the tautomer, the stereoisomer, the prodrug, the polymorph, the hydrate or the solvate thereof according to claim 1, **characterized in that** the compound is selected from the following:

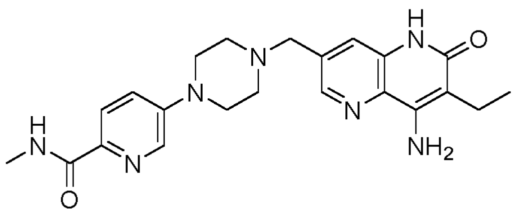

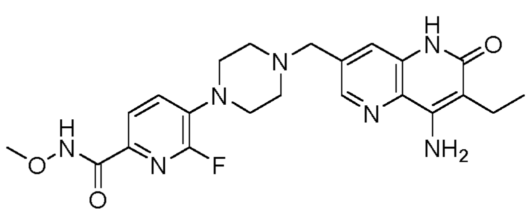

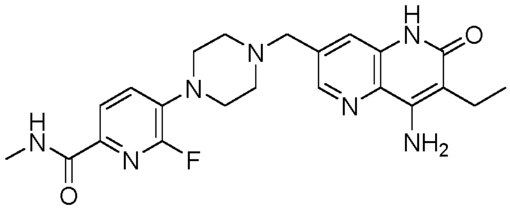 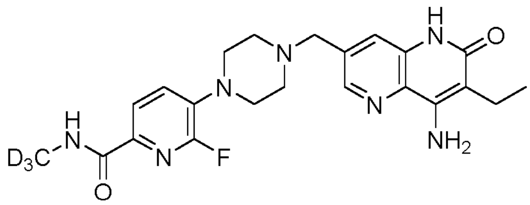.

**27.** A pharmaceutical composition, **characterized by** comprising the compound, or the pharmaceutically acceptable salt, the isotopic variant, the tautomer, the stereoisomer, the prodrug, the polymorph, the hydrate or the solvate thereof according to any one of claims 1 to 26, and a pharmaceutically acceptable carrier, adjuvant or vehicle, and optionally an additional therapeutic agent.

**28.** Use of the compound, or the pharmaceutically acceptable salt, the isotopic variant, the tautomer, the stereoisomer, the prodrug, the polymorph, the hydrate or the solvate thereof according to any one of claims 1 to 26, or the pharmaceutical composition according to claim 27 in the preparation of a medicament for treating or preventing a PARP-mediated disease, **characterized in that** preferably, the PARP is PARP1.

**29.** A method for treating or preventing a PARP-mediated disease in a subject, **characterized by** comprising administering to the subject the compound, or the pharmaceutically acceptable salt, the isotopic variant, the tautomer, the stereoisomer, the prodrug, the polymorph, the hydrate or the solvate thereof according to any one of claims 1 to 26, or the pharmaceutical composition according to claim 27, wherein preferably, the PARP is PARP1.

**30.** The compound, or the pharmaceutically acceptable salt, the isotopic variant, the tautomer, the stereoisomer, the prodrug, the polymorph, the hydrate or the solvate thereof according to any one of claims 1 to 26, or the pharmaceutical composition according to claim 27 for use in the treatment or prevention of a PARP-mediated disease, **characterized in that** preferably, the PARP is PARP1.

**31.** The use according to claim 28 or the method according to claim 29 or the compound or pharmaceutical composition for use according to claim 30, **characterized in that** the disease is selected from cancer, an ischemic disease and a neurodegenerative disease.

**32.** The use or method according to claim 31, **characterized in that** the cancer lacks an HR-dependent DNA DSB repair pathway.

**33.** The use or method according to claim 31, **characterized in that** the cancer has a BRCA1- or BRCA2-deficient phenotype.

**34.** The use or method according to claim 31, **characterized in that** the cancer is selected from breast cancer, ovarian cancer, pancreatic cancer, prostate cancer, blood cancer, gastrointestinal cancer and lung cancer.

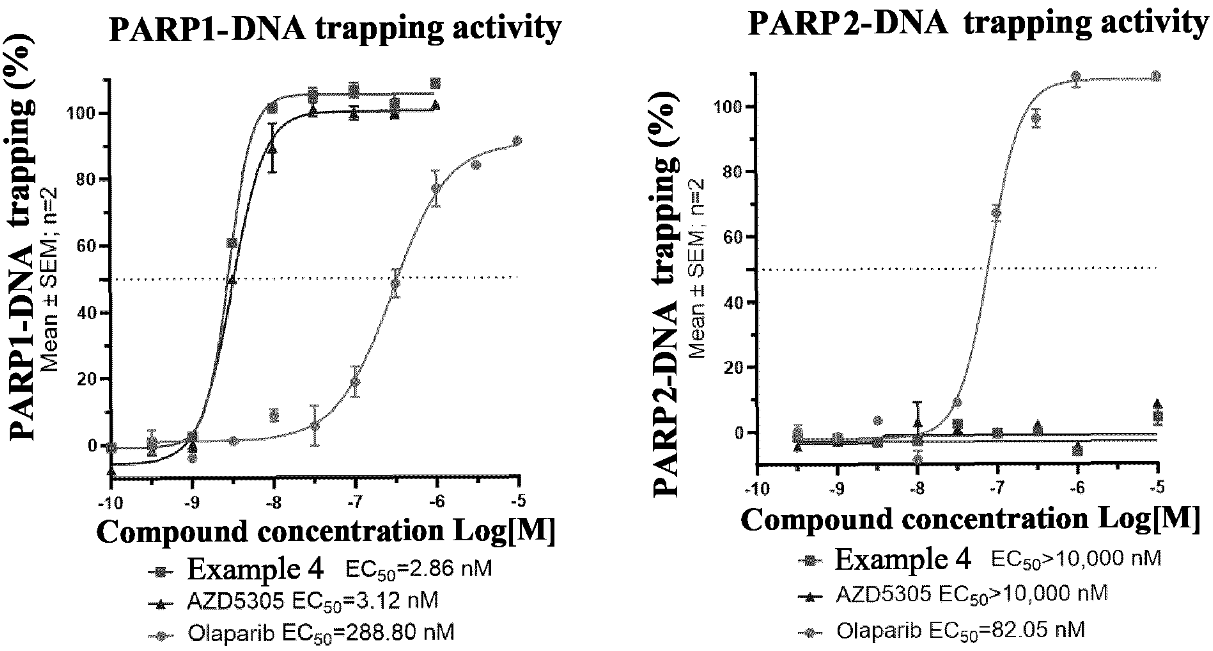
PARP1-DNA trapping activity
PARP1-DNA trapping (%)
Mean ± SEM; n=2
Compound concentration Log[M]
Example 4 $EC_{50}$=2.86 nM
AZD5305 $EC_{50}$=3.12 nM
Olaparib $EC_{50}$=288.80 nM
PARP2-DNA trapping activity
PARP2-DNA trapping (%)
Mean ± SEM; n=2
Compound concentration Log[M]
Example 4 $EC_{50}$>10,000 nM
AZD5305 $EC_{50}$>10,000 nM
Olaparib $EC_{50}$=82.05 nM

*FIG. 1*

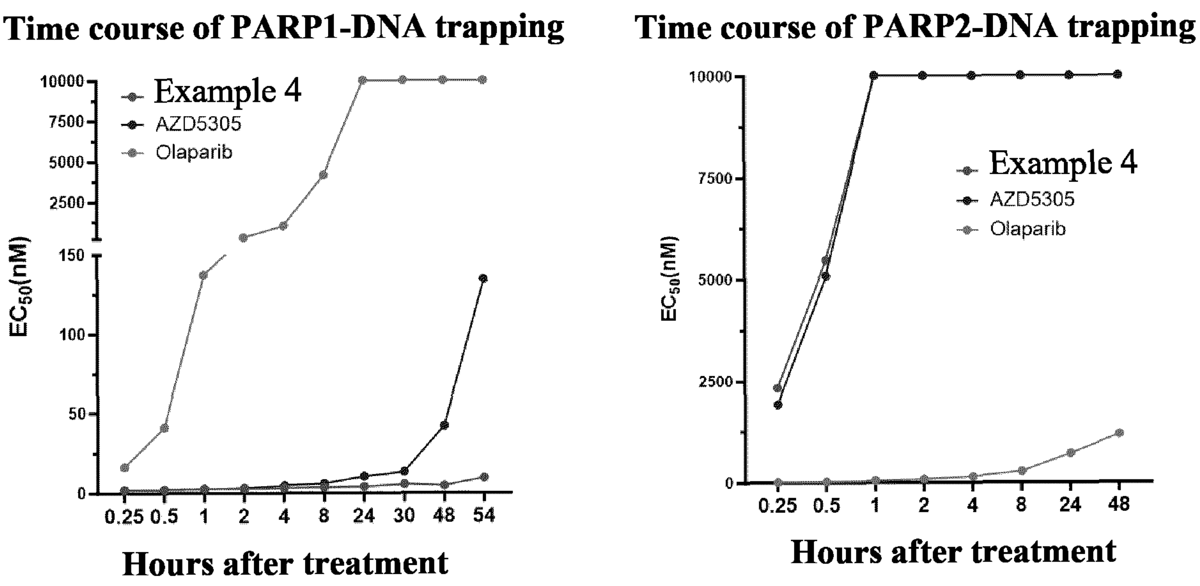
Time course of PARP1-DNA trapping
$EC_{50}$(nM)
Example 4
AZD5305
Olaparib
Hours after treatment
Time course of PARP2-DNA trapping
$EC_{50}$(nM)
Example 4
AZD5305
Olaparib
Hours after treatment

*FIG. 2*

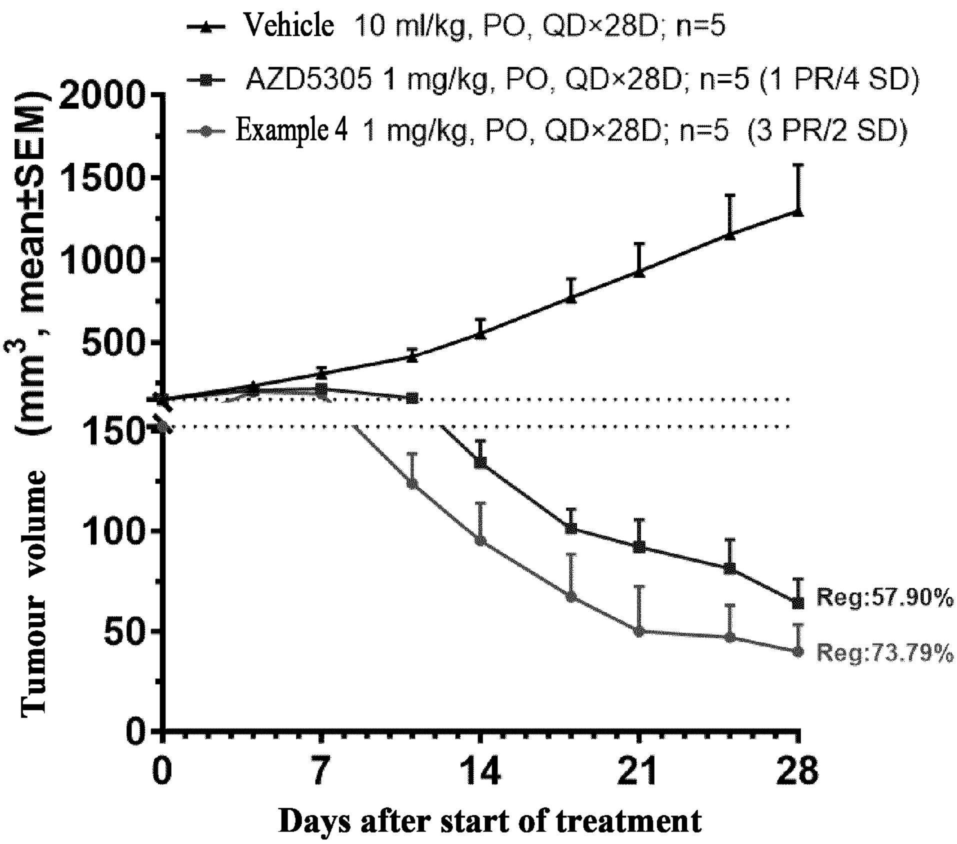
Vehicle 10 ml/kg, PO, QD×28D; n=5
AZD5305 1 mg/kg, PO, QD×28D; n=5 (1 PR/4 SD)
Example 4 1 mg/kg, PO, QD×28D; n=5 (3 PR/2 SD)
Tumour volume (mm3, mean±SEM)
Days after start of treatment
Reg:57.90%
Reg:73.79%

*FIG. 3*

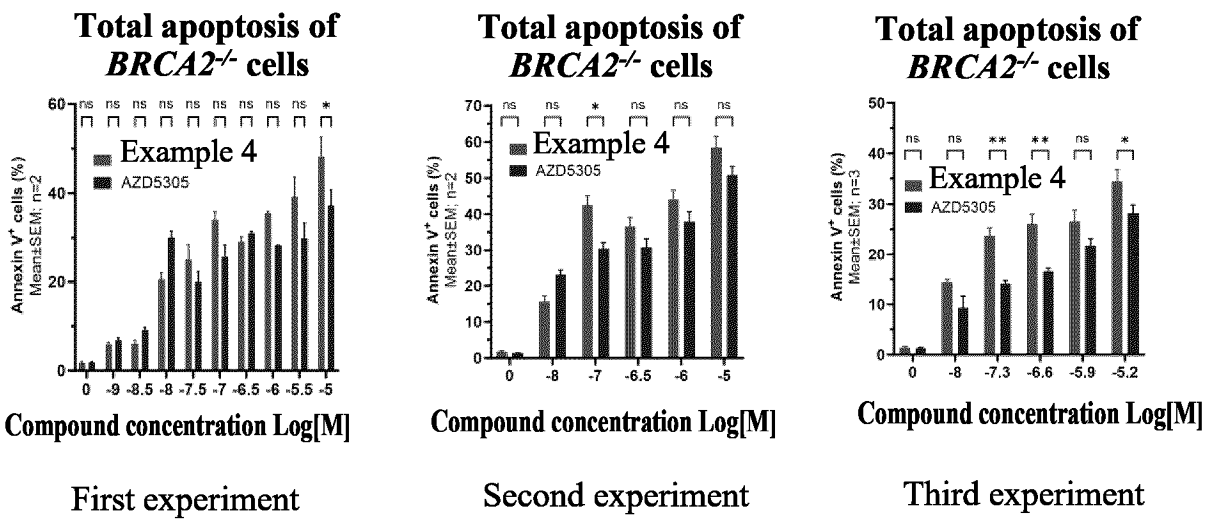
Total apoptosis of BRCA2-/- cells
Example 4
AZD5305
Annexin V+ cells (%) Mean±SEM; n=2
Compound concentration Log[M]
First experiment
Total apoptosis of BRCA2-/- cells
Example 4
AZD5305
Annexin V+ cells (%) Mean±SEM; n=2
Compound concentration Log[M]
Second experiment
Total apoptosis of BRCA2-/- cells
Example 4
AZD5305
Annexin V+ cells (%) Mean±SEM; n=3
Compound concentration Log[M]
Third experiment

*FIG. 4*

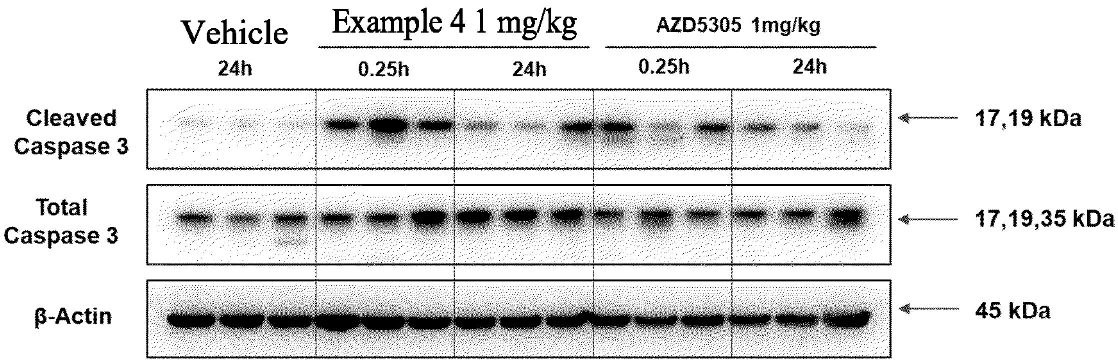
Vehicle
24h
Example 4 1 mg/kg
0.25h
24h
AZD5305 1mg/kg
0.25h
24h
Cleaved Caspase 3
Total Caspase 3
β-Actin
17,19 kDa
17,19,35 kDa
45 kDa

*FIG. 5*

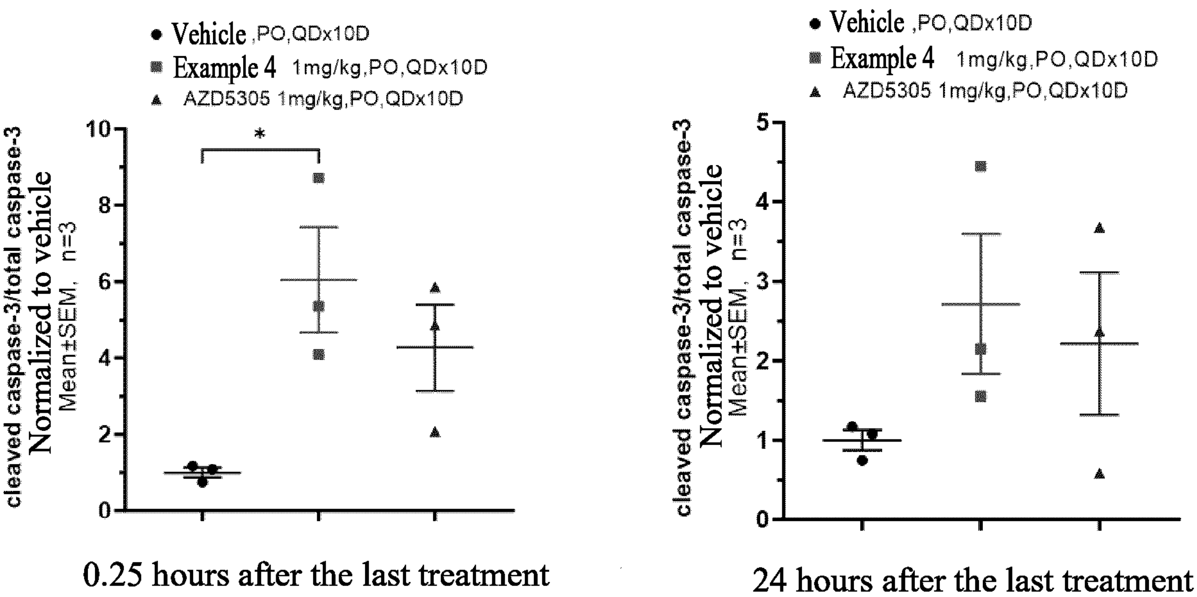
Vehicle ,PO,QDx10D
Example 4 1mg/kg,PO,QDx10D
AZD5305 1mg/kg,PO,QDx10D
cleaved caspase-3/total caspase-3
Normalized to vehicle
Mean±SEM, n=3
*
0.25 hours after the last treatment
24 hours after the last treatment

*FIG. 6*

## INTERNATIONAL SEARCH REPORT

International application No.
**PCT/CN2023/141057**

**A. CLASSIFICATION OF SUBJECT MATTER**

C07D471/04(2006.01)i; A61K31/4353(2006.01)i; A61K31/495(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: C07D,A61K,A61P

Documentation searched other than minimum documentation to the extent that such documents are included in the fields searched

Electronic data base consulted during the international search (name of data base and, where practicable, search terms used)

CNKI, CNABS, CNTXT, VEN, DWPI, ENTXT, WOTXT, REGISTRY, CAPLUS: 聚腺苷二磷酸核糖聚合酶, 抑制剂, PARP, 癌症, 肿瘤, 增殖, inhibitor, caner, tumor, tumour, proliferation, 根据权利要求1进行的子结构检索, substructure search according to claim 1

**C. DOCUMENTS CONSIDERED TO BE RELEVANT**

| Category* | Citation of document, with indication, where appropriate, of the relevant passages | Relevant to claim No. |
|---|---|---|
| X | WO 2022225934 A1 (XINTHERA INC.) 27 October 2022 (2022-10-27) claims 36 and 43-47 | 1-25, 26-34 |
| X | WO 2022228387 A1 (FOCHON BIOSCIENCES LTD.) 03 November 2022 (2022-11-03) claims 1-23 | 1-25, 27-34 |
| A | WO 2009053373 A1 (JANSSEN PHARMACEUTICA NV et al.) 30 April 2009 (2009-04-30) claims 1-13 | 1-34 |
| A | WO 2022222921 A1 (WIGEN BIOMEDICINE TECH SHANGHAI CO., LTD.) 27 October 2022 (2022-10-27) claims 1-12 | 1-34 |
| A | WO 2022225934 A1 (XINTHERA INC.) 27 October 2022 (2022-10-27) claims 36 and 43-47 | 26 |

☐ Further documents are listed in the continuation of Box C.  ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **22 March 2024** | **22 March 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
**PCT/CN2023/141057**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **29, 31-34**
because they relate to subject matter not required to be searched by this Authority, namely:

Claims 29 and 31-34 relate to a treatment method implemented on the human or animal body (PCT Rule 39.1(iv)). Nevertheless, a search is still performed on the basis of the technical subject matter of the use of the compound/composition in the preparation of a corresponding drug.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/141057**

| Patent document cited in search report | Publication date (day/month/year) | Patent family member(s) | Publication date (day/month/year) |
|---|---|---|---|
| WO 2022225934 A1 | 27 October 2022 | AU 2022260495 A1 | 05 October 2023 |
| | | US 2022348574 A1 | 03 November 2022 |
| | | US 11591331 B2 | 28 February 2023 |
| | | US 2023128041 A1 | 27 April 2023 |
| | | CA 3216373 A1 | 27 October 2022 |
| | | EP 4326720 A1 | 28 February 2024 |
| | | TW 202309025 A | 01 March 2023 |
| | | KR 20230172550 A | 22 December 2023 |
| | | CN 117177972 A | 05 December 2023 |
| WO 2022228387 A1 | 03 November 2022 | TW 202309027 A | 01 March 2023 |
| | | CN 117321051 A | 29 December 2023 |
| WO 2009053373 A1 | 30 April 2009 | PL 2215075 T3 | 30 April 2014 |
| | | US 2010222348 A1 | 02 September 2010 |
| | | US 8404713 B2 | 26 March 2013 |
| | | ES 2448870 T3 | 17 March 2014 |
| | | EP 2215075 A1 | 11 August 2010 |
| | | EP 2215075 B1 | 11 December 2013 |
| | | JP 2011500758 A | 06 January 2011 |
| | | JP 5525447 B2 | 18 June 2014 |
| WO 2022222921 A1 | 27 October 2022 | KR 20230175225 A | 29 December 2023 |
| | | AU 2022261011 A1 | 16 November 2023 |
| | | BR 112023021383 A2 | 19 December 2023 |
| | | EP 4328224 A1 | 28 February 2024 |
| | | CA 3213029 A1 | 27 October 2022 |
| | | CN 117279916 A | 22 December 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- CN 202211668333
- CN 202311484011
- WO 2021013735 A1
- US 5376645 A

### Non-patent literature cited in the description

- **FARRÉS J et al.** *Cell Death Differ.*, July 2015, vol. 22 (7), 1144-57
- **BERGE S.M. et al.** Pharmaceutical Salts. *J. Pharm. Sci.*, 1977, vol. 66, 1-19
- Prodrugs as Novel Delivery Systems. **T. HIGUCHI** ; **V. STELLA**. Bioreversible Carriers in Drug Design. American Pharmaceutical Association and Pergamon Press, 1987, vol. 14
- **D. FLEISHER** ; **S. RAMON** ; **H. BARBRA**. Improved oral drug delivery: solubility limitations overcome by the use of prodrugs. *Advanced Drug Delivery Reviews*, 1996, vol. 19 (2), 115-130
- Remington's Pharmaceutical Sciences. Mack Publishing Company, 1985